# EUROPEAN PATENT APPLICATION

(11) **EP 3 826 025 A2**
(43) Date of publication of application: **26.05.2021**
(21) Application number: 20200392.7
(22) Date of filing: 07.02.2012
(51) Int. Cl.: G16H 20/13, G07F 17/00, G06Q 50/22, G06Q 10/06, G06Q 40/08, G16H 70/40, G07F 9/02, G16H 40/67, G16H 50/20, A61J 7/00, G16H 40/00

(54) **PHARMACEUTICAL PACKAGING AND METHOD FOR DELIVERY OF SAME**

(30) Priority: 07.02.2011 US 201161462685 P; 21.04.2011 US 201113091979
(62) Divisional of application: 12744924.7
(71) Applicant: Daya Medicals, Inc., Boca Raton, FL 33486 (US)
(72) Inventor: DAYA, Kantilal Kasan, Boca Raton, Florida 33486 (US); DAYA, Justin K., Boca Raton, Florida 33486 (US)
(74) Representative: HGF

(57) **Abstract**

A disease management system and therapeutic hub are provided. In certain embodiments, a dispensing apparatus used as part of a compliance monitoring system for the disease management system can function as a therapeutic hub that interacts with a plurality of peripheral devices to accumulate, communicate, and analyze a variety of medical and non-medical related data of the patient.

## Description

### BACKGROUND OF INVENTION

Modifiable major health risk factors can be controlled with appropriate medicaments. It is well known that three major risk factors--serum cholesterol level, blood pressure, and smoking--increase the incidence of coronary heart disease (CHD) and related end points. Long-term studies have amassed extensive data on the relationships of major coronary-cardiovascular, cerebrovascular and diabetic sequelae risk factors--particularly serum cholesterol level, blood pressure (BP), cigarette smoking, and the consequent uncontrolled action of platelets--with incidence of coronary heart disease (CHD), stroke and cardiovascular disease (CVD) to mortality from these causes. These relationships have been characterized as strong, continuous, graded, consistent, independent, predictive, and etiologically significant for CHD, CVD, and diabetic sequelae.

Hypertension is a well documented risk factor for coronary artery, cerebrovascular, renovascular, and ocular vascular diseases. Unfortunately, hypertension remains vastly untreated. High blood pressure is also a major risk factor for stroke and heart disease.

Studies have also shown that reducing total / gross cholesterol levels, particularly Low Density Lipoproteins (LDL), Very Low Density Lipoproteins (VLDL), and an increase in High Density Lipoproteins (HDL) in patients, ranging from pediatric, teen and adult ages, prevents the onset of heart disease in apparently healthy persons. Treatment of relatively high risk men with clearly elevated cholesterol levels significantly reduced risk of heart attack and death from heart disease. Persons with heart disease or those deemed to be at high risk for stroke or heart attack should seriously consider treatment if their LDL cholesterol level is greater than about 130 mg/dl. Reports have indicated that reducing LDL cholesterol and total blood cholesterol can reduce the incidence of coronary heart disease and heart attacks in men at high risk because of significant amounts of plasma cholesterol.

Several other studies have shown that treating abnormal lipid levels can reduce cardiovascular morbidity and mortality.

A patient can reasonably control his or her elevated blood pressure, abnormal cholesterol level, elevated triglycerides, blood glucose levels, tobacco use and the consequent aberrant adherent behavior of platelets, uncontrolled diabetes, obesity, and physical activity. Patients cannot control their age, family history of early heart disease (having a father or brother diagnosed with heart disease before age 55 or having a mother or sister diagnosed before age 65), and strongly imbedded habits, such as smoking.

In the healthcare community, there is consistent agreement that modifiable risk factors responsible for the cardiovascular and stroke epidemic in the Western World include: elevated uncontrolled blood pressure (> 115 / 75 mm Hg), elevated cholesterol (> 130 mm L), elevated triglycerides, cigarette smoking and its consequent aberrant adherent stimulation of platelets, elevated levels of C-Reactive proteins, obesity, lack of exercise, uncontrolled diet, and absence of consumption of fruits and vegetables.

In the past decade the concept has evolved that the intensity of risk factor management should be adjusted according to the severity of estimated risk. Global risk patient assessment is the estimation of absolute risk based on the summation of risks contributed by each risk factor. Several methods have been used to sum risks. The Framingham, Mass. researchers recently proposed a method in which the continuous relationship between risk-factor intensity and coronary risk is employed. Framingham scoring uses only the "standard" risk factors (smoking, blood pressure, total serum cholesterol, HDL cholesterol, blood glucose and age). But, conditional and predisposing risk factors are not used in the Framingham risk equation because of lack of evidence for a strong, independent contribution to CHD risk prediction. Several of the conditional and predisposing risk factors may contribute to the development of CHD. Thus, their detection and therapeutic modification may be appropriate in some patients.

The past decade has witnessed major strides in the prevention of CHD through modification of its causes. The most dramatic advance has been the demonstration in a "mega International" study of 17,800 men and women with normal cholesterol levels that found rosuvastatin (statin use) cuts deaths from heart attacks and strokes of healthy individuals (primary prevention). Other dramatic advances have indicated that aggressive medical therapy can substantially reduce the likelihood of recurrent major coronary syndromes in patients with established CHD (secondary prevention).

A similar potential exists for risk reduction in patients without established CHD (primary prevention). However, the risk status of persons without CHD varies greatly, and this variability mandates a range in the intensity of interventions. Effective primary prevention thus requires an assessment of risk to categorize patients for selection of appropriate interventions. Some of the major and independent risk factors for CHD are cigarette smoking of any amount, elevated blood pressure, elevated serum total cholesterol and low-density lipoprotein cholesterol (LDL-C), low serum high-density lipoprotein cholesterol (HDL-C), elevated triglycerides, obesity, the presence and levels of C-Reactive Proteins, diabetes mellitus, and advancing age. The quantitative relationship between these risk factors and CHD risk has been elucidated by The Framingham Heart Study and other studies. These studies show that these major risk factors are additive in predictive power. Accordingly the total risk of the person can be estimated by the summing of the risk imparted by each of the major risk factors. Other factors are also associated with increased risk for CHD, for example: (a) Predisposing Risk Factors such as obesity, abdominal obesity, physical inactivity, family history of premature coronary heart disease, ethnic characteristics, psychosocial factors; and (b) Conditional Risk Factors, such as elevated serum triglycerides, small LDL particles, elevated serum homocysteine, elevated serum Lipoprotein (a), prothrombotic factors (e.g. Fibrinogen), and inflammatory markers (e.g. C-Reactive protein).

These risk factors are generally categorized into two types: Predisposing risk factors and Conditional risk factors. Conditional risk factors are associated with increased risk for CHD, although their causative, independent, and quantitative contributions to CHD have not been well documented. Predisposing risk factors are those that worsen the independent risk factors. Two of them - obesity and physical inactivity - are designated major risk factors by the American Heart Association because abdominal obesity is an indicator of insulin resistance. Conditional risk factors are those that have been correlated with CHD risk, but their quantitative relationship to major coronary events remains to be defined adequately in large prospective studies. The predisposing risk factors contribute to the development of the causal and conditional risk factors.

Accordingly, medical therapy for prevention or mitigation of coronary heart disease, stroke, cardiovascular disease, diabetic sequelae, or the reoccurrence of each disease or malady thereof preferably is customized based on the predictive measures disclosed in related US Patent Application 11/348,786 and herein.

### Compliance--A Behavioral Phenomenon

Compliance is commonly understood and defined as "the extent to which the patient's behavior (in terms of taking medication, following diets, or executing other lifestyle changes) coincides with medical recommendations." Compliance is sometimes defined as patients doing what health professionals want them to do. Compliance with prescribed therapeutic regimens has been a documented concern to health professionals since the time of Hippocrates. Patient compliance with medical regimens is a behavioral problem of interest because it affects the patient's health. If the therapeutic regimen is to be effective, the patient must comply with that regimen. No regimen of medication, diet, or behavioral change will benefit the patient who does not follow it.

The role of compliance with medical regimens as a predictor of health outcomes in chronic diseases conditions such as cerebrovascular disorders and diabetes has been the intense focus of recent research. Increasing patient compliance with treatment regimens may decrease hospitalizations and mortality in patient populations as well as improve quality of life (QOL).

Variations in compliance rates have been found to range between 10%-85% depending on the population, the definition of compliance used and the medical regimen studied. E.g., the Pitney-Bowes Compliance Study. While findings have varied, poor compliance with prescribed therapy has been identified in the literature as an issue that encompasses serious problems. Poor compliance has direct negative correlations for the health of the patient, effective use of resources and assessments of the clinical efficacy of the treatment.

Recent noncompliance rates for general health-seeking behaviors and lifestyle modifications are set forth below. The results show low general health-seeking behavior. Not only do patients fail to seek medical attention, they also most likely will not stay in care or comply with follow up appointments over 50% of the time.

**TABLE 1. Non-Compliance Behavior Table**

| TASKS | RATES |
|---|---|
| Community Screening | 35%-90% |
| Referral After Screening | 50%-65% |
| Staying in Care | 31%-66% |
| Follow-up Appointments | 16%-84% |
| Medications | 31%-58% |
| e.g.: Statin Compliance: | |
| After 3 Months | 60% |
| After 6 Months | 43% |
| After 60 Months | 26% |
| Diet | 13%-76% |
| Physical Activity | 40%-50% |
| Smoking Cessation | 71%-96% |

Even when appropriate treatments are offered, patients do not always adhere to the prescribed treatment regimens. Fourteen to 21% of patients never fill their original prescription. 30%-50% of patients ignore or otherwise compromise their medication instructions.

Compliance rates have been examined for heart failure patients. The results are summarized below. Findings showed that a majority of patients failed to recall elements of potentially important medical advice. Despite some differences in compliance rates in circumstances in which patients did recall medical advice, those that did recall the advice did not always comply with the advice recalled.

**TABLE 2.**

| Compliance of Patients with Heart Disease | | | |
|---|---|---|---|
| TASKS | Recalled MD Advice % Total | Noncompliance Rate (%) | Did Not Recall Advice |
| Medications | 97.9 | 8.7 | 66.7 |
| Diet (Low Salt) | 83.6 | 23.6 | 55.8 |
| Physical Activity | 70.8 | 76.4 | 84.5 |
| Smoking Cessation | 76.3 | 90.4 | 60.0 |
| Alcohol Use | 42.1 | 60.0 | 81.8 |

In another study involving African American patients with heart conditions measuring the relationship between medication and dietary compliance with hospital readmissions or heart failure HF decompensation, noncompliance was the leading cause for heart failure decompensation, accounting for 43% of hospital admissions. Non-compliance with medication and diet was as high as 64% and 22%, respectively.

Causative factors were identified in 85.5% of the patients in a further study of German heart failure patients. Non-compliance with the medication regimen was the most common identified factor causing heart failure decompensation in 41.9% of cases. Noncompliance with drugs was found in 23% of patients.

A survey of U.S. residents reported that 42% of hypercholesterolemic patients were aware of their condition, although only 4% were adequately treated and controlled.

High Blood Pressure (HBP) is among the most prevalent and important risk factors for cardiovascular, cerebrovascular, and renal disease. Effective care and control of HBP cannot be achieved without compliance to recommended treatment regimens. Estimates of controlled Blood Pressure (BP) among identified HBP patients typically range from 20%-30% in the U.S., in large part, because only one half of the individuals diagnosed with hypertension are in treatment and one half of these are not receiving treatment adequate to control BP.

In another critical review, it was found that noncompliance rates with prescribed therapeutic regimen range from 30%-60%, and at least 50% of patients for whom drugs are prescribed fail to receive full benefit through inadequate compliance. The high noncompliance rates in HBP treatment have multiple implications at the individual and societal levels. These rates jeopardize patients' health and well being, result in suboptimal health outcomes, lead to inefficient use of health resources, and incur costly treatment for the complications of untreated or inadequately treated HBP. In spite of the role played by compliance and the control of HBP, clinicians are not routinely assessing patients' compliance level and patients rarely volunteer this information to their clinician.

The current therapy practice involves prescribing multiple pills for a patient to treat multiple health conditions. Medication compliance for multiple pills is poor. Further, many people forget to take or become confused as to which pills are to be taken at certain times on certain days. The longer the timespan following the doctor's appointment, the greater a failure of medication compliance by the patient will likely occur.

### SUMMARY OF THE INVENTION

Embodiments of the present invention relate to a primary care and/or a secondary care clinical protocol and a therapeutic medical treatment regimen. An aspect of an embodiment of the present invention comprises of a diagnostic criteria, an evaluation of a patient's total risk for Heart Attack, Stroke, and manifestation of diabetic sequelae, a treatment regimen that relates to a selection system enabling the physician or other health care provider to select one of a plurality of one-a-day combinatory drug therapeutic regiments, and a dispensing system for dispensing the selected regiment.

One such selection system includes a printed substrate having at least three parts and each subpart having a sub plurality of indicia representing the one-a-day drug doses and/or treatment regimen formulations and means for moving the sub-parts with respect to each other such as flip charts or pullout plates. In an embodiment, the plurality of indicia are arranged in matricies. In an embodiment, the matrices of data (treatment regimens) are color coded. In an embodiment, the matrices of data are provided to enable the physician to titrate dosages over weekly, monthly or quarterly periods of time based upon the condition of the patient. In an embodiment, a primary matrix has four sub-parts which list treatment regimens (or representative indicia). Further matrices can show additional treatment regimens. Therefore, the selection system enables the physician to pick one selected treatment regimen from the listed formulations, adjust dosages over time (titration of medication), and enables the physician to use the matrices as an educational tool to motivate the patient. The patient sees his or her dosages drop over time by viewing the matrix.

In another embodiment, the selection system comprises an information processing system. The information processing system enables the physician or healthcare provider to select one of the treatment regimens employing a computer (or other electronic device) with a memory, display, and operator input controls. The display shows, upon respective operator inputs, pluralities of treatment regimens and an output generator shows a selected treatment regimen from one of the pluralities of treatment regimens based upon the operator's selection. The information processing system may generate a printed version (script) showing indicia of the selected treatment regimen and may be electronically coupled to a dispensing system which dispenses the selected treatment regimen ordered by the physician.

The dispensing system includes respective storage containers for each formulation of the treatment regimen, each treatment regimen and each respective storage container having a unique formulation of commonly prescribed dosages of widely use medicaments. A treatment regimen dispensing interface accepts a data input from an operator or an electronic data transmission permitting selection of the treatment regimen and dispensing from the corresponding container, the selected treatment regimen. In an embodiment, dispensing control interfaces use an operator confirmation feedback before the pills are finalized for dispensing.

In another aspect of an embodiment of the present invention, a method of treating a patient with a one-a-day, orally administered, treatment regimen is provided, including selecting one of the plurality of treatment regimens and titrating the dosages supplied to the patient after weekly, monthly or quarterly periods of time. Some embodiments of the present invention facilitate the selection of a treatment regimen and/or the change in dosages or titration of dosages of a treatment regimen over time.

In alternative embodiments, rather than combining the customized combination of medications into a single one-a-day treatment regimen, the present invention also relates to a packaging system enabling the physician or other healthcare provider to deliver the medications in a plurality of treatment regimens in a Multi Unit dose (one-a-day or, if necessary, more than once a day) packaging combination to increase compliance with medical therapy. In an embodiment, the Multi Unit Dose Package ("MUDP") contains one dosage of medicaments prescribed in the treatment regimen. In some embodiments, the MUDP is a pouch containing the medicaments. In embodiments, the MUDP of the subject invention comprises a container that has multiple indented "pie-shaped" pockets. In a particular embodiment, each pie-shaped pocket is separated from the others by plastic packaging material of the MUDP. The packaging system may contain multiple medicaments in the pie-shaped arrangement that may appear as one, preferably multicolored, object when packaged. In embodiments, each medication is provided in a different color. Each treatment regimen of the plurality is selected based on the determined customary therapeutic needs of the individual under treatment. In an embodiment, a selection system and combination therapy matrices of data enable the physician to titrate dosages of each pharmaceutical therapy.

The present invention also relates to the methods of delivery for the treatment regimen Multi Unit Dose Packages over weekly, monthly, quarterly, or longer periods of time based upon the condition of the patient. The use of a MUDP for the individual's treatment regimen with various commonly prescribed dosages of preventive medications may enhance compliance.

The present invention provides packaging for a plurality of pills into a MUDP corresponding to the selection system and the combination treatment matrices mentioned above used in treating a patient for hypertension, hypercholesterolemia, hypertriglyceridemia, anti-platelet aggregation, and related health issues over a weekly, monthly, quarterly, or longer period of time. Ideally, the MUDPs are delivered directly to the patient from the pharmacy. In an embodiment, Multi Unit Dose packaging is provided for complete primary and/or secondary treatment on one strip packaging dispensing system for direct shipment to the patient from a location remote from the patient.

The present invention can further greatly improve patient compliance by providing the Multi Unit Dose Packaging over a period of time (e.g. week, month, quarter, or longer) and having the physician titrate the dosages (adjust the dosages of each effective therapeutic ingredient) over the period of time based upon the condition of the patient. If necessary, the combination treatment pack may provide alternative dosages for treatment at more than one time per day. The MUDP may thus be taken once, two, three, four, or more times per day. The MUDP operates to greatly improve patient compliance with drug treatment regimens and treatment end point elevation to healthier states and potentially reduction for Heart Attack and Stroke to absolute minimums.

An embodiment of the present invention directs the management of the risk factors that cause Cardiovascular and Cerebrovascular accidents. The treatment regimens may include any existing and/or new Drug(s) or Drug Compound that would control the risk factors that would cause Cardiovascular and/or Cerebrovascular accidents. The treatment regimens can also include tests or other activities for the patient (e.g., diet and exercise) in addition to or instead of the prescribed medicaments.

In another aspect of an embodiment of the subject invention, a dispensing device provided capable of detecting when a MUDP or a medicament is used. Thus, the dispensing device can facilitate tracking of the patient's compliance with a treatment regimen. In a further embodiment, a communication device is also incorporated into the dispensing device capable of communicating such information to a computer on a network. Thus, the tracking information can be evaluated and communicated accordingly.

In yet another aspect of an embodiment of the subject invention, a Disease Management System is provided. The Disease Management System can include: a Diagnostic Module, which provides access to patient information and scientific guidelines for patient treatment; a Diagnostic Interpretive Module, which provides tools to evaluate risk of particular diseases or conditions based on patient information and an evaluative methodology; a Prescriptive Module, which is used to recommend, select, and/or evaluate one or more treatment regimens based on patient information and guidelines; a Dispensing Module, which evaluates a patient's compliance with a treatment regimen; and/or a Feedback and Patient Management Module, which gathers compliance information and evaluates efficacy of a treatment regimen for a patient. In embodiments of the subject invention, some or all of the modules described can communicate to manage a disease, medical condition, and/or health problem in a patient.

According to yet another aspect of the invention, in addition to dispensing medication and tracking compliance, a dispensing device can further function as a therapeutic hub that interacts with a plurality of peripheral devices to accumulate, communicate, and analyze a variety of medical and non-medical related data of a user.

Although the treatment of CHD, diabetes, and other conditions and their risk factors are described in detail herein, aspects of the present invention can be applied to other diseases, medical conditions, or health problems.

### BRIEF DESCRIPTION OF DRAWINGS

In order that a more precise understanding of the above recited invention be obtained, a more particular description of the invention briefly described above will be rendered by reference to specific embodiments thereof that are illustrated in the appended drawings. Understanding that these drawings depict only example embodiments of the invention and are not therefore to be considered as limiting in scope, the invention will be described and explained with additional specificity and detail through the use of the accompanying drawings in which:
FIG. 1 diagrammatically illustrates an information processing system for selecting a treatment regimen from a plurality of treatment regimens in accordance with an embodiment of the subject invention;
FIG. 2 diagrammatically illustrates a dispensing system for a plurality of medicaments in accordance with an embodiment of the subject invention;
FIG. 3 diagrammatically illustrates a dispensing system for a plurality of medicaments in accordance with another embodiment of the subject invention;
FIG. 4 diagrammatically illustrates a dispensing system for a plurality of medicaments in accordance with yet another embodiment of the subject invention;
FIGS. 5A-5E diagrammatically illustrate a system for selecting a treatment regimen from a plurality of treatment regimens in accordance with an embodiment of the subject invention;
FIGS. 6A-6C diagrammatically illustrate a system for selecting a treatment regimen from a plurality of treatment regimens in accordance with another embodiment of the subject invention;
FIG. 7 diagrammatically illustrates a system for selecting a treatment regimen from a plurality of treatment regimens in accordance with yet another embodiment of the subject invention;
FIGS. 8A-8C illustrate various apparatuses for packaging multiple medicaments for convenient dosing according to embodiments of the subject invention;
FIG. 9 diagrammatically illustrates a method for diagnosing disease in a patient in selecting a treatment regimen in accordance with an embodiment of the subject invention;
FIG. 10 diagrammatically illustrates a method for delivering a medicament to a patient in accordance with an embodiment of the subject invention;
FIG. 11 illustrates example apparatuses for packaging one or more medicaments for delivery to a patient in accordance with an embodiment of the subject invention;
FIG. 12 diagrammatically illustrates a Disease Management System in accordance with an embodiment of the subject invention;
FIG. 13 illustrates an apparatus for dispensing medicaments in accordance with an embodiment of the subject invention;
FIG. 14 illustrates an interface to a Diagnostic Module in accordance with an embodiment of the subject invention;
FIG. 15 illustrates an interface to a Disease Management System in accordance with an embodiment of the subject invention;
FIG. 16 illustrates an interface to a Diagnostic Module in accordance with an embodiment of the subject invention;
FIG. 17 illustrates an interface to a Diagnostic Module in accordance with an embodiment of the subject invention;
FIG. 18 illustrates an interface to a Diagnostic Module in accordance with an embodiment of the subject invention;
FIG. 19 illustrates an interface to a Diagnostic Module in accordance with an embodiment of the subject invention;
FIG. 20 illustrates an interface to a Feedback and Patient Management Module in accordance with an embodiment of the subject invention;
FIG. 21 illustrates an interface to a Feedback and Patient Management Module in accordance with an embodiment of the subject invention;
FIG. 22 illustrates an interface to a Diagnostic Interpretive Module in accordance with an embodiment of the subject invention; and
FIGS. 23A-23C illustrate an interface to a Prescriptive Module in accordance with an embodiment of the subject invention.
FIG. 24 illustrates a compliance monitoring device in accordance with an embodiment of the subject invention.
FIG. 25 illustrates a compliance management system in accordance with an embodiment of the subject invention.
FIG. 26 illustrates a method for tracking and/or using patient compliance information in accordance with an embodiment of the subject invention.
FIG. 27A shows a representation of a dispensing device in accordance with an embodiment of the subject invention.
FIG. 27B shows a diagram of a system for a dispensing device according to an embodiment of the subject invention.
FIG. 28 shows a representation illustrating a dispensing method of medicine package according to an embodiment of the subject invention.
FIG. 29 illustrates connectivity of a dispensing device in accordance with an embodiment of the subject invention.
FIG. 30 shows a representation of a stand-alone dispensing device according to an embodiment of the subject invention.
FIG. 31 shows a representation of a low processing (or dummy) type dispensing device according to an embodiment of the subject invention.
FIG. 32 shows representations of a dispensing device dispensing a series of pouches of medication according to a time schedule in accordance with an embodiment of the subject invention.
FIGS. 33A-33C show representations of a dispensing device having multiple lines of medication in accordance with certain embodiments of the subject invention. Each line has a series of pouches of medication.
FIG. 34 shows an example of how a dispensing device as a therapeutic hub interacts with third parties and peripheral devices in accordance with an embodiment of the subject invention.

### DETAILED DISCLOSURE

The present invention relates to a selection system for selecting one of a plurality of one-a-day combination medical treatment regimen, or alternatively Multi Unit Dose Package(s), for treatment of hypertension, hypercholesterolemia, hypertriglyceridemia and antiplatelet treatment, an information processing system therefore, a dispensing system for dispensing the selected one-a-day combination medical treatment regimen or MUDP from said plurality of one-a-day combination medical treatment regimen and MUDP(s), and a method of treatment using said one-a-day combination medical treatment regimen or MUDP, and a follow-up or feedback system to monitor the patients state of health and compliance.

In certain aspects, the present invention relates to a packaging system for the delivery of a medical treatment regimen for hypertension, hypercholesterolemia, hypertriglyceridemia, anti-platelet aggregative treatment, treatment of type 2 diabetes and/or reduction and postponement of diabetic sequelae, among other conditions or health issues. In an embodiment, the packaging system utilizes the drug combination matrices and a clinical protocol to provide a MUDP to increase patient compliance with drug treatment. In an embodiment, the combination matrices and clinical protocol are determined from the risk factor studies as detailed below and incorporated from US Patent Application 11/348,786. Therefore, customized treatment protocols have been determined to optimize patient response, disease management, and evidenced based treatment medical regimen. In addition to the treatment protocol, the present invention maximizes treatment efficacy by improving aspects of drug delivery to patients to counteract detrimental behavioral issues, namely patient compliance with drug treatment regimens. In an embodiment, MUDPs are provided in a strip format for variable therapeutic periods and time frames.

In an embodiment of the invention, the Multi Unit Dose Packaging system comprises an individual pouch to contain the patient's determined prescribed therapy. In embodiments, the pouch contains a plurality of pocketed or indented compartments to hold therapeutic regimen or medication. The pouch commonly contains a range of two to six, and occasionally even more, separate and distinct pockets or indents to hold the therapeutic regimen or medication. The distinct pockets or indents can be separated within the pouch by a thin partition. Thus, each therapeutic regimen or medication can be separated from the other constituents of the pouch. The pouch thus comprises a therapeutic regimen container, and serves as a Multi Unit Dose Package, to simplify the patient's treatment protocol.

In another embodiment of the invention, the MUDPs are then packaged into MUDP supply boxes containing a plurality of MUDPs. In an embodiment, the MUDPs can be packaged into 7-day, 30-day, 90-day, or even longer supply boxes, customized for the patient. In an embodiment, the supply boxes contain one MUDP corresponding to each day of the treatment indicated on the supply box. The physician can therefore direct the pharmacy to deliver direct to the patient the drug treatment customized for each individual patient in both a condensed MUDP and long-term package to simplify patient compliance.

In yet other embodiments, each MUDP can represent one combination dosage of multiple prescribed daily drug treatments. The MUDP can be taken one, two, three, four, or more times per day as prescribed. Accordingly, the long-term MUDP supply boxes reflect the increase in daily drug treatment with a corresponding increase in MUDPs delivered by the supply boxes.

In another aspect of an embodiment of the subject invention, a dispensing device is incorporated into the supply box capable of detecting when a MUDP or a medicament is removed from the supply box or when such an event has not occurred within a given period of time. Thus, the dispensing device can facilitate tracking of the patient's compliance with a treatment regimen. In a further embodiment, a communication device is also incorporated into the dispensing device capable of communicating such information to a computer on a network. Thus, the tracking information can be evaluated and communicated accordingly.

In yet another aspect of an embodiment of the subject invention, a Disease Management System is provided. The Disease Management System can include: a Diagnostic Module, which provides access to patient information and scientific guidelines for patient treatment; a Diagnostic Interpretive Module, which provides tools to evaluate risk of particular diseases or conditions based on patient information and an evaluative methodology; a Prescriptive Module, which is used to recommend, select, and/or evaluate one or more treatment regimens based on patient information and guidelines; a Dispensing Module, which evaluates a patient's compliance with a treatment regimen; and/or a Feedback and Patient Management Module, which gathers compliance information and evaluates efficacy of a treatment regimen for a patient. In embodiments of the subject invention, some or all of the modules described can communicate to manage a disease, medical condition, and/or health problem in a patient.

The subject matter of the present invention is described with specificity to meet statutory requirements. But this description is not intended to limit the scope of this patent. Rather, the inventors have contemplated that the claimed subject matter might also be embodied in other ways, to include different steps or combinations of steps similar to those described in this document, in conjunction with other present or future technologies.

Aspects of the invention can be described in the general context of computer-executable instructions, such as program modules, being executed by a computer. Generally, program modules include routines, programs, objects, components, data structures, etc., that perform particular tasks or implement particular abstract data types. Such program modules can be implemented with hardware components, software components, or a combination thereof. Moreover, those skilled in the art will appreciate that the invention can be practiced with a variety of computer-system configurations, including multiprocessor systems, microprocessor-based or programmable-consumer electronics, minicomputers, mainframe computers, and the like. Any number of computer-systems and computer networks are acceptable for use with the present invention.

Specific hardware devices, programming languages, components, processes, protocols, formats, and numerous other details including operating environments and the like are set forth to provide a thorough understanding of the present invention. In other instances, structures, devices, and processes are shown in block-diagram form, rather than in detail, to avoid obscuring the present invention. But an ordinary-skilled artisan would understand that the present invention can be practiced without these specific details. Computer systems, servers, work stations, and other machines can be connected to one another across a communication medium including, for example, a network or networks.

As one skilled in the art will appreciate, embodiments of the present invention can be embodied as, among other things: a method, system, or computer-program product. Accordingly, the embodiments can take the form of a hardware embodiment, a software embodiment, or an embodiment combining software and hardware. In one embodiment, the present invention takes the form of a computer-program product that includes computer-useable instructions embodied on one or more computer-readable media. Methods, data structures, interfaces, and other aspects of the invention described above can be embodied in such a computer-program product.

Computer-readable media include both volatile and nonvolatile media, removable and nonremovable media, and contemplate media readable by a database, a switch, and various other network devices. By way of example, and not limitation, computer-readable media comprise media implemented in any method or technology for storing information. Examples of stored information include computer-useable instructions, data structures, program modules, and other data representations. Media examples include, but are not limited to, information-delivery media, RAM, ROM, EEPROM, flash memory or other memory technology, CD-ROM, digital versatile discs (DVD), holographic media or other optical disc storage, magnetic cassettes, magnetic tape, magnetic disk storage, and other magnetic storage devices. These technologies can store data momentarily, temporarily, or permanently. In an embodiment, non-transitory media are used.

The invention can be practiced in distributed-computing environments where tasks are performed by remote-processing devices that are linked through a communications network or other communication medium. In a distributed-computing environment, program modules can be located in both local and remote computer-storage media including memory storage devices. The computer-useable instructions form an interface to allow a computer to react according to a source of input. The instructions cooperate with other code segments or modules to initiate a variety of tasks in response to data received in conjunction with the source of the received data.

The present invention can be practiced in a network environment such as a communications network. Such networks are widely used to connect various types of network elements, such as routers, servers, gateways, and so forth. Further, the invention can be practiced in a multi-network environment having various, connected public and/or private networks.

Communication between network elements can be wireless or wireline (wired). As will be appreciated by those skilled in the art, communication networks can take several different forms and can use several different communication protocols. And the present invention is not limited by the forms and communication protocols described herein.

Embodiments of the subject invention can be embodied in a processing system. Components of the processing system can be housed on a single computer or distributed across a network as is known in the art. In an embodiment, components of the processing system are distributed on computer-readable media. In an embodiment, a user can access the processing system via a client device. In an embodiment, some of the functions or the processing system can be stored and/or executed on such a device. Such devices can take any of a variety of forms. By way of example, a client device may be a desktop or laptop computer, a personal digital assistant (PDA), an MP3 player, a communication device such as a telephone, pager, email reader, or text messaging device, or any combination of these or other devices. In an embodiment, a client device can connect to the processing system via a network. As discussed above, the client device may communicate with the network using various access technologies, both wireless and wireline. Moreover, the client device may include one or more input and output interfaces that support user access to the processing system. Such user interfaces can further include various input and output devices which facilitate entry of information by the user or presentation of information to the user. Such input and output devices can include, but are not limited to, a mouse, touch-pad, touch-screen, or other pointing device, a keyboard, a camera, a monitor, a microphone, a speaker, a printer, a scanner, among other such devices. As further discussed above, the client devices can support various styles and types of client applications.

The invention is described more fully hereinafter with reference to the accompanying drawings, in which embodiments of the invention are shown. This invention can, however, be embodied in many different forms and should not be construed as limited to the embodiments set forth herein. Rather, these embodiments are provided to fully enable those of ordinary skill in the art to embody and practice the invention.

In an embodiment, the treatment protocols set forth herein and accomplished by use of the MUDP and/or Disease Management System are aimed at reducing the incidence of cerebrovascular and cardiovascular diseases, and diabetic sequelae by primary prevention approaches. In an embodiment, the therapeutic modality utilized is that of secondary prevention. In an embodiment, preventive efforts target each major therapeutically modifiable risk factor. In an embodiment, the treatment protocol assesses global risk based on the summation of risk factors and utilizes them clinically by (a) identifying and measuring patients at risk and (b) managing risk factors. In an embodiment, the protocol identifies and measures patients at risk--patients at high risk and patients at low risk--for immediate attention and intervention of the major modifiable risk factors by secondary treatment protocols. In an embodiment, risk factor management entails: (a) Patient enlightenment and motivation, that is, motivate patients to join and adhere to traditional risk reduction therapies ranging in spectrum from diet modification, lifestyle changes to medical therapy; and (b) Modification of the intensity of risk-reduction therapy based upon the global risk estimate, such as stringent diet, exercise, and titration of medication to maintain the risk factors within the parameters of a low risk state.

In an embodiment, the first step in the implementation of the MUDP combination therapeutic regimen treatment is to identify the risk of the patient to CHD, cerebrovascular disease and diabetic sequelae. There are several clinical protocols which may be employed to determine the risk to the patient. The Framingham Heart Study or Framingham Report defined Low Risk as the risk for CHD at any age that is conferred by any combination of all the following parameters: blood pressure<120/<80 mm Hg., total cholesterol 160 to 199 mg./dL. (or LDL-C 100 to 129 mg/dL) for men and 55 mg/dL for women in a non-smoking person with no diabetes. The Framingham study defines a low-risk state as: serum total cholesterol 160 to 199 mg/dL; LDL-C 100 to 129 mg/dL; HDL-C 45 mg/dL. in men and 55 mg/dL in women, blood pressure<120 mm Hg systolic and <80 mm Hg diastolic, nonsmoker; no diabetes mellitus. Table 3 provides one clinical protocol for accessing hypertension in a patient.

**TABLE 3. Hypertension can be rated as follows:**

| Category | Systolic (mmHg) | | Diastolic Result | |
|---|---|---|---|---|
| Normal | 115 or lower | And | 75 or lower | Good for you |
| Prehypertension | 115-139 | Or | 75-89 | Your blood pressure could be a problem. Make changes in what you eat and drink, be physically active and lose extra weight. If you also have diabetes, see your physician. |
| Hypertension Stage 1 | 140-159 | Or | 90-99 | You have high blood pressure. Ask your doctor or nurse how to control it. |
| Hypertension Stage 2 | 160 or higher | Or | 100 or higher | You have high blood pressure. |

| | | | | |
|---|---|---|---|---|
| Government agencies have recommended the following treatment for hypertension: Stage 1 hypertension: thiazide-type diuretics, Ace Inhibitors, Beta Blockers, ACEI, ARB, BB, CCB; Stage 2 hypertension: 2 drug combination-thiazide-type diuretics and ACEI, ARB, BB or CCB. After this treatment, if the patient is not at blood pressure or BP goal, optimize dosages or add additional drugs until goal BP is reached. | | | | |

Absolute risk is defined as the probability of developing CHD over a given period of time. A recent Framingham report specifies absolute risk for CHD over the next 10 years. The relative risk is the ratio of the absolute risk of the given patient (or group) to that of low-risk group. Literally, the term relative risk represents the ratio of the incidence in the exposed population divided by the incidence in the unexposed persons. The denominator of the ratio can be either the average risk of the entire population or the risk of a group devoid of risk factors. Both the absolute and relative risk is derived from the recently published risk score sheets.

One methodology for risk estimation builds upon the Framingham study and assigns points for indications of various risk factors. For example, risk factors can include age (in gradations from in 5 year blocks from age 30 and points from -1 to 7 up to age 74), LDL cholesterol, HDL cholesterol, total cholesterol, blood pressure (BP) (systolic, diastolic, either, or both), diabetes (male: Y or N, 0 or 2 points or female: Y or N, 0 or 4 points, defined as a fasting plasma glucose level>126 md/dL), smoker (Y or N, 0 or 2 points, defined any smoking in the past month). Other risk factors, can be included, for example hyperlipidemia and obesity, which can be defined by Body Mass Index (BMI) or another method known in the art, among other factors. In addition, the number of points assigned to various levels of indications of risk factors may also vary. In a further embodiment, the points from the various risk factors are totaled to provide a point total that is correlated with an estimated risk based on those factors. Table 4, below, provides one example of a scoring protocol referred to as the Framingham Scoring System. Other protocols or variations on this protocol will be known to those skilled in the art and can be used with the subject invention.

**TABLE 4.**

| Global Risk Assessment Scoring Chart | | |
|---|---|---|
| Age | | |
| Risk | Male | Female |
| <35 | -1 | -9 |
| 35-39 | 0 | -4 |
| 40-44 | 1 | 0 |
| 45-49 | 2 | 3 |
| 50-54 | 3 | 6 |
| 55-59 | 4 | 7 |
| 60-64 | 5 | 8 |
| 65-69 | 6 | 8 |
| >69 | 7 | 8 |

| Total Cholesterol (mg/dl) | | |
|---|---|---|
| < 160 | -3 | -2 |
| 160-199 | 0 | 0 |
| 200-239 | 1 | 1 |
| 240-279 | 2 | 2 |
| > 279 | 3 | 3 |

| HDL Cholesterol (mg/dl) | | |
|---|---|---|
| <35 | 2 | 5 |
| 35-44 | 1 | 2 |
| 45-49 | 0 | 1 |
| 50-59 | 0 | 0 |
| >59 | -2 | -3 |

| Systolic Blood Pressure (mmHg) | | |
|---|---|---|
| < 120 | 0 | -3 |
| 120-129 | 0 | 0 |
| 130-139 | 1 | 1 |
| 140-159 | 2 | 2 |
| > 159 | 3 | 3 |

| Diastolic Blood Pressure (mmHg) | | |
|---|---|---|
| <85 | 0 | 0 |
| 85-89 | 1 | 1 |
| 90-99 | 2 | 2 |
| >99 | 3 | 3 |

| Diabetes | | |
|---|---|---|
| No | 0 | 0 |
| Yes | 2 | 4 |

| Smoker | | |
|---|---|---|
| No | 0 | 0 |
| Yes | 2 | 2 |

| | | |
|---|---|---|
| According to this protocol, points for age, total cholesterol, HDL cholesterol, blood pressure, diabetes, are assessed and the points are totaled to obtain a Framingham Score. In a variation on this protocol, LDL cholesterol, and/or the ratio between LDL cholesterol and HDL cholesterol, can be assessed in addition to or instead of total cholesterol. In another variation, only systolic pressure is assessed, or both systolic and diastolic pressure are assessed but only the category with the greatest point result is counted toward the total. Next, a 10 year CHD risk projection can be correlated with the total points. Table 5 provides CHD Risk estimates based on scores developed from the protocol of Table 4 (the Framingham Scoring System). | | |

**TABLE 5. CHD Risk Table (determined CHD risk from Framingham Score)**

| Point Total | 10 Yr CHD Risk |
|---|---|
| <-3 | 1% |
| -2 | 2% |
| -1 | 2% |
| 0 | 3% |
| 1 | 4% |
| 2 | 4% |
| 3 | 6% |
| 4 | 7% |
| 15 | 9% |
| 6 | 11% |
| 7 | 14% |
| 8 | 18% |
| 9 | 22% |
| 10 | 27% |
| 11 | 33% |
| 12 | 40% |
| 13 | 47% |
| >14 | >58% |

The individual's CHD risk can be compared to his or her population as follows.

**TABLE 6. CHD Comparison to Others Table (compared to man of the same age)**

| Age (years) | Average 10 Yr CHD Risk | Low 10 Yr CHD Risk |
|---|---|---|
| 30-34 | 3% | 2% |
| 35-39 | 5% | 3% |
| 40-44 | 7% | 4% |
| 45-49 | 11% | 4% |
| 50-54 | 14% | 6% |
| 55-59 | 16% | 7% |
| 60-64 | 21% | 9% |
| 65-69 | 25% | 11% |
| 70-74 | 30% | 14% |

Being overweight or obese increases the risk of developing high blood pressure and type 2 diabetes. Blood pressure (BP) rises as body weight increases. Studies have shown that losing even 10 pounds can lower the blood pressure and losing weight has the biggest effect on those who are overweight and already have hypertension. The two measures used to determine factors of overweight or obesity are body mass index, or BMI, and waist circumference. BMI is a measure of weight relative to height. It gives an approximation of total body fat. By motivating the patient to lose weight, his or her blood pressure improves.

Table 7 provides relative and absolute risk estimates for CHD in men as determined based on Framingham Score. The relative risk estimates for each age range are compared with baseline risk conferred by age alone (in the absence of other major risk factors). Relative risk is graded to include below average, average and moderately above average and high-risk categories. Distinctions in relative risk are arbitrary. Average risk refers to that observed in the Framingham population. Absolute risk estimates are given in the two right hand columns. Absolute risk is expressed as a percentage likelihood of developing CHD per decade. Total CHD risk equates to all forms of clinical CHD, whereas hard CHD includes clinical evidence of myocardial infarction and coronary death. Hard CHD estimates are approximated from the published Framingham Data.

**TABLE 7. Risk Assessment Table.**

| Age | 30-34 | 35-39 | 40-44 | 45-49 | 50-54 | 55-59 | 60-64 | 65-69 | 70-74 | | |
|---|---|---|---|---|---|---|---|---|---|---|---|
| Low | (2%) | (3%) | (4%) | (5%) | (6%) | (7%) | (8%) | (10%) | (13%) | Absolute Risk | Absolute |

| Risk Level | | | | | | | | | | | Risk |
|---|---|---|---|---|---|---|---|---|---|---|---|
| Points | | | | | | | | | | Total CHD | Hard CHD |
| 0 | 1.0 (1) | | | | | | | | | 2% | 2% |
| 1 | 1.5(2) | 1.0 | 1.0 | | | | | | | 3% | 2% |
| 2 | 2.0 (3) | 1.3(1) | 1.3 | 1.0 | | | | | | 4% | 3% |
| 3 | 2.5 (3) | 1.7(2) | 1.7(1) | 1.3 | 1.0 | | | | | 5% | 4% |
| 4 | 3.5 (3) | 2.3 (3) | 2.3 (2) | 1.8 | 1.4 | 1.0 | | | | 7% | 5% |
| 5 | 4.0 (4) | 2.6 (3) | 2.6 (2) | 2.0 (1) | 1.6 | 1.1 | 1.0 | | | 8% | 6% |
| 6 | 5.0 | 3.3 (3) | 3.3 (3) | 2.5 (2) | 2.0 (1) | 1.4 | 1.3 | 1.0 | | 10% | 7% |
| 7 | 6.5 | 4.3 (4) | 4.3 (4) | 3.3 (3) | 2.6 (2) | 1.9 (1) | 1.6 | 1.3 | 1.0 | 13% | 9% |
| 8 | 8.0 | 5.3 | 5.3 | 4.0 (4) | 3.2 (3) | 2.3 (2) | 2.0 (1) | 1.6 | 1.2 | 16% | 13% |
| 9 | 10.0 | 6.7 | 6.7 | 5.0 | 4.0 (4) | 2.9 (3) | 2.5 (2) | 2.0 (1) | 1.5 | 20% | 16% |
| 10 | 12.5 | 8.3 | 8.3 | 6.3 | 5.0 | 3.6 (4) | 3.1 (3) | 2.5 (2) | 1.9 (1) | 25% | 20% |
| 11 | 15.5 | 10.3 | 10.3 | 7.8 | 6.1 | 4.4 | 3.9 (4) | 3.1 (3) | 2.3 (2) | 31% | 25% |
| 12 | 18.5 | 12.3 | 12.3 | 9.3 | 7.4 | 5.2 | 4.6 | 3.7 (4) | 2.8 (3) | 37% | 30% |
| 12 | 22.5 | 15.0 | 15.0 | 11.3 | 9.0 | 6.4 | 5.6 | 4.5 | 5.3 (4) | 45% | 35% |
| >14 | 26.5 | >17.7 | >17.7 | >13.3 | >10.6 | >7.6 | >6.6 | >5.3 | >4.1 | >53% | >45% |
| | | | | | | | | | | | |
| Boundary markers for risk categories denoted in (): | | | | | | | | | | | |
| 1 = Below Average Risk; | | | | | | | | | | | |
| 2 = Average Risk; | | | | | | | | | | | |
| 3 = Moderately Above Average Risk; | | | | | | | | | | | |
| 4 = High Risk | | | | | | | | | | | |

| | | | | | | | | | | | |
|---|---|---|---|---|---|---|---|---|---|---|---|
| "Low Risk Level" provides the 10-year absolute risk for total CHD end points for persons in the age group with good blood pressure (<120/<80 mmHg.), total cholesterol (160-199 mm/dL.), HDL-C mg/dL., nonsmoker, and no diabetes. Points are the number of points accumulated from the Global Risk Assessment Scoring Chart provided above. The absolute risk columns represent the 10-year absolute risk for total CHD and hard CHD end points respectively and are estimated from Framingham Data corresponding to Framingham Score. | | | | | | | | | | | |

The Framingham Scoring System takes into account gradations in risk factors when estimating absolute risk. The scoring does not adequately account for severe abnormalities or risk factors, e.g. severe hypercholesterolemia, severe hypertension, uncontrolled diabetes, or cigarette smoking. In these cases, Framingham scores can underestimate absolute risk. This underestimation is particularly evident when only one risk factor is present. Thus, heavy smoking or severe hypercholesterolemia can lead to premature CHD even when the summed score for the absolute risk is not high. Similarly, the many dangers of prolonged, uncontrolled hypertension are well known. Thus, in embodiments, a physician is consulted to exercise subjective clinical acumen to control severe risk factors regardless of absolute short-term risk estimates. The Framingham Scoring System is merely one example of a system known in the art for estimating disease risk. Other systems are known and can be used with the subject invention.

In an embodiment, the protocol for selecting which "Multi Unit Drug Packaging" (MUDP) combination pill treatment or one-a-day treatment regimen formulation should be prescribed to a patient is based upon an initial screening and assessment of a patient. In an embodiment, this includes (1) measurement of serum levels of total cholesterol (or LDL-C) and HDL-C and evaluation of cholesterol disorders requires measurement of LDL-C, which is the primary target of cholesterol lowering therapy; and (2) measurement of blood pressure (regardless of whether the patient is taking antihypertensive drugs. The average of several blood pressure measurements can be used for an accurate determination of the baseline level pursuant the CDC recommended protocols.

Other factors such as the patient's age, ECG or EKG abnormalities, ABI tests, B-mode ultrasound of carotid, aorta and femoral arteries, ultrasound of carotid arterial intima and media thickness are also diagnostic tools that may be employed by the physician to determine which of the many one-a-day treatment regimens should be prescribed in the Multi Unit Drug Packaging System (MUDP) should be prescribed.

A patient's age is an indicator of absolute risk, because it reflects the total burden of atherosclerosis that has accumulated; the probability of suffering a major coronary event (unstable angina or myocardial infarction) is correlated with total plaque burden.

ECG or EKG abnormalities, such as abnormalities in the rest ECG, nonspecific ST-segment changes and left ventricular hypertrophy, also carry predictive power and can improve office-based risk assessment.

Noninvasive tests of atherosclerotic Burde Ankle-brachial blood pressure Index (ABI) is a simple diagnostic test for lower-extremity peripheral arterial disease (PAD). It is simply the ratio of blood pressure measured in the arteries at the foot or ankle (dorsalis pedis and posterior tibialis arteries, measured by a hand-held Doppler probe) to the blood pressure measured by traditional blood pressure cuff in the arm (brachial artery). Among well-trained operators, test-retest reliability is excellent and the validity of the test for =50% stenosis in leg arteries is high (90% sensitivity and 98% specificity). In population studies, patients with low ABI have been found to have a considerably higher prevalence of CVD (history of myocardial infarction, coronary artery bypass graft surgery, stroke or stroke surgery, or other measures of clinical CVD such as angina or congestive heart failure) compared to those with normal ABI. Such data confirm that atherosclerosis is a diffuse (i.e., systemic) disease and that an abnormal ABI test (ratio<0.90) suggests significant atherosclerosis in other vascular beds. At least 3 prospective studies have shown a strong predictive role for the ABI for CVD morbidity and mortality prediction in persons with PAD detected by ABI.

Many asymptomatic persons aged 50 and over will have abnormal ABI values. Follow-up studies have shown that abnormal ABI provides incremental coronary and all-CVD risk assessment information, over and above that provided by traditional risk factors. For example, in one study, an abnormal ABI increased relative risk for CVD mortality by nearly 4-fold over standard CV risk factors.

B-mode ultrasound is a relatively inexpensive and safe technique that visualizes the lumen and walls of selected arteries, including carotid, aorta, and femoral. B-mode ultrasound has been validated for measuring intima-media thickness (IMT). Cross-sectional associations between common carotid artery IMT and CVD risk factors have been demonstrated in several studies. Similarly, common carotid IMT has been associated with prevalent CVD in cross-sectional studies. At least 4 published studies show that carotid IMT measurement predicts the presence of CHD and its clinical sequelae.

Noninvasive measurements of the intima and media of the common and internal carotid arteries made with high-resolution ultrasonography can form a base for risk diagnostic purposes. The incidence of cardiovascular events has been correlated with measurements of carotid-artery intima-media thickness. The relative risk of myocardial infarction or stroke increased with intima-media thickness. The relative risk of myocardial infarction or stroke (adjusted for age and sex) for the quintile with the highest thickness as compared with the lowest quintile was 3.87 (95% confidence interval, 2.72 to 5.51). The association between cardiovascular events and intima-media thickness remained significant after adjustment for traditional risk factors, showing increasing risk for each quintile of combined intima-media thickness, from the second quintile (relative risk, 1.54, 95% confidence interval, 1.04 to 2.28), to the third (relative risk 1.84, 9r % confidence interval, 1.26 to 2.67), fourth (relative risk, 2.01; 95% confidence interval, 1.38 to 2.91) and fifth (relative risk, 3.15; 95% confidence interval, 2.19 to 4.52). The results of separate analyses of myocardial infarction and stroke paralleled those for the combined endpoint. The study showed that increases in the thickness of the intima and media of the carotid artery, as measured noninvasive by ultrasonography, are directly associated with an increased risk of myocardial infarction and stroke in older adults without a history of cardiovascular disease.

Prevention of clinical atherosclerotic sequelae (myocardial infarction, stroke and peripheral vascular disease) can involve modifying reversible risk factors such as systemic hypertension, dyslipidemia, hypertriglyceridemia, tobacco smoking and its consequent elevation of platelett aggregation, and excess body mass index. Studies have demonstrated that atherosclerosis begins at an early age and progresses in an asymptomatic manner over decades.

Patients at high-risk because of multiple risk factors may require intensive modification of risk factors to maximize risk reduction. These guidelines are currently endorsed or supported by various medical organizations and governmental bodies. The reports advocate adjusting the intensity of risk-factor management to the global risk of the patient. In certain reports, overall risk is estimated by adding the categorical risk factors. They do not use a total risk estimate based on summation of risk factors that have been graded according to risk severity. This latter approach is advocated by the Framingham investigators. Framingham reported that some clinicians believe that the summation of graded risk factors provides advantages over the addition of categorical risk factors. The use of graded risk factors has been recommended in risk-management guidelines developed in Europe.

There are several independent factors that can affect the selection and use of the "Multi Unit Drug Packaging" (MUDP) treatment regimen or one-a-day treatment regimen. These are: (a) Diabetes Mellitus (a major risk factor for CHD, both Type I and Type II); (b) Elderly Patients (a prominent feature of the Framingham risk score); (c) Hypertriglyceridemia (elevated serum triglycerides are independent risk factor and elevated triglycerides consequently become a target of therapy independent of LDL lowering); (d) Family History of Premature CHD (imparts an incremental risk at any level of global risk factors); (e) Psychosocial Factors (contribution of personality and socioeconomic factors such as economic standing, evocation, racial background, lifestyle, and personality type, increase CHD risk); and/or (f) Homocysteine (high serum concentration of homocysteine is associated with increased risk for CHD). Not all factors are used in all embodiments of the subject invention. Any subset may be selected or used, or in other embodiments none of these factors are used.

### Treatment of Diabetes with the MUDP treatment regimen

In an embodiment, the MUDP treatment regimen can also be used to delay the onset of type 2 diabetes or the progression of diabetic sequelae. The primordial risk factors for CHD are the same as those which cause targeted organ disease, such a type 2 diabetes, that is high blood pressure, cholesterol and triglyceride levels. Patients with type 2 diabetes often have high blood pressure and high cholesterol and are at increased risk of heart attack and stroke. With the MUDP treatment regimen, (a) patients with type 2 diabetes can have a greater medication compliance (b) can be less subject to the "sick patient" syndrome; (c) the cost of treatment should be less due to the reduced cost of medication (rather than take 4 - 5 medications, a MUDP with 4 - 5 medications is more cost effective); and/or (d) the total healthcare cost for the patient with type 2 diabetes can be reduced since the risk of heart attack and stroke and subsequent treatment therefore is reduced. Since patients with type 2 diabetes sometimes take several other medicaments each day, a reduction of 4 - 5 pills and the substitution of a MUDP treatment regimen can increase compliance and improve patient health. The MUDP treatment regimen can also reduce patient error involving taking the wrong pills in the wrong dosages.

### Primary Care Usages and Secondary Care Usage

The MUDP treatment regimen is effective both as a primary care treatment and as a secondary care treatment plan. Primary care is called for before the patient suffers from his or her first heart attack or stroke (prior to diagnosis of the CVD or CHD ailment and type 2 diabetes), or other condition. The risk factor assessment discussed herein enables the physician to employ the MUDP treatment regimen as a primary care treatment plan. The doctor and the patient can see improvements by titration of dosages and with the use of the treatment regimen Matrix discussed below.

Secondary care can also involve the use of the MUDP treatment regimen. After a heart attack or a stroke or onset of another condition, the MUDP treatment regimen may be used to titrate the correct dosages for the patient. After the patient leaves the health care facility, the MUDP treatment regimen is easier to prescribe and its use increases patient compliance. Further, patient memory loss, even on a temporary basis, sometimes reduces medication compliance. The MUDP treatment regimen improves compliance since the entire drug treatment regimen is contained in one easy to use package. In an embodiment, the primary care protocol also prescribes the use of a thrombolitic agent. The FDA has indicated that the thrombolitic agent, in the formulations set forth herein, may be taken 4, 8, 16 hours after a Heart Attack as Secondary care in conventional, conservative therapy. The primary care protocol has also been approved by the FDA in an IND Waiver for the use as a thrombolitic agent in the event of an impending cerebrovascular accident whose etiology is based on a stenosed artery due to the accumulation of plaque. A thrombolitic agent is generally not taken unless the target blood vessel is at least 66% occluded., i.e. Streptokinase or a new generation thrombolytic agent.

### MUDP Treatment Regimen, Clinical Protocol, Risk Factor Management

Having established the absolute risk of the patient to CHD, cerebrovascular disease and diabetic sequelae, the MUDP treatment regimen protocol can address the management of these independent modifiable risk factors.

In an embodiment of the present invention, diagnostic criteria of the Cerebrovascular and Cardiovascular status and diabetic sequelae status are used to establish a process to assure compliance and titration with administration of medication to maintain the risk factors within the parameters of a low risk state, and the administration of thrombolytics when clinically indicated. In an embodiment, upon establishment of absolute risk and selection of the patient for treatment, the patient is treated aggressively as follows. The patient is interviewed in detail with the objective of gleaning information concerning implementation of behavioral and lifestyle modification changes. i.e. diet, exercise and rest. The physician needs to establish a chemotherapeutic regimen prescriptive to the unique needs of the patient. Each modifiable risk factor, such as: hypertension, hypercholesterolemia, hypertriglyceridemia, blood glucose levels (diabetes) and the elevated aggregative potential of platelets caused by smoking, is assessed individually and treated.

Each of the medical compounds in the MUDP treatment regimen targeted at reducing the risk potential of the modifiable risk factors is prescribed by many medical protocols. In an embodiment, to improve the probability of compliance of taking prescribed medication at a specified time of the day, the MUDP treatment regimen protocol requires medication aimed at treating elevated BP (i.e., ACE INHIBITOR, B-Blocker, Diuretic, etc.), and medication aimed at treating hypercholestrolemia (i.e., A Statin drug compound - Lipator, Zocor, etc.), Elevated triglycerides (i.e., Tricor - Fenobibrate), type 2 diabetes (i.e. Insulin and/or Diabenese), and an anti-platelet aggregative medication (i.e., Plavix/Aspirin), be combined in one MUDP treatment regimen. The MUDP treatment regimen can encourage compliance and assist in eliminating the notions of "a sick person."

In an embodiment, the patient is evaluated at least monthly for the first three months for purposes of titration of dosage and monitoring of side effects for the medications. Titration of the medication is an essential and integral part managing potentially modifiable risk factors. In other embodiments, the patient is evaluated more or less frequently. For example, in an embodiment the patient is evaluated weekly for four to six weeks, or longer. Other useful frequencies can be suggested by one skilled in that are and can be used with the subject invention.

Since the risk assessment can vary for each patient and the patient's dosage for each element in the MUDP treatment regimen can change over time, it is helpful to present to the physician a matrix of available treatment regimens. As noted below, in an embodiment, the matrix is quite long and complex. In an embodiment, the designating indicia S-D-A-T adjacent the mg dosage also greatly assists the physician in selecting one of the treatment regimens. In an embodiment, upon reaching the physician's expectations of maximal management results based upon further visits over time and titration of dosage, office visits are then prescribed by the physician based upon individual patient histories.

An embodiment of the MUDP treatment regimen Matrix (below) is understood by the following example: First, each one-day MUDP Combination Medical Therapeutic Regimen has either aspirin or clopidogrel (a.k.a. Plavix) or both and each unique MUDP treatment regimen is designated with an S-D-A-T indicia code such as S10-D25-A10-T160 wherein S=Statin Drugs to lower cholesterol; D=Diuretic-a drug to reduce the water in the patient's body thus lowering his or her blood pressure; A=Ace Inhibitor-a hypertension drug that inhibits substances in the body from producing substances that cause high blood pressure (e.g., the following is a list of some Inhibitors that are available in the United States: captopril (CAPOTEN), benazepril (LOTENSIN), enalapril (VASOTEC), lisinopril (PRINIVIL, ZESTRIL) fosinopril (MONOPRIL), ramipril (ALTACE), perindopril (ACEON), quinapril (ACCUPRIL), moexipril (UNIVASC), and trandolapril (MAVIK)); T=TRICOR-a drug used to lower triglycerides and, to a small degree, cholesterol. TRICOR (fenofibrate tablets), is a lipid regulating agent available as tablets for oral administration; and the addition of one or more diabetic treatment drug compound. Each tablet contains fenofibrate. The chemical name for fenofibrate is 2-[4-(4-chlorobenzoyl)phenoxy]-2-methyl-propanoic acid, 1-methylethyl ester.

The number following the letter is the medicament's strength in milligrams. For example, S10-D25-A10-T160 is equivalent to S10 or 10 mg of a statin; D25 is 25 mg of a diuretic; A10 is 10 mg of an Ace Inhibitor; and T160 is 160 mg of TRICOR. People need various strengths of these widely prescribed drugs to control their risk factors, cholesterol, hypertension, and triglycerides. Rather than giving a person 4 or 5 pills in the differing dosages appropriate for a patient, in different prescriptions and in different packages, the drugs are provided in one MUDP treatment regimen. Commonly prescribed dosages of S-D-A-T are employed in the MUDP treatment regimen formulations of the Primary Matrix.

In an embodiment, the first ingredient is either Aspirin 325 mg or PLAVIX 75 mg or both. Aspirin is approved in one strength 325 mg and PLAVIX is approved in one strength 75 mg to keep blood platelets from sticking together. In an embodiment, the MUDP treatment regimen contains either 325 mg of aspirin or 75 mg of PLAVIX or both. PLAVIX (clopidogrel bisulfate) is an inhibitor of ADP-induced platelet aggregation acting by direct inhibition of adenosine diphosphate (ADP) binding to its receptor and of the subsequent ADP-mediated activation of the glycoprotein GPIIb/IIIa complex. Chemically it is methyl (+)-(S)-a-(2-chlorophenyl)-6,7-dihydrothieno [3,2-c]pyridine-5(4H)-acetate sulfate (1:1).

Ace Inhibitors block the formation of chemicals in the body that signal the body to increase blood pressure and increase heart rate in addition to the constriction of blood vessels. Beta blockers lower blood pressure.

To lower cholesterol, a patient can take a statin. Very few people need 80 mg of a statin. To lower blood pressure, a patient might take a diuretic, either 25 mg or 50 mg. Very few people would take 80 mg of a diuretic. A diuretic is the first line of defense to lower blood pressure. Diuretics help reduce excess water in blood and body tissue. High volumes of water cause the heart to pump harder, thereby increasing BP. An effective drug to lower blood pressure is an Ace Inhibitor. Patients can take 10 mg, 20 mg, or 40 mg of an Ace Inhibitor. About one third of the population has elevated triglycerides. To lower them, most patients can take a mid-level strength dose of TRICOR 160 mgs or a high strength of TRICOR 200 mg and a few take the small dose 67 mg. TRICOR reduces triglycerides in the blood by altering blood sugar levels. The strength of each drug is dependent on which drug in the class of drugs is used.

In an embodiment, the MUDP treatment regimen has aspirin and/or clopidogrel. In an embodiment, the formulations of statins considered have strengths of 10-20-40 and perhaps 80 mg. The MUDP treatment regimen can have ZOROR or LIPITOR but it is possible to use a treatment regimen with SIMVASTATIN or a range of other Ethical/Branded and Generic ACE Inhibitors can also be included in the treatment regimen. The Ace Inhibitor can be MONOPRIL, but others could be used are 10-20-40 mg also. In an embodiment, the diuretic is SPIRONOLACTONE at 25 or 50 mg. HYDROCHLOROTHIAZIDE can be used that comes in 25, 50 mg, or LASIX that comes in 20, 40, and 80 mg. Other branded and generic drugs, as well as diabetic drug treatment can be used in the MUDP treatment regimen. Other dosage levels than those mentioned here can also be used.

The term "commonly prescribed dosages" means those dosages that are customarily used by a wide percentage of the population designated to receive the particular drug. The term "unitized" means that the physician would recognize the typical unit dosage such as a statin at 10, 20, 40, 80 mg or any other dosage level that may be necessary for the patient. For example, in an embodiment, each MUDP treatment regimen formulation has a unitized dosage of the medicament in that the dosage listed "S10" is readily recognizable as a widely prescribed statin 10 mg dosage.

Spironolactone (common brand name ALDACTONE) and Triamterine are medications commonly known in medical practice as "potassium sparing" diuretics. Diuretics are used to remove a surplus of fluid from the body's bloodstream or tissues. It also acts as an aldosterone inhibitor (prevents salt retention), and is used to treat advanced heart failure when symptoms persist after other drug therapies are maximized. When it is used in this manner, it is not used as a diuretic to remove extra fluid from the body.

Hydrochlorothiazide is a diuretic and antihypertensive. It is the 3,4-dihydro derivative of chlorothiazide. Its chemical name is 6-chloro-3,4-dihydro-2H-1,2,4-benzothiadiazine-7-sulfonamide 1,1-dioxide. Its empirical formula is C₇H₈ClN₃O₄S₂.

LASIX is a diuretic which is an anthranilic acid derivative. Lasix for oral administration contains furosemide as the active ingredient and the following inactive ingredients: lactose monohydrate NF, magnesium stearate NF, starch NF, talc USP, and colloidal silicon dioxide NF. Chemically, it is 4-chloro-N-furfuryl-5-sulfamoylanthranilic acid. Furosemide is available as white tablets for oral administration in dosage strengths of 20, 40 and 80 mg.

### Overview of the Diagnostic and Prescriptive Criteria

In an embodiment, upon completion of the diagnosis and evaluation of the patients Cardiovascular and Cerebrovascular risk factors, as either recommended and delineated by the clinical protocol described or independently established by the diagnosing authority, the patient's customized risk parameters are established. These risk parameters specifies the patient's risk category as delineated by the clinical protocol used. In an embodiment, an objective of the clinical protocol is to reduce the Risk category to the "Lowest Possible Risk" for a Cardiovascular or Cerebrovascular event as specified by the clinical protocol or any other quantitative or qualitative system of measurement of risk factors.

In an embodiment, the Risk Factors delineate the patient's requisite therapeutic regimen as prescribed by the clinical protocol in the form of a MUDP. In an embodiment, the combinatory drug compounds prescribed are in accordance with the guidelines established by the US National Institute of Health (NIH), The Framingham Heart Study, US Center for Disease Control and Prevention (CDC), The US Food and Drug Administration (FDA), the American Heart Association (AHA), The American Medical Association (AMA), the American Diabetic Association (ADA), The United Sates Pharmacopeia, any other governmental or recognized entity or institution in the United Sates or other countries of the world, and/or any prescriptive guidelines utilized by the physician for the implementation of the clinical protocol in whole or in part.

The MUDP as delineated by the appropriate section of the clinical protocol prescriptive matrices (or any additions or changes approved by the criteria established above) empowers the disease management authority (physician, or other entity) to customize the patient's dose for each component of the MUDP.

### Optional Genetic Testing Component of the Clinical Protocol relating to the variation of Human Genes as applicable to drugs utilized by the Clinical Protocol.

Note that any genetic methodology or diagnostic criteria currently available or that may become available toward establishing an elevated level of diagnostic and / or prescriptive criteria can be used with the subject invention. For example, in an embodiment, the clinical protocol therapeutic regimen is in accordance with the recommendations of the NIH relating to the prescribing of Plavix (clopedrogrel) for cardiovascular and cerebrovascular patients.

In an embodiment, the clinical protocol recommends adherence to the prescriptive guidelines established by the NIH for Plavix as it relates to the Gene known as **CYP2C19.** In an embodiment, in the presence of a variation / mutation of the Gene **CYP2C19,** clinical protocol recommends use of Aspirin alternately or in combination with Clopidrogrel (Plavix), as advised by the prescribing authority.

**TABLE 8. Statin Drug Table (examples)**

| Trade Name | Generic Name | Sponsor |
|---|---|---|
| PRAVACHOL | Pravastatin | Bristol-Myers Squibb |
| MEVACOR | Lovastatin | Merck |
| ZOCOR | Simvastatin | Merck |
| LESCOL | Fluvastatin | Novartis |
| LIPITOR | Atorvastatin | Pfizer |
| BAYCOL | Cerivastatin | Bayer |
| CRESTOR | Rosuvastatin | Astra-Zeneca |
| ADVICOR | Lovastatin + extended Release Niacin | Kos Pharmaceutical |

In an embodiment, elevated blood pressure, the most prevalent risk factor, is identified as a "primordial risk factor" and is targeted by the MUDP treatment regimen with the chemotherapeutic management tool of a Diuretic and an ACE inhibitor. This is represented in the matrices described from the most prevalent dosage used to the least prevalent dosage used. This is categorized by row in each of the 4 columns.

In an embodiment, elevated cholesterol, the next primordial risk factor, is targeted by the use of a statin and is listed in 3 groups of 6 rows based on the most prevalent dosage used to the least prevalent dosage used.

In an embodiment, elevated triglycerides, the third most often treated risk factor, is listed in order of prevalence from left to right in columns A, B and C. TRICOR (Fenofibrate) is not prescribed in all cases shown, thus the need for column D.

In an embodiment, to aid in making the matrix easy to use, color coding can be employed. For example, the least prescribed drug strengths, for statins, i.e. 80 mg, and diuretics, also 80 mg, can be listed in an orange segment, rows 19 to 36 (matrix range D19-D36-A36-A19). The bottom orange segment might appear out of order, but ranks use according to Blood Pressure and then by most frequent matching Statins.

In an embodiment, aspirin can be prescribed at the dose of 325 mg or any dose level for the patients needs. In an embodiment, to facilitate the physician in selecting one of the many MUDP treatment regimens, Matrix I uses color coding of groups or pluralities of selected ones of the MUDP treatment regimens. In an embodiment, the color code employed is: highest use is RED, 2nd highest use is BLUE, 3^{rd} highest use is GREEN, and least used MUDP Combination Medical Therapeutic Regimen is colored ORANGE.

**TABLE 9.**

| **MUDP Treatment Regimen Matrix I** | | | | |
|---|---|---|---|---|
| ASPIRIN or CLOPEDROGREL, plus, STATIN, DIURETIC, ACE INHIBITOR, & With or Without TRICOR | | | | |
| Diabetic Treatment Drug(s) added for Patients with Type 2 Diabetes | | | | |
| | A | B | C | D |
| 1 | S10-D25-A10-T160 | S10-D25-A10-T200 | S10-D25-A10-T67 | S10-D25-A10 |
| 2 | S10-D25-A20-T160 | S10-D25-A20-T200 | S10-D25-A20-T67 | S10-D25-A20 |
| 3 | S10-D25-A40-T160 | S10-D25-A40-T200 | S10-D25-A40-T67 | S10-D25-A40 |
| 4 | S10-D50-A10-T160 | S10-D50-A10-T200 | S10-D50-A10-T67 | S10-D50-A10 |
| 5 | S10-D50-A20-T160 | S10-D50-A20-T200 | S10-D50-A20-T67 | S10-D50-A20 |
| 6 | S10-D50-A40-T160 | S10-D50-A40-T200 | S10-D50-A40-T67 | S10-D50-A40 |
| 7 | S20-D25-A10-T160 | S20-D25-A10-T200 | S20-D25-A10-T67 | S20-D25-A10 |
| 8 | S20-D25-A20-T160 | S20-D25-A20-T200 | S20-D25-A20-T67 | S20-D25-A20 |
| 9 | S20-D25-A40-T160 | S20-D25-A40-T200 | S20-D25-A40-T67 | S20-D25-A40 |
| 10 | S20-D50-A10-T160 | S20-D50-A10-T200 | S20-D50-A10-T67 | S20-D50-A10 |
| 11 | S20-D50-A20-T160 | S20-D50-A20-T200 | S20-D50-A20-T67 | S20-D50-A20 |
| 12 | S20-D50-A40-T160 | S20-D50-A40-T200 | S20-D50-A40-T67 | S20-D50-A40 |
| 13 | S40-D25-A10-T160 | S40-D25-A10-T200 | S40-D25-A10-T67 | S40-D25-A10 |
| 14 | S40-D25-A20-T160 | S40-D25-A20-T200 | S40-D25-A20-T67 | S40-D25-A20 |
| 15 | S40-D25-A40-T160 | S40-D25-A40-T200 | S40-D25-A40-T67 | S40-D25-A40 |
| 16 | S40-D50-A10-T160 | S40-D50-A10-T200 | S40-D50-A10-T67 | S40-D50-A10 |
| 17 | S40-D50-A20-T160 | S40-D50-A20-T200 | S40-D50-A20-T67 | S40-D50-A20 |
| 18 | S40-D50-A40-T160 | S40-D50-A40-T200 | S40-D50-A40-T67 | S40-D50-A40 |
| 19 | S80-D25-A10-T160 | S80-D25-A10-T200 | S80-D25-A10-T67 | S80-D25-A10 |
| 20 | S80-D25-A20-T160 | S80-D25-A20-T200 | S80-D25-A20-T67 | S80-D25-A20 |
| 21 | S80-D25-A40-T160 | S80-D25-A40-T200 | S80-D25-A40-T67 | S80-D25-A40 |
| 22 | S80-D50-A10-T160 | S80-D50-A10-T200 | S80-D50-A10-T67 | S80-D50-A10 |
| 23 | S80-D50-A20-T160 | S80-D50-A20-T200 | S80-D50-A20-T67 | S80-D50-A20 |
| 24 | S80-D50-A40-T160 | S80-D50-A40-T200 | S80-D50-A40-T67 | S80-D50-A40 |
| 25 | S10-D80-A10-T160 | S10-D80-A10-T200 | S10-D80-A10-T67 | S10-D80-A10 |
| 26 | S10-D80-A20-T160 | S10-D80-A20-T200 | S10-D80-A20-T67 | S10-D80-A20 |
| 27 | S10-D80-A40-T160 | S10-D80-A40-T200 | S10-D80-A40-T67 | S10-D80-A40 |
| 28 | S20-D80-A10-T160 | S20-D80-A10-T200 | S20-D80-A10-T67 | S20-D80-A10 |
| 29 | S20-D80-A20-T160 | S20-D80-A20-T200 | S20-D80-A20-T67 | S20-D80-A20 |
| 30 | S20-D80-A40-T160 | S20-D80-A40-T200 | S20-D80-A40-T67 | S20-D80-A40 |
| 31 | S40-D80-A10-T160 | S40-D80-A10-T200 | S40-D80-A10-T67 | S40-D80-A10 |
| 32 | S40-D80-A20-T160 | S40-D80-A20-T200 | S40-D80-A20-T67 | S40-D80-A20 |
| 33 | S40-D80-A40-T160 | S40-D80-A40-T200 | S40-D80-A40-T67 | S40-D80-A40 |
| 34 | S80-D80-A10-T160 | S80-D80-A10-T200 | S80-D80-A10-T67 | S80-D80-A10 |
| 35 | S80-D80-A20-T160 | S80-D80-A20-T200 | S80-D80-A20-T67 | S80-D80-A20 |
| 36 | S80-D80-A40-T160 | S80-D80-A40-T200 | S80-D80-A40-T67 | S80-D80-A40 |

| | | | | |
|---|---|---|---|---|
| Color code boundary chart: high usage is red A1-B1-B6-A6; blue, second highest usage C12-C12-C12-A12; green 3rd highest usage D1-D18-D18-A-18; orange least used dosages D19-D36-A36-A19 | | | | |

### MUDP Treatment Regimen Matrix II

In an embodiment, elevated Blood Pressure, the most prevalent risk factor, identified by the MUDP treatment regimen protocol as a "primordial risk factor", is targeted by the MUDP treatment regimen with the chemotherapeutic management tool of an ACE inhibitor without a diuretic. This option is for those physicians who choose not to use a diuretic for the management of elevated blood pressure, and is represented in the matrix from the most prevalent dosage used to the least prevalent dosage used. This is categorized by row in each of the 3 columns.

In an embodiment, elevated Cholesterol, the next primordial risk factor, is targeted by the use of a Statin and is listed in 4 groups of 3 rows based on the most prevalent dosage used to the least prevalent dosage used. The third most often treated risk factor, elevated Triglycerides, is listed in order of prevalence from left to right in columns E, F, and G. The least prescribed drug strengths, for statins, i.e. 80 mg, and diuretics, also 80 mg, are listed in the orange segment, rows 46 to 48. The bottom orange segment might appear out of order but ranks use by Blood Pressure and then by most frequent matching statins.

Color code for Matrix II: highest usage is RED, 2^{nd} highest usage is Blue, 3^{rd} highest use is Green, and least used is Orange.

**TABLE 10.**

| **MUDP Treatment Regimen Matrix II** | | | |
|---|---|---|---|
| ASPIRIN or CLOPEDROGREL plus, STATIN, ACE INHIBITOR, TRICOR | | | |
| Diabetic Treatment Drug(s) added for Diabetic Patients | | | |
| | E | F | G |
| 37 | S10-A10-T160 | S10-A10-T200 | S10-A10-T67 |
| 38 | S10-A20-T160 | S10-A20-T200 | S10-A20-T67 |
| 39 | S10-A40-T160 | S10-A40-T200 | S10-A40-T67 |
| 40 | S20-A10-T160 | S20-A10-T200 | S20-A10-T67 |
| 41 | S20-A20-T160 | S20-A20-T200 | S20-A20-T67 |
| 42 | S20-A40-T160 | S20-A40-T200 | S20-A40-T67 |
| 43 | S40-A10-T160 | S40-A10-T200 | S40-A10-T67 |
| 44 | S40-A20-T160 | S40-A20-T200 | S40-A20-T67 |
| 45 | S40-A40-T160 | S40-A40-T200 | S40-A40-T67 |
| 46 | S80-A10-T160 | S80-A10-T200 | S80-A10-T67 |
| 47 | S80-A20-T160 | S80-A20-T200 | S80-A20-T67 |
| 48 | S80-A40-T160 | S80-A40-T200 | S80-A40-T67 |

| | | | |
|---|---|---|---|
| Color code boundaries: red highest use E37-F37-F39-E39; blue 2nd highest use G37-G37-G42-E42; green 3rd highest use G43-G45-E45-E43; orange least used dosages E46-G46-G48-E48. | | | |

### MUDP Treatment Regimen Matrix III

In an embodiment, Matrix Three (3) is comprised of Aspirin or PLAVIX, plus various drug combinations to be considered to capture a wider share of the market at the fringe of the MUDP treatment regimen model. In an embodiment, Matrix Three combinations are for the occurrence of an individual risk factor or combinations of risk factors not commonly occurring.
TABLE 11.

**MUDP Treatment Regimen Matrix III Elevated Blood Pressure Only ASPIRIN or CLOPEDROGREL Plus: DIURETIC and an ACE INHIBITOR Diabetic Treatment Drug(s) added for Diabetic Patients**

| | H | I | J |
|---|---|---|---|
| 49 | D25-A10 | D25-A20 | D25-A40 |
| 50 | D50-A10 | D50-A20 | D50-A40 |
| 51 | D80-A10 | D80-A20 | D80-A40 |

**Elevated Blood Pressure and Elevated Triglycerides ASPIRIN or CLOPEDROGREL Plus DIURETIC, ACE INHIBITOR and TRICOR Diabetic Treatment Drug(s) added for Diabetic Patients**

| | K | L | M |
|---|---|---|---|
| 52 | D25-A10-T160 | D25-A10-T200 | D25-A10-T67 |
| 53 | D25-A20-T160 | D25-A20-T200 | D25-A20-T67 |
| 54 | D25-A40-T160 | D25-A40-T200 | D25-A40-T67 |
| 55 | D50-A10-T160 | D50-A10-T200 | D50-A10-T67 |
| 56 | D50-A20-T160 | D50-A20-T200 | D50-A20-T67 |
| 57 | D50-A40-T160 | D50-A40-T200 | D50-A40-T67 |
| 58 | D80-A10-T160 | D80-A10-T200 | D80-A10-T67 |
| 59 | D80-A20-T160 | D80-A20-T200 | D80-A20-T67 |
| 60 | D80-A40-T160 | D80-A40-T200 | D80-A40-T67 |

**Elevated Blood Pressure and Elevated Triglycerides ASPIRIN or CLOPEDROGREL Plus ACE INHIBITOR and TRICOR Diabetic Treatment Drug(s) added for Diabetic Patients**

| | N | O | P |
|---|---|---|---|
| 61 | A10-T160 | A10-T200 | A10-T67 |
| 62 | A20-T160 | A20-T200 | A20-T67 |
| 63 | A40-T160 | A40-T200 | A40-T67 |

**Elevated Blood Pressure and Elevated Cholesterol ASPIRIN or CLOPEDROGREL Plus STATIN and ACE INHIBITOR Diabetic Treatment Drug(s) added for Diabetic Patients**

| | Q | R | S |
|---|---|---|---|
| 64 | S10-A10 | S10-A20 | S10-A40 |
| 65 | S20-A10 | S20-A20 | S20-A40 |
| 66 | S40-A10 | S40-A20 | S40-A40 |
| 67 | S80-A10 | S80-A20 | S80-A40 |

**Elevated Blood Pressure and Elevated Cholesterol ASPIRIN or CLOPEDROGREL Plus STATIN and DIURETIC Diabetic Treatment Drug(s) added for Diabetic Patients**

| | T | U | V |
|---|---|---|---|
| 68 | S10-D25 | S10-D50 | S10-D80 |
| 69 | S20-D25 | S20-D50 | S20-D80 |
| 70 | S40-D25 | S40-D50 | S40-D80 |
| 71 | S80-D25 | S80-D50 | S80-D80 |

**Elevated Cholesterol and Elevated Triglycerides ASPIRIN or CLOPEDROGREL Plus STATIN and TRICOR Diabetic Treatment Drug(s) added for Diabetic Patients**

| | W | X | Y |
|---|---|---|---|
| 72 | S10-T160 | S10-T200 | S10-T67 |
| 73 | S20-T160 | S20-T200 | S20-T67 |
| 74 | S40-T160 | S40-T200 | S40-T67 |
| 75 | S80-T160 | S80-T200 | S80-T67 |

### MUDP treatment regimen Matrix IV

In an embodiment, Matrix Four (**4**) is comprised of Aspirin or Clopedrogrel, plus various drug combinations to be considered to capture a wider share of the market at the fringe of the MUDP combination treatment model. In an embodiment, MUDP treatment regimen Matrix Four combinations are for the occurrence of combinations of risk factors not commonly occurring or treatment regimens not commonly prescribed in the United States.
TABLE 12.

**Elevated Blood Pressure, Cholesterol and Triglycerides ASPIRIN or CLOPEDROGREL Plus: STATIN, DIURETIC and an TRICOR Diabetic Treatment Drug(s) added for Diabetic Patients**

| | AA | BB | CC |
|---|---|---|---|
| 76 | S10-D25-T160 | S10-D25-T200 | S10-D25-T67 |
| 77 | S10-D50-T160 | S10-D50-T200 | S10-D50-T67 |
| 78 | S20-D25-T160 | S20-D25-T200 | S20-D25-T67 |
| 79 | S20-D50-T160 | S20-D50-T200 | S20-D50-T67 |
| 80 | S40-D25-T160 | S40-D25-T200 | S40-D25-T67 |
| 81 | S40-D50-T160 | S40-D50-T200 | S40-D50-T67 |
| 82 | S80-D25-T160 | S80-D25-T200 | S80-D25-T67 |
| 83 | S80-D50-T160 | S80-D50-T200 | S80-D50-T67 |
| 84 | S10-D80-T160 | S10-D80-T200 | S10-D80-T67 |
| 85 | S20-D80-T160 | S20-D80-T200 | S20-D80-T67 |
| 86 | S40-D80-T160 | S40-D80-T200 | S40-D80-T67 |
| 87 | S80-D80-T160 | S80-D80-T200 | S80-D80-T67 |

**Elevated Blood Pressure and Elevated Triglycerides ASPIRIN or CLOPEDROGREL Plus DIURETIC, and TRICOR Diabetic Treatment Drug(s) added for Diabetic Patients**

| | DD | EE | FF |
|---|---|---|---|
| 88 | D25-T160 | D25-T200 | D25-T67 |
| 89 | D50-T160 | D50-T200 | D50-T67 |
| 90 | D25-T160 | D25-T200 | D25-T67 |
| 91 | D80-T160 | D80-T200 | D80-T67 |

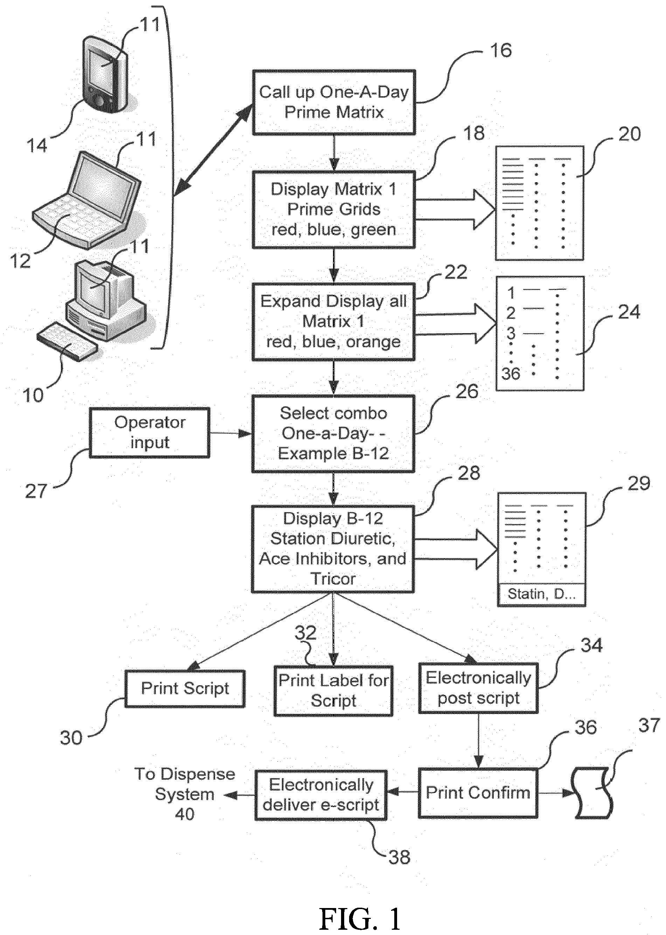

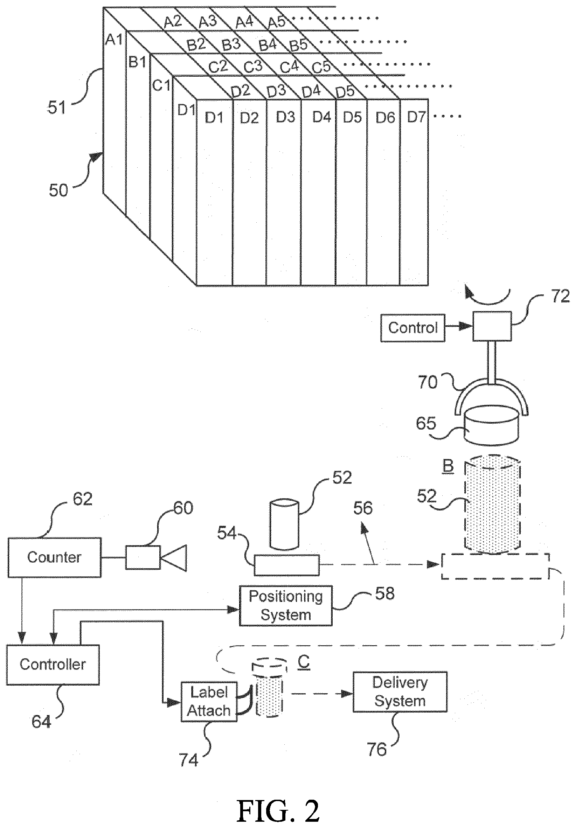

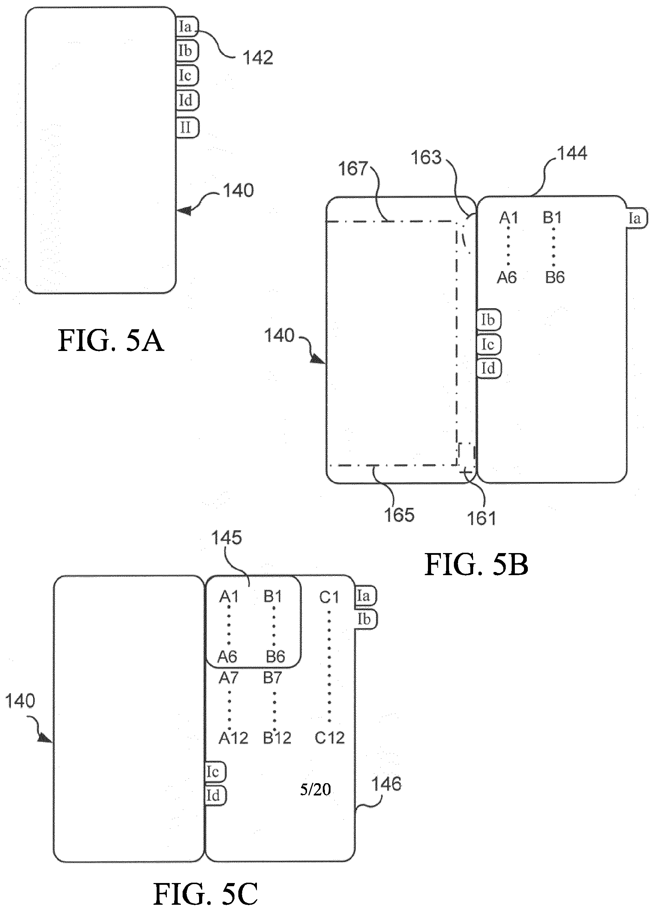

In an embodiment of the present invention, medicaments are delivered in a MUDP to be taken by the patient once a day or otherwise as prescribed. Previously, the patient would be prescribed 3-4 pills to be taken at various times during the day to improve the patient's health and reduce the patient's risk of CHD and CVD described above. In order to accomplish this selection activity by the healthcare provider, an information processing system is the subject of the present invention (shown in FIG. 1) as is a system for selection of one of the large plurality of MUDP treatment regimens (shown in FIGS. 5A-5E, 6A-6C and 7). Since the dispensing of a MUDP treatment regimen from a large plurality of MUDP treatment regimens is challenging, a dispensing system is diagrammatically illustrated in FIGS. 2-4. Similar numerals designate similar items throughout the drawings.

FIG. 1 diagrammatically illustrates an information processing system for selecting a treatment regimen from a plurality of treatment regimens in accordance with an embodiment of the subject invention. In an embodiment, each treatment regimen includes different formulations of commonly prescribed medicaments in commonly prescribed or unitized dosages. In an embodiment, at least some of the treatment regimens also include other prescribed activities such as tests or exercises. In and embodiment, the information processing system enables the physician to select one of a large plurality of MUDP treatment regimens. The information processing system can be employed over any type of computer system. In an embodiment, the computer system includes a memory, a display screen and operator input controls. As discussed above, various networks can be employed, including, but not limited to, Local area networks, wide area networks, wireless networks, and the Internet. In FIG. 1, a personal computer system **10** is shown, a laptop **12** is shown and a personal data assistant or PDA **14** is shown. These systems can have display screens **11** and include some type of operator input. The keyboard and mouse input on laptop **12** and on personal computer **10** may also include a touch screen input device as is common in PDA **14,** display **11.** In any event, in functional step **16,** a physician, other healthcare provider, or other user refers to a data chart for the MUDP treatment regimen primary matrix. The MUDP treatment regimen Matrices I, II, III and IV can be stored in memory. Functional step **18** displays a matrix I (or a primary grid portion thereof). In an embodiment, the primary grids are displayed in red, blue, and green as described. The primary grids shown in red, blue and green colors are listed at treatment regimen Matrix I, column and row A-I, B-1, B-6 and A-6 (grid 1-red); secondary group in blue shown in matrix I at C-I, C-12, A-12 andA-7; and tertiary group without TRICOR in matrix I, matrix coordinates D-I, D-18, A-18 and A-13. This is shown diagrammatically in the figure as a matrix with subgroup red, blue and green **20.** The display has matrix subgroups red, blue and green (see matrix boundary markers above) which enables the physician to select commonly prescribed medications, in commonly prescribed dosages, in a MUDP treatment regimen. Also, upon repeat visits, the physician can titrate dosages using the matrix to optimize the patient's health. Further, the healthcare provider can use the matrix with the patient as an educational and motivational tool to motivate the patient to titrate dosages to sub matrix or primary red grid AI, B1, B6, A6.

In functional step **22,** the healthcare provider expands the display to include the entire MUDP Treatment Regimen Matrix I. In an embodiment, subgroups are displayed in red, blue, green, and orange. The display is shown in FIG. 1 as display **24** which includes the red grid, blue grid and green grid as well as the orange, least used group of treatment regimens from range A-19, D-19, D-36 and A-36. The information system can quickly shift displays from the primary grid to the secondary, tertiary and full matrix displays for titration purposes and educational purposes.

In functional step **26,** the operator has an input (**27**) which selects one of the plurality of MUDP treatment regimens such as that listed at grid location B-12. As shown in the matrix I, above, at B-12, in the blue subgroup, the matrix display shows S-20-D50-A40-T200 which represents 20 mg of a statin, 50 mg of diuretic, 40 mg of ace inhibitor and 200 mg of TRICOR/FENOFIBRATE. The use of the abbreviations S-D-A-T also enable the physician to review many of the combinatory drugs and quickly select one. The indicia S-D-A-T greatly assists in the selection as does the mg dosage next to the letter indicia. In functional step **28,** the system, upon operator selection, displays the full formula for the selected MUDP treatment regimen combinatory drug at grid location B-12. Display screen **29** is shown in FIG. 1. From functional step **28,** the information processing system can provide various outputs, one of them being a print script or print prescription function **30,** a second being a print label function showing the script as a label but not the entire script prescription pad at functional step **32,** and also electronically posting the script at function **34.** In function **36,** the system prints a confirmation on paper **37** to show the selected one of the plurality of MUDP treatment regimens. In step **38,** the system electronically delivers the electronic script to a dispensing system **40.** Optionally, matrix grid portions may be printed for reference or to motivate the patient.

With respect to the information processing system, the groupings of sub-pluralities of MUDP treatment regimen formulations can be stored in memory in one of the electronic devices **10, 12,** and **14.** Of course, a dumb terminal may be utilized rather than a completely independent functional personal computer **10.** Further, PDA **14** may be linked via wireless network connection to a main frame or server computer. The output generator is a combination of the touch screen display or the keypad or the mouse in electronic devices **10, 12,** and **14** in addition to either a printer output or print script function **30** (also print label **32**) or an electronic output as noted in electronic posting of the script in function **34.** The output functions **30, 32, 34** may be local or near PDA **14** or may be remote with respect to display screens **11.** Or the function described can be otherwise distributed across a network as described above.

FIGS. 2-4 each diagrammatically illustrate a dispensing system for a plurality of medicaments in accordance with an embodiment of the subject invention.

With respect to the embodiment of FIG. 2, the dispensing system includes a plurality of containers **50** wherein each respective container, for example container **51,** retains one MUDP formulation. For ease explanation, these containers are marked A1, A2, A3, A4, A5, D 1, D2, D3, D4, C1 ... and B1... These labels are shorthand designations for the formulations in MUDP Matrix I and relate to the column and row designators. However, other types of designators may be utilized including the S-D-A-T indicators. If the S-D-A-T indicia are used on the MUDP and used on storage containers **50,** the dispensing operator could easily check and verify inventory. Since a large plurality of MUDPs is available to the consumer, a dispensing system confirming the selected MUDP is an objective of an embodiment of the present invention. Below each respective storage container **51** is a door or a MUDP dispensing opening with a control latch.

Supply container **52** is mounted, in the illustrated embodiment, on positioning trolley **54** which is adapted to move in the x-y direction **56** beneath the plurality of respective storage containers **50.** A positioning system controller **58** controls positioner **54** and hence supply container **52.** A sensing system **60** coupled to a counter detector **52** feeds control MUDP count signals into controller **64.** Controller **64** also provides controlling signal to positioning system **58** thereby moving supply container **52** beneath the appropriate respective storage container **51.** After the latch or door is open beneath the storage container **51** and MUDPs having a single formulation are dispensed into supply container **52** (subject to sensor **60** and counter **62**), supply container **52** is moved to position B wherein cap **65** is attached to the supply container **52.** Cap retainer **70** is rotated by drive motor **72** under a control signal from controller **64.** Thereafter, supply container **52** with cap **65** is moved to position C wherein label attachment system **74** attaches the label that matches the respective storage container **51** established by controller **64.** After position C, the supply container **52** is delivered to delivery system **76** which ultimately delivers the product to the consumer. Although a cylindrical container and cap are presented herein various shapes and configurations can be used for supply container **52.** In an embodiment, a shape and configuration is chosen that is well adapted to hold the MUDPs used. In an embodiment, the supply container **52** is closed by means other than a cap which is twisted on.

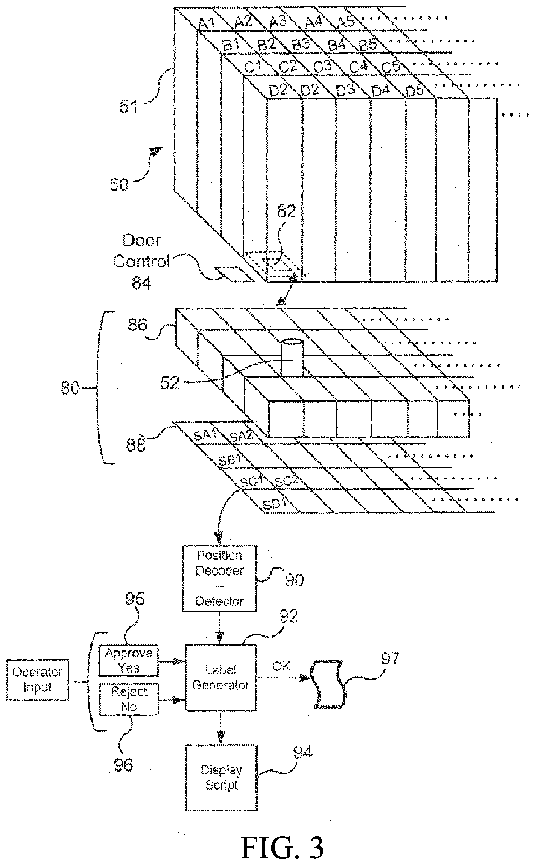

FIG. 3 diagrammatically shows another dispensing system having respective storage containers for each formulation (see plurality of storage containers **50**), and a control dispensing interface **80** that differs from the dispensing interface in FIG. 2. The interface in FIG. 2 includes position system **58,** positioner **54** and controller **64.** It should be noted with respect to both FIG. 2 and FIG. 3, rather than moving the supply container **52,** the plurality of storage containers **50** may be moved. The important point is that the storage container for the selected MUDP is placed above or adjacent the supply container **52** such that the selected MUDP as ordered by the healthcare provider are dispensed from the respective container into the empty supply container **52.** Intermediate hoses or chutes may be interposed between supply container **52** and the chosen, selected storage container. The chutes or hoses may be moved rather than the storage rack.

In FIG. 3, each of the respective storage chambers **50,** each having a single MUDP formulation, includes a dispensing port **82** that is opened or closed via a door or latch under door control **84.** Door control **84** is subject to control signals from a controller (not shown) similar to controller **64** in FIG. 2. In FIG. 3, the supply container **52** is put in an open top grid structure **86** which has beneath it a plurality of sensors **88.** When the MUDPs from storage container C2 (for example) are loaded into supply container **52,** sensor SC2 is activated thereby indicating that the MUDPs have been dispensed from dispenser storage container C2. Sensor SC2 may also weigh the supply container **52** to sense the condition (e.g., empty, partly full, or full) or number of MUDPs in the container. Position decoding detector **90** decodes the signal from the sensor grid **88.** The output from positioned decoder/detector **90** is fed to label generator **92.** The label generator is connected to a display unit **94** which shows the storage container which dispensed the MUDPs into supply container **52.** The operator or pharmacist operating the dispensing system in FIG. 3 should confirm that the displayed formulation on screen **94** conforms to the written prescription related to the patient. The operator then provides an input to approve the MUDP dispensing or reject the dispensing operation as noted in operator input **95, 96.** If rejected, a label is not generated and the MUDPs are not released. If the dispensing process has been approved by the pharmacist or healthcare worker, a label **97** is generated by label generator **92.** The label is then attached to supply container **52.** An automated label attachment sub-system may be incorporated with label generator **92.**

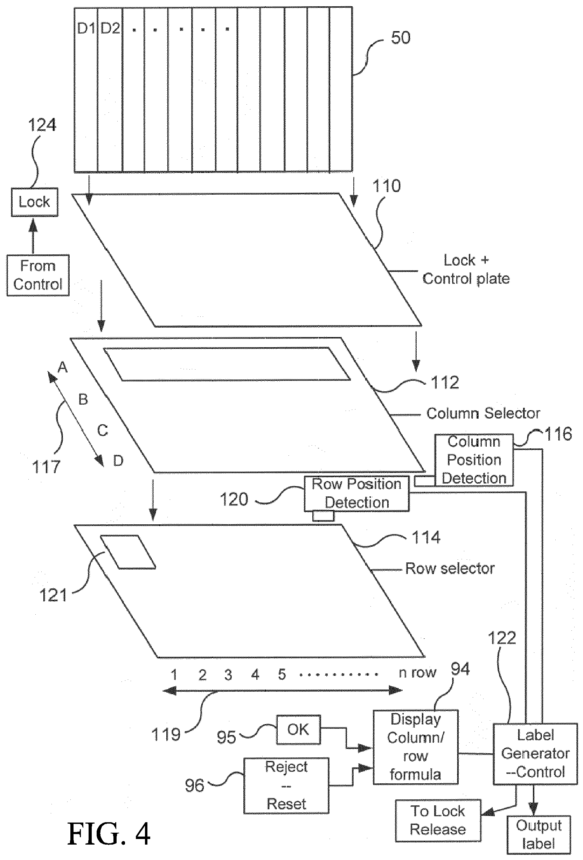

FIG. 4 shows another dispensing system which includes a three panel controlled MUDP dispensing interface consisting of lock and control plate **110,** column selector plate **112** and row selector plate **114.** In operation, a control unit (similar to control unit **64** in FIG. 2) moves column selector **112** to the appropriate column A, B, C, D (or otherwise) and the position of column selector **112** is detected and recorded by column position detector **116.** The opening in column plate **112** is placed beneath the selected storage container. The column selector moves in the direction of double headed arrow **117.** Row selector **114** is then moved to the particular row such as row and column D2 and under the selected storage container from the plurality of containers **50.** Row selector **114** moves in direction shown in double headed arrow **119.** The position of row selector **114** is detected by row position detector **120.** When the correct column and row is detected by position detectors **116, 120,** these data signals are applied to label generator and control **122.** The label generator and control **122** generates a lock release signal which is applied to lock unit **124.** Lock unit **124** then releases the lock and control plate **110** and moves the lock and control plate away from the plurality of MUDP storage containers **50.** Otherwise, lock plate may be moved manually away to expose the open row and column. This permits the one selected storage container to dispense MUDPs since a particular row has been selected by row selector **116** and column has been selected by column selector **112.** Label generator and control **122** generates a label for the supply container which is located beneath space **121** in row selector **114.**

As a safety precaution, display unit **94** lists the row and column as well as the selected formula. The healthcare provider must approve or reject the selected row and formula prior to the release of lock **124,** the movement of control plate **110** and the production of the label from the label generator control **122.**

FIGS. 5A-5E and 6A-6C and 7 show various systems for selecting one of the plurality of MUDP treatment regimens from various substrates which display various matrices and portions of Matrix I. See the red, green, blue and orange subgroups in Matrix I discussed above. In FIG. 5A, booklet **140** has a plurality of tabs **142** which extend from the side, top or bottom. Each tab is labeled with an identifier for the subgroup of treatment regimen Matrices such as Matrix la which distinguishes the subgroup at MUDP treatment regimen Matrix I grid coordinates A-I, B-1, B-6 and A-6. Tab Ib shows the matrix of the first and the second matrix subgroups (red and green) thereby showing the physician grid group AI, C1, C12 and A12. By providing a selection system which lists the treatment regimens in a particular subgroup, this greatly assists the physician or healthcare provider to select one of the identified treatment regimens and also provides a motivational tool for the patient to move from one lower grid into a higher quality grid such as moving from matrix grid region Ib to grid region la. Color coding also provides patient motivation, patient education and dosage titration assistance to the physician. Since titration or the gradual change of medication dosage is contemplated by this invention, grid subgroups and matrix displays are quite helpful to the physician. FIG. 5B shows booklet **140** with a pullout sleeve **144** associated with the tab la. The printed subpart substrate shows the primary grid AI, B1, B6 and A6 MUDP treatment regimen formulations. FIG. 5C shows a second printed subpart substrate with the treatment regimen grid pattern C1, C12, A12 and A7 (secondary sub-matrix). A window **145** on printed substrate **146** shows the underlying or primary grid AI, B1, B6, A6 which is the lowest formulation for the MUDP treatment regimen.

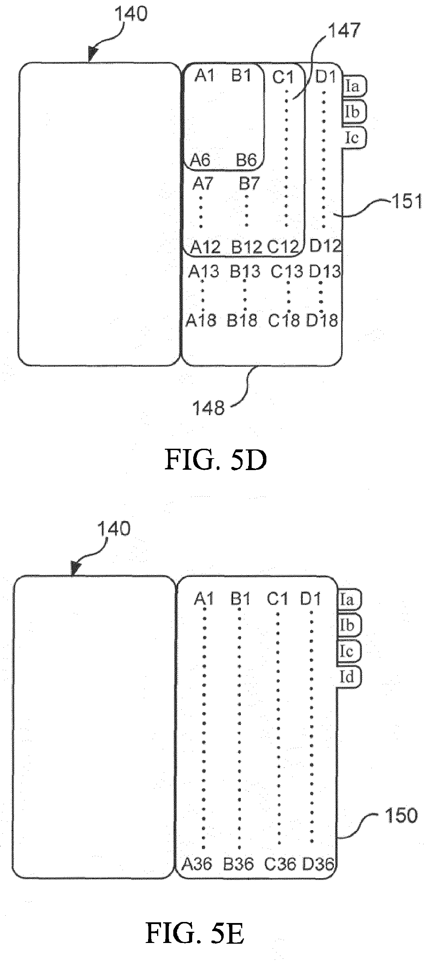

FIG. 5D shows that subpart substrate **148** includes a larger cutout **147** which shows the second grid matrix CI, C12, A12, A7. The next larger grid matrix D1, D18, A18, A13 (tertiary submatrix) is printed as indicia on surface **151** of subpart substrate **148.** Windows or openings in substrates **144, 146** permit the user-healthcare provider to titrate dosages.

In FIG. 5E, the printed substrate **150** shows the entire matrix I. This permits the doctor to titrate dosages and show these dosages as he titrates the medication through the various grid levels.

The substrates shown in FIGS. 5A-5E are movable with respect to each other based upon outboard tabs **161, 163,** slidably movable in slots **165, 167.** The tab in slot slide system in FIGS. 5A-5E operates on substrates **144, 146.** Other movable systems permitting movement of the printed substrates having indicia of the subpluralities and formulations can be utilized.

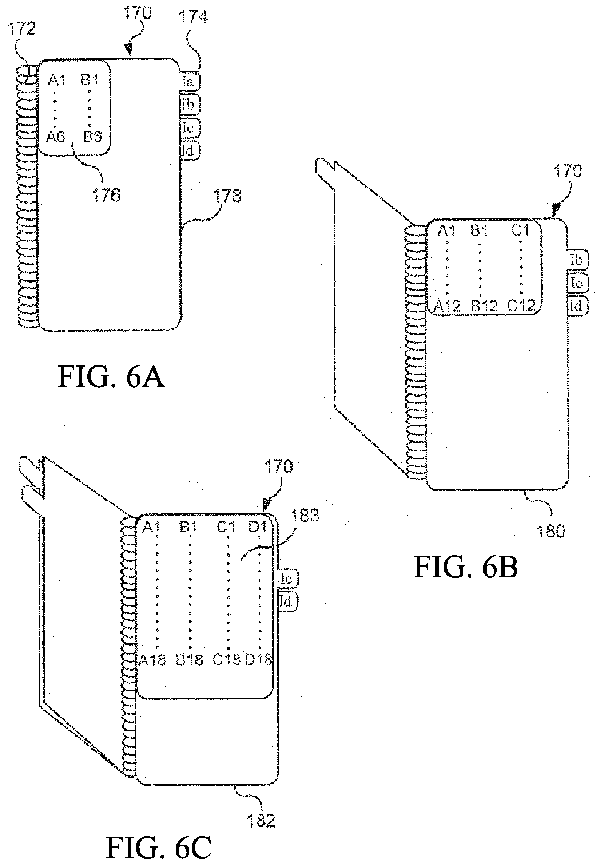

FIGS. 6A-6C show a booklet **170** with hinges, spiral, or other binding **172.** The bound substrates have tabs **174** each marked with indicia representing both the MUDP treatment regimen matrix as well as the subpart of the matrix. Therefore, matrix sub grid la is displayed by the use of tab **174** and primary sub grid pattern AI, BI, B6, A6 is shown in window **176** of substrate card **178.** With respect to FIG. 6B, substrate card **180** is keyed to secondary sub matrix grid portion 1b which shows grid pattern AI, C1, C12, A12. Card **182** associated with the indicia for tertiary matrix sub-group Ic has a cutout or window **183** permitting the view of sub grid AI, D1, D18, A18. A similar system can be employed for the other matrixes and sub grids.

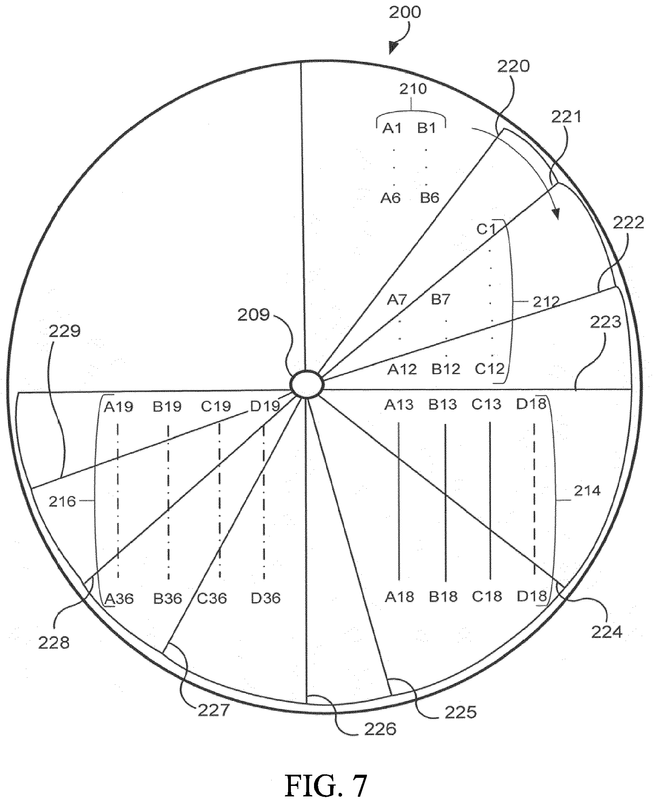

The selection system shown in FIG. 7 is a generally circular or oval substrate **200** having printed thereon portions of Matrix I. In region **210,** indicia representing the first sub grid AI, B1, B6, A6 is shown. In region **212,** the sub grid C1, C12, A12, A7 is shown. In substrate region **214,** sub grid A13, D1, D18, A18 is shown. In substrate region **216,** treatment regimens at subgrid A19, D19, D36, A36 are shown. Movable slides **220-229** cover all or substantially all of these grid indicia. In order to expose substrate portion **210,** as well as substrate portion **212,** rotatable fan collapsible elements **220, 221, 222,** and **223** collapse on top of each other about rotation point **209.** In order to expose the sub grid region and printed sub grid portion **214,** slidable elements **220-226** are slid and rotated such that they lay adjacent on top of each other. In order to expose the entire grid, the user moves slidable elements **226, 227, 228,** and **229** thereby exposing all of Matrix 1.

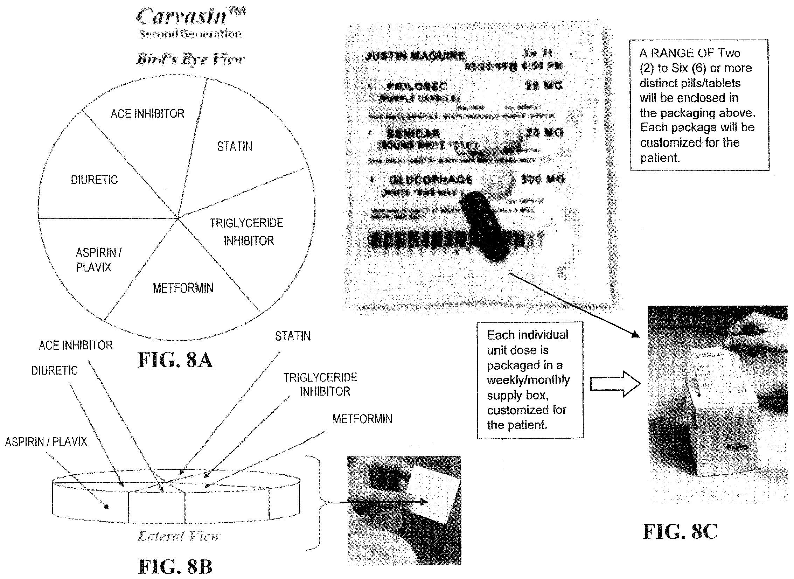

FIGS. 8A-8C illustrate various apparatuses for packaging multiple medicaments for convenient dosing according to embodiments of the subject invention. FIG. 8A illustrates a bird's eye view (top view) and FIG. 8B illustrates a lateral view of a packaging apparatus for a medicament according to an embodiment of the subject invention. The representation illustrates the separation of each pharmaceutical into a "pie-shaped" section or pocket. In an embodiment, each pharmaceutical pill or tablet is separated from the adjacent pill or tablet by a thin plastic partition and the whole packaging element comprises a Multi Unit Dose Package, or "MUDP." In FIG. 8C, the MUDPs comprising the customized and separated pharmaceutical prescriptions are packaged individually and then packaged into a multi-unit supply box for delivery to the patient. In the embodiment shown, the MUDPs can include two to six pills and the supply box includes a week or month-long supply. In another embodiment, the MUDPs can include one pill comprising multiple medicaments. In another embodiment, the MUDPs can include more than six pills. In another embodiment, the supply box can include less than a week's supply. In another embodiment, the supply box can include more than a week's supply. In a particular embodiment, the supply box contains a three-month supply of medicaments.

In an embodiment, the subject invention comprises a packaging system intended to increase patient compliance and improve efficacy of treatment for disorders such as hypertension, hypercholesterolemia, hypertriglyceridemia, anti-platelet aggregation, type 2 diabetes and related health issues, among other medical conditions or health problems. As shown in Figure 8, the packaging system includes drug treatments such as aspirin and/or Clopidrogrel (also known as PLAVIX), diuretics, ACE inhibitors, statins, triglyceride inhibitors, and metformin and/or diabenase, and/or any other drug compound that may manage the Diabetic Risk Factor(s) created by hyperglycemia and/or Insulin resistance. The packaging system enables the physician or healthcare provider to deliver a plurality of pills in a "Multi Unit Dose Package" (MUDP). The packaging system of the subject invention comprises a pouch that in embodiments has multiple indented "pie-shaped" pockets. Each pie-shaped pocket is separated by the plastic packaging material of the MUDP. The packaging system can contain multiple pills, preferably in different colors, in the pie-shaped arrangement that can appear as one multicolored pill when packaged. Therefore, the physician or healthcare provider can prescribe multiple pills based on the determined customary therapeutic needs of the individual under treatment based on a clinical protocol and/or combination therapy matrices. The MUDP treatment regimens thus deliver a complete drug treatment dosage in one easy-to-use package that greatly increases patient drug therapy compliance. Concurrent use of the clinical protocol and combination therapy matrices described also permit simplified titration of dosages to create new drug treatment regimens as a patient's needs change. To accommodate the changing therapeutic needs, new or titrated MUDP treatment regimens may be readily created for delivery to patients without disruption to the patient's level of compliance in the drug therapy.

FIG. 8 also illustrates methods of delivery for the MUDP treatment regimen supply boxes over weekly, monthly, quarterly, or longer periods of time based upon the condition of the patient. The multi-day treatment supply boxes comprising MUDP treatment regimens based on a prescribed drug treatment regimen are thus packaged to further improve patient compliance. The use of a MUDP treatment regimen for the individual's pills with various commonly prescribed dosages of preventive medications greatly enhances compliance. Most people remember to take a single dose each day. Similarly, the combination of pills into a MUDP treatment regimen can greatly increase compliance and the overall health of the patient.

Embodiments of the present invention relate to a diagnostic criteria, medical regimen prescribing system, and packaging systems aimed at improving patient compliance with prescribed drug treatment regimens. Embodiments of the subject invention are meant to target various disorders, conditions, health issues, and/or physiological systems including, but not limited to: Cerebrovascular and Cardiovascular Systems; Diabetes; Gastrointestinal System (GI); Urogenital System, including the Gaul Bladder, and Kidneys; Respiratory System; Neurological System; Endocrine System; Reproductive System; Dermatologic Diseases; and/or Electrolyte and Fluid Systems of the Body. In embodiments, the packing, distribution, and/or compliance monitoring systems of the subject invention can be used to package, distribute, and/or monitor compliance with various commonly used medicaments. In a particular embodiment, the invention is used to package, distribute, and/or monitor compliance with birth control medicaments.

Either the multi-pocketed pouch or pouch containing multi drug combinations of the subject invention creates the "Multi Unit Dose Package." The Multi Unit Dose Package permits the easy prescription of various drugs at interchangeable dosages by the physician or health care provider. The Multi Unit Dose Package (MUDP) also improves patient compliance (an issue with many patients in multi-drug treatment) with the prescribed drug treatment regimen. The packaging system and MUDP also permits the ready packaging and delivery of long-term drug treatment by using MUDP supply boxes. The long-term packaging also works to improve patient compliance with drug treatment.

The following diagnostic criteria is an example of a diagnostic criteria which can be used with the subject invention; however, the systems and methods of the subject invention can be implemented with other diagnostic criteria. In an embodiment, the objective of the diagnostic criteria is to reduce and maintain the patient's Cardiovascular and Cerebrovascular Risk to a "low risk state." Various definitions can be used for the low risk state. In an embodiment, the low risk state is defined as indicated in table 13.

**TABLE 13.**

| DEFINITION OF A LOW RISK STATE | |
|---|---|
| Total Serum Cholesterol | 160 - 199 mg / dL |
| Low Density Lipoproteins - C | 100 - 129 mg / dL |
| High Density Lipoproteins - C | 45 mg / dL in Men and 55 mg / dL. In Women |
| Blood Pressure | ≤ 115 mm Hg. Systolic and≤75 mm Hg. Diastolic |
| Nonsmoker | Maintain abstinence |
| Control of Diabetes | HbA1 c ≤ 6% and Glucose levels between 70mg and 150 mg. |
| Blood Triglycerides | ≤150 mg/dL |

| | |
|---|---|
| Other definitions of the low risk state can be selected by one skilled in the art and used with the subject invention. | |

Various diagnostic modalities can be used with the subject invention to assess patient health. By way of example, the following modalities, among others, can be used:
1. Patient's Age;
2. ECG and EKG abnormalities;
3. Noninvasive Tests of Atherosclerotic Burden ;
4. Ankle-Brachial Blood Pressure Index;
5. Arterial B-Mode Ultrasound;
6. Status of Aortic atherosclerosis;
7. Status of Total Serum Cholesterol;
8. Status of Low Density Lypoproteins-C;
9. Status of High Density Lypoproteins - C;
10. Blood Pressure;
11. Control of Diabetes;
12. Blood Triglycerides;
13. Status of Homocysteine;
14. Imaging of the Heart, Brain, Aorta, Carotids, and other organs including, but not limited to:
   a. CT Scan;
   b. MRI; and
   c. Ultrasound.

In an embodiment, CHD is managed in accordance with severity of condition determined by imaging including, but not limited to, Plaque formation, constriction of artery, etc.

In an embodiment, various risk factors are assessed as part the diagnostic criteria. Such risk factors include, but are not limited to, predisposing risk factors, conditional risk factors, and controllable risk factors. Nonexclusive examples of these types of risk factors are provided in table 14 and 15.

**TABLE 14.**

| PREDISPOSING RISK FACTORS | CONDITIONAL RISK FACTORS |
|---|---|
| Obesity | Elevated Serum Triglycerides |
| Abnormal Obesity | Elevated LDL particles |
| Physical Inactivity | Elevated Serum Homocysteine |
| Family History of Premature Heart Disease | Elevated Serum Lypoprotein "a" |
| Ethnic Characteristics | Prothrombotic Factors (i.e. Fibrinogen) |
| Psychosocial Factors | Inflammatory Markers (i.e. C-Reactive Protein) |

**TABLE 15.**

| CONTROLLABLE RISK FACTORS | |
|---|---|
| VITAL FACTOR | MANAGEMENT |
| High Blood Pressure | ≤ 115 mm Hg. Systolic and≤75 mm Hg. Diastolic |
| Abnormal Cholesterol | 160 - 199 mg / dL. |
| Abnormal Triglycerides | ≤ 150 mg/dL |
| Tobacco Use | Abstinence of tobacco use: |
| | - Health Insurance Incentives |
| | - Family and Community Involvement |
| | - Governmental Support |
| Diabetes | HbA1c ≤ 6% |
| | Glucose levels between 70 mg and 150 mg |
| Overweight | Body weights are currently defined according to BMI as follows: Normal weight 18.5 - 24.9 Kg/m² |
| Physical Inactivity | Daily exercise |

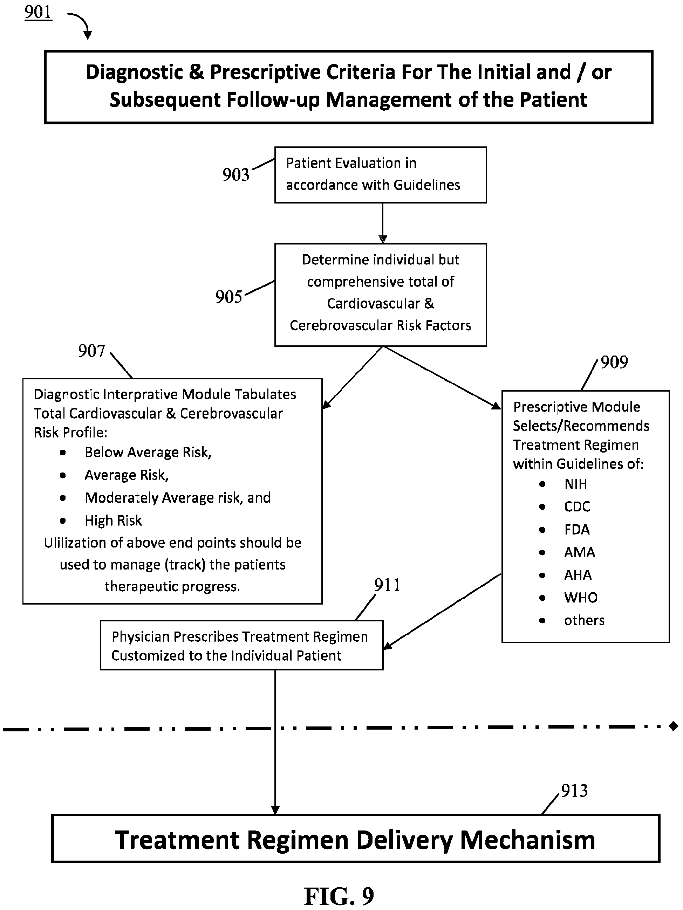

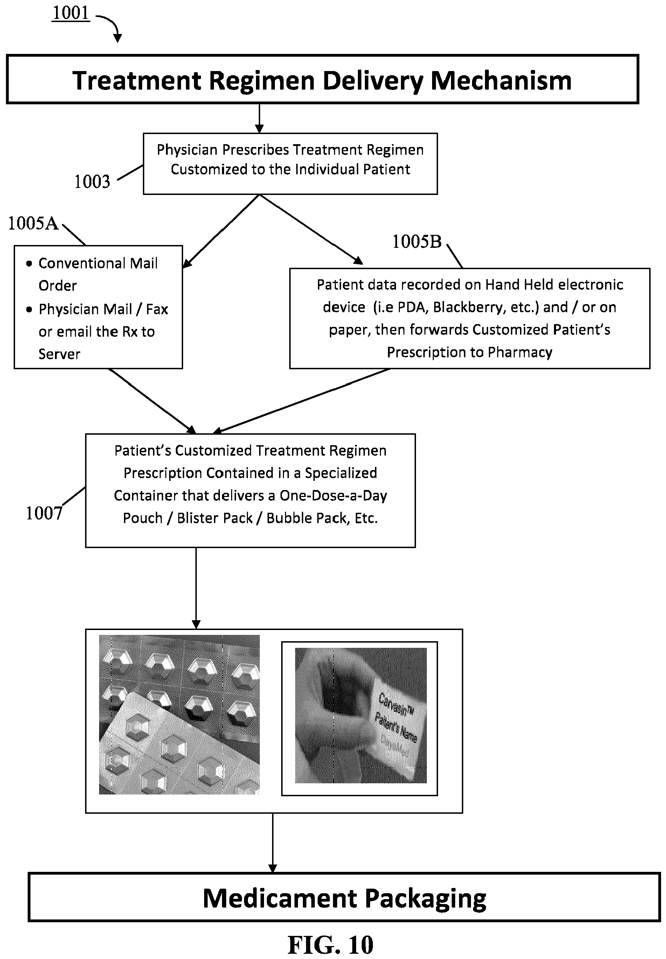

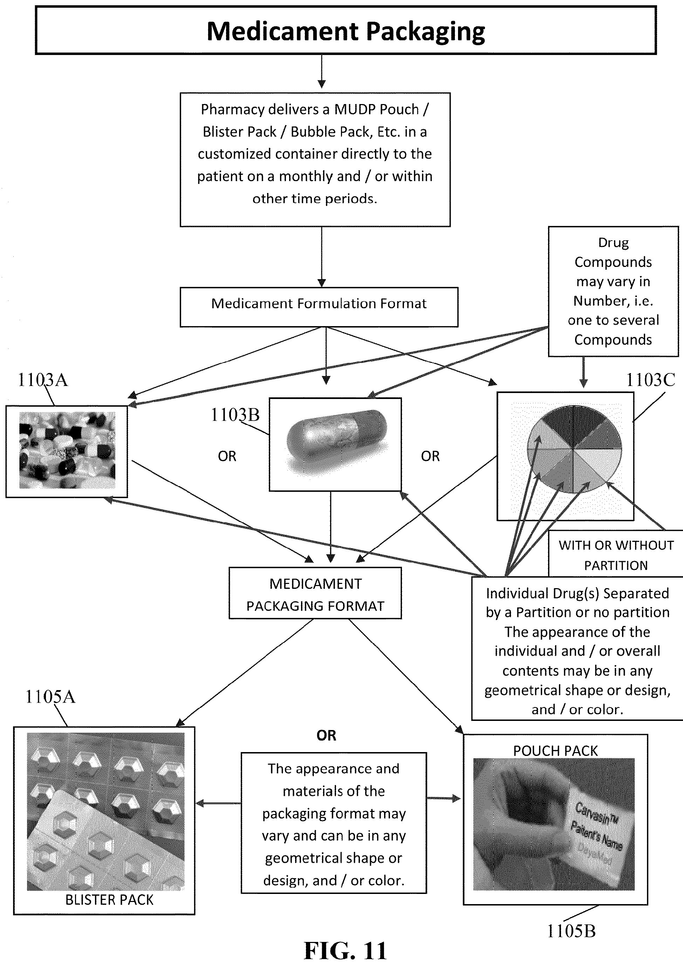

FIGS. 9-11 illustrate methods and apparatuses for disease management according to embodiments of the subject invention. FIG. 9 illustrates a method for diagnosing a disease in a patient and selecting a treatment regimen in accordance with an embodiment of the subject invention. FIG. 10 illustrates a method for delivering a medicament to a patient in accordance with an embodiment of the subject invention. FIG. 11 illustrates example apparatuses for packaging one or more medicaments for delivery to a patient in accordance with an embodiment of the subject invention. These methods can be used separately or linked together as indicated.

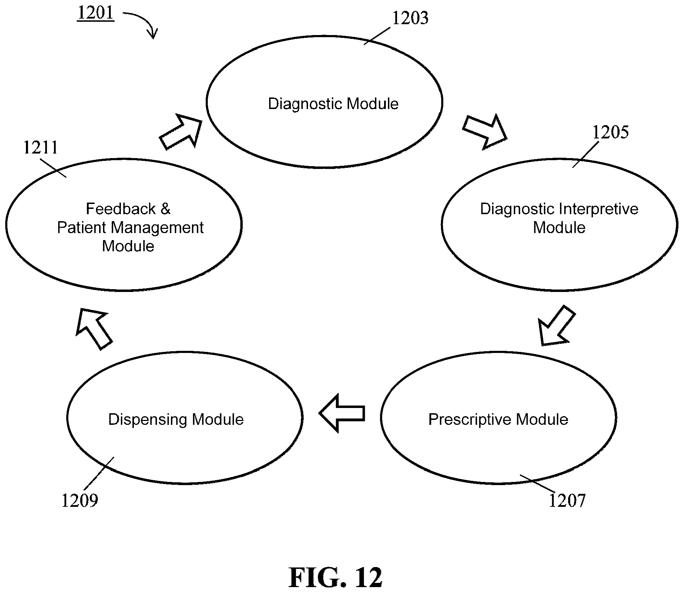

In an embodiment of the subject invention, the method **901** of FIG. 9 is used to diagnose and select a treatment regimen for a patient. As indicated the method **901** can be used for initial diagnosis of disease or subsequent follow-up. In an embodiment, the method **901** is used in primary care of the patient. In another embodiment, the method **901** is used in secondary care of the patient. At a step **903,** the patient is evaluated in accordance with various guidelines. As further discussed below, a Diagnostic Module, such as Diagnostic Module **1203** (see FIG. 12), can be used to collect and evaluate various information about the patient ("patient information"). At a step **905,** the patient's risk factors for disease are determined. Again, although cardiovascular and cerebrovascular risk factors are indicated here, the method **901** and other embodiment of the invention can be used to diagnose and treat other diseases or conditions. Regardless, at a step **907,** a Diagnostic Interpretive Module, such as Diagnostic Interpretive Module **1205** described below, can be used to evaluate risk factors and thereby access the patient's disease risk. As indicated, this step can be used to determine the patient's initial disease risk as well as to track the patient's progress in reducing such risk. At a step **909,** a Prescriptive Module, such as the Prescriptive Module **1207** described below, is used to select and/or recommend a treatment regimen based on some or all of the patient information and guidelines. At a step **911,** a physician or other healthcare provider prescribes a treatment regimen based on the selection/recommendation or the step **909.** In an embodiment, a plurality of treatment regimens are recommended in the step **909** and physician selects one of the recommended treatment regimens in the step **911.** In another embodiment, a single treatment regimen is selected in the step **909** and the physician confirms, modifies, titrates, or otherwise customizes the selected treatment regimen in the step **911.** In another embodiment, the physician is not consulted and the method **901** proceeds directly from the step **909** to the step **913.** At a step **913,** the selected treatment regimen is communicated to a treatment regimen delivery mechanism, such as the Dispensing Module **1209** described below, for delivery and/or communication to the patient. In an embodiment, the method **1001,** described next, is part of a treatment regimen delivery mechanism.

In an embodiment of the subject invention, a method **1001** of FIG. 10 is used to deliver components of a treatment regimen to a patient. In an embodiment, the method **1001** is preformed by a Dispensing Module, such as the Dispensing Module **1209** described below. According to the method **1001,** at a step **1003** a physician or other health care provider selects a treatment regimen for the patient. In an embodiment, the method **901** is used for this selection. In an embodiment, the selection is communicated at this point to the Dispensing Module. Next, the selected treatment regimen is communicated to a pharmacy, Dispensing Module, or other source of treatment components. As indicated by steps **1005A** and **1005B,** this communication can occur via various communication technologies known in the art. In a step **1007,** the components of the treatment regimen are delivered to the patient or other location. In an embodiment, a dispensing apparatus, such as the dispensing apparatus **1301** described below, is delivered as part of the step **1007.** In an embodiment, one or more of the example apparatuses illustrated in FIG. 11 are used to package medicaments included in the delivered treatment regimen.

FIG. 11 illustrates example apparatuses for packaging one or more medicaments for delivery to a patient in accordance with an embodiment of the subject invention. As shown, the medicament formulations can have various formats (e.g., **1103A-C**) and the medicaments can be packaged in using various packing formats (e.g., **1105A-B**). The various formats shown in FIGS. 8A-C can also be used with the subject invention. These formats are merely examples. Other formats can be used with the subject invention.

FIG. 12 diagrammatically illustrates a Disease Management System **1201** in accordance with an embodiment of the subject invention. In the embodiment shown, the Disease Management System **1201** includes a Diagnostic Module **1203,** a Diagnostic Interpretive Module **1205,** a Prescriptive Module **1207,** a Dispensing Module **1209,** and a Feedback and Patient Management Module **1211.** Many different arrangements of the various components depicted, as well as components not shown, are possible without departing from the spirit and scope of the present invention. In embodiments, subsets of the components depicted are utilized. In embodiments, each component is used alone without the others depicted.

The arrows shown in the diagram of Fig. 12 illustrate a possible information flow between the components depicted. In other embodiments of the subject invention, information can flow in either direction between any pair of components forming part of the Disease Management System **1201.** In embodiments, intermediate components not shown can be included. In the embodiment shown, the information flows in a circle to complete a feedback loop as further described below.

In an embodiment of the subject invention, the Diagnostic Module **1203** provides access to patient information, such as the patient's chart, medical records, imaging, etc., as well as scientific guidelines for patient treatment. In an embodiment, the Diagnostic Module **1203** provides an interface that can be used to both read and write this type of information. In another embodiment, the Diagnostic Module **1203** only provides read access and the information is recorded via a different interface.

Various patient information can be viewed and/or recorded via the Diagnostic Module **1203.** For example, among other patient information, the Diagnostic Module **1203** can provide access to:
1. Patient History, such as:
   a. Current Symptoms /Pathologies;
   b. Current Medications;
   c. Patient Identification Data;
   d. Race/ National Origin;
   e. Education;
   f. Occupation;
   g. Family History / Composition;
   h. Life Style / Diet;
   i. Salt Intake;
   j. Fruits & Vegetables;
   k. Low Fat Dairy Products;
   l. Alcohol Intake;
   m. Physical Activity;
   n. Tobacco Use; and
   o. Other relevant historic information about the patient;
2. Physical Examination, such as:
   a. Height;
   b. Weight;
   c. Temperature;
   d. Blood Pressure;
   e. Heart rate;
   f. Respiration rate;
   g. Review of systems; and
   h. Other relevant exam results.
3. Blood Profile and Laboratory Tests, such as:
   a. CBC with Differential;
   b. Blood Glucose;
   c. 3 month Average Glucose;
   d. Hemotocrit;
   e. Lipid Panel;
   f. Serum Potassium;
   g. Creatinine;
   h. Calcium;
   i. Urine Analysis: Albumin / Creatinine Ratio;
   j. Homocysteine Profile; and
   k. Other relevant Laboratory Testing;
4. Imaging and Electronic Tests, such as:
   a. Ankle/Brachial BP;
   b. EKG;
   c. BMI;
   d. CT Scan;
   e. MRI;
   f. B-Mode Ultrasound (Intima/Media Thickness):
      i. Carotid;
      ii. Aorta;
      iii. Femoral Artery; and
   g. Other relevant imaging and electronic testing.

In an embodiment of the subject invention, the Diagnostic Interpretive Module **1205** provides tools to evaluate risk of particular diseases based on patient information and an evaluative methodology. In a further embodiment, the level of risk is categorized into predetermined categories. In an embodiment, the categories Below Average Risk, Average Risk, Moderately Above Average Risk, and High Risk are used. Other methods of categorizing disease risk will be known to those skilled in that art and can be used with the subject invention. Various evaluative methodologies known in the art can also be employed. In an embodiment, the risk factors and/or co-morbidities assessed include, but are not limited to, the presence of diabetes mellitus, hypertension, dyslipidemia, obesity, hypertriglyceridemia, tobacco use, microalbuminuria, target organ damage, sleep apnea, kidney disease, primary Aldosteronism, renovascular disease, Cushing's Syndrome, Pheochromocytoma, Coarctation of Aorta, as well as the patient's thyroid/parathyroid, Glomerular filtration rate, age, physical activity, and family history of CVD or premature CVD. In an embodiment, a subset of these risk factors and/or co-morbidities are assessed. In an embodiment, male patients over 55 and female patients over 65 are deemed to be at increased risk. Body Mass Index among other methods can be used to determine the presence of obesity. In an embodiment, the risk associated with hypertension is assessed according to the methodology described in Table 3 above. Other methodologies for assessing hypertension are known in the art and can be used with the subject invention.

In an embodiment of the subject invention, the Prescriptive Module **1207** is used to recommend, select, and/or evaluate one or more treatment regimens based on patient information and guidelines. In a further embodiment, the Prescriptive Module **1207** also transmits an indication of a selected treatment regimen to one or more persons involved in the care or treatment of the patient. For example, the Prescriptive Module **1207** can transmit an indication to the patient, one or more health care providers treating the patient, and/or one or more payors financing the treatment of the patient, among other persons or entities participating in the provision of the patient's care or health management. Payors can include persons paying directly for some or all of the care or treatment of the patient or those providing insurance or other coverage which pays for some or all of the treatment of the patient. In an embodiment, state or federal governments may be considered payors. The treatment regimen may include one or more medicaments, tests, activities, or other treatment to be taken by the patient. The indication of the treatment may describe some or all of the treatment regimen at various levels of detail depending on need. In a further embodiment, the Prescriptive Module **1207** also directs medicaments or other items needed for the treatment regimen to be delivered to the patient. In an embodiment, the Prescriptive Module **1207** directs a dispensing device, such as the Dispensing Device **1303** described below, to be delivered to the patient.

In an embodiment, the Prescriptive Module **1207** recommends one or more treatment regimens based on the patient information and the guidelines. A physician or other healthcare provider can then evaluate the recommendations and select a treatment regimen for the patient. In a further embodiment, the Prescriptive Module **1207** selects the treatment regimen without selection advice from a health care provider. In an embodiment, the health care provider is able to review and modify the selection made by the Prescriptive Module. In another embodiment, the Prescriptive Module **1207** assists in evaluating one or more treatment regimens. For example, the Prescriptive Module **1207** can consider counter indications for a treatment regimen. In another embodiment, the Prescriptive Module **1207** assists a health care provider in titrating one or more medicaments included in a treatment regimen. In an embodiment, the Prescriptive Module **1207** recommends and/or selects a specific dosage of one or more medicaments to be given to the patient.

In an embodiment of the subject invention, the Dispensing Module **1209** evaluates a patient's compliance with a treatment regimen. In a further embodiment, the Dispensing Module **1209** transmits an indication of compliance or noncompliance with the treatment regimen to one or more persons involved in the care or treatment of the patient. As discussed above, persons involved in the care or treatment of the patient can include the patient, one or more health care providers treating the patient, and/or one or more payors financing the treatment of the patient, among other persons. The indication transmitted can serve various purposes and be received in various forms. For example, an alert message can be transmitted to the patient indicating that they forgot to take a medicament, or go to the gym, or take a test. In another embodiment, an alert message is sent to a health care provider indicating that the patient has repeatedly failed to take a medicament as directed. In another embodiment, an alert message is sent to a payor indicating that the patient has repeatedly failed to exercise as prescribed in the treatment regimen. In other embodiments, alert messages (or reminders) can be sent before a failure occurs, such as a reminder to take medication or go to the gym.

As discussed above, various communication technologies can be used with the subject invention to accomplish the transmission of such information. The subject invention is not limited to any particular communication technology. Communication can be over a network, such as the Internet, a LAN, WAN, VPN. Communication can occur via a mainframe or point-to-point connection. Various client devices can be used to send or receive such communication including, but not limited to, computers, PDAs, cellular phones, other client devices. Communication can also occur via various communication protocols known in the art. For example, various cellular communication technologies can be used. In an embodiment, Short Message Service (SMS) protocol is used. In another embodiment, email message protocols are used, such as Simple Mail Transfer Protocol (SMTP).

In an embodiment, the Dispensing Module **1209** utilizes a dispensing device, such as the Dispensing Device **1303** described below, to capture compliance information regarding a patient's taking of one or more medicaments. In an embodiment, the dispensing device includes sensors adapted to sense the presence, absence, or movement of one or more medicaments and/or their packaging so as to assess whether or not a patient has taken one or more medicaments. In a further embodiment, the dispensing device also includes a communication device adapted to transmit compliance information to one or more persons involved in the care or treatment of the patient. In a particular embodiment, the communication device transmits such compliance information to a server where it is processed and communicated on to one or more persons involved in the care or treatment of the patient.

In an embodiment, the Dispensing Module **1209** captures information regarding one or more tests, activities, or other treatments to be taken by the patient. For example, the Dispensing Module **1209** can receive blood glucose levels from a blood glucose meter, or the Dispensing Module **1209** can receive a message from a fitness center computer indicating that the patient entered or left an associated fitness center, or the Dispensing Module **1209** can receive a heart rate from a heart rate monitor worn by the patient or information from a pedometer worn or similar device worn by the patient. Other methods of monitoring patient compliance without action by the patient themselves can be used with the subject invention. In an alternative embodiment, the Dispensing Module **1209** receives compliance information from the patient themselves.

In an embodiment of the subject invention, the Feedback and Patient Management Module ("FPMM") **1211** gathers compliance information and evaluates efficacy of a treatment regimen for a patient. In a further embodiment, the Feedback and Patient Management Module **1211** transmits information about the efficacy of the treatment regimen to one or more persons involved in the care or treatment of the patient. As discussed above, persons involved in the care or treatment of the patient can include the patient, one or more health care providers treating the patient, and/or one or more payors financing the treatment of the patient. In an embodiment, the Feedback and Patient Management Module **1211** tracks the patient's noncompliance, progress, and/or success with the treatment regimen. In an embodiment, the FPMM **1211** provides feedback to the patient regarding such noncompliance, progress, and/or success. In a further embodiment, the feedback provided to the patient is tailored for greater relevance to the patient's pattern of behavior, patient information, or other data. In an embodiment, the FPMM **1211** provides management advice to other persons involved in the patient's care or treatment regarding such noncompliance, progress, and/or success. In a further embodiment, the advice provided is tailored for greater relevance to the patient's pattern of behavior, patient information, or other data. In an embodiment, economic incentives or disincentives are offered to the patient based on such noncompliance, progress, and/or success. In another embodiment, the incentives or disincentives are mandatory. In an embodiment, reduced health premiums are offered for compliance with health diet or exercise activities required by the treatment regimen. In another embodiment, discounts on prescription drugs are offered for compliance with dosage requirements of the treatment regimen.

Various communications technologies can be used to deliver feedback and/or management advice to person in accordance with the subject invention. For example, such information can be delivered via mail, facsimile, email, automated telephone message, Internet Relay Chat, SMS message, website, newsgroup, or other post, among other possible communication methods. In an embodiment, persons select a preferred communication method from a set of available methods.

In an embodiment of the subject invention, information provided via one or more modules of the system **1201** causes an update to information in one or more other modules of the system thus creating a feedback loop. In another embodiment, such feedback occurs with human intervention. For example, a physician can receive management advice via the FPMM **1211** and decide to select a different treatment regimen via the Prescriptive Module **1207.** In an embodiment, such feedback occurs electronically or independent of human intervention. For example, FPMM **1211** can automatically update patient information via the Diagnostic Module **1203** based on the patients progress. For example, the FPMM **1211** may update a patients weight received via the Dispensing Module **1209.**

In an embodiment of the subject invention, the system **1201** includes a social networking component that allows persons involved in a patient's or patients' care or treatment to network. Various facilities can be used for such networking including, but not limited to, online communities, blogs, and other social media. In an embodiment, various networks are customized to the persons' particular needs. In an embodiment, such networks provide education, support, guidance, information, and/or motivation to the patient or other person involved in the patient's care. In a further embodiment, network members are suggested or selected by a FPMM, such as FPMM **1211.** In an embodiment, various social networking actions and/or events can be prescribed as part of a treatment regimen.

In an embodiment of the subject invention, health clubs partner with person's involved in a patient's care or treatment. In an embodiment, such health clubs are provided financial or other incentives to partner. In a further embodiment, a portion of those incentives are passed on to the patient. In an embodiment, such health clubs are required to implement a reporting process by which patient compliance information is communicated to a FPMM, such as FPMM **1211.** In a further embodiment, such patient compliance information is transmitted electronically via a communication device. Such compliance information can include, but is not limited to, attendance, progress, weight, bad-fat, and/or exercise activities of the patient, among other information.

In an embodiment of the subject invention, health food retailers and/or grocery stores partner with person's involved in a patient's care or treatment. In an embodiment, such stores are provided financial or other incentives to partner. In a further embodiment, a portion of those incentives are passed on to the patient. In an embodiment, such stores are required to implement a reporting process by which patient compliance information is communicated to a FPMM, such as FPMM **1211.** In a further embodiment, such patient compliance information is transmitted electronically via a communication device. Such compliance information can include, but is not limited to, a record of purchase made by the patient at the store.

In an embodiment, a Disease Management System is provided comprised of five separate interrelated modules as shown in FIG. 12. In an embodiment, the Diagnostic Module exposes the patient and provides access to various disease management guidelines. The module can be accessed by a physician, care provider, or other user to obtain patient information or review guidelines for treatment. In an embodiment, patient information and/or guidelines are accessed via a database on a network. In an embodiment, the user - irrespective of his or her geographic location - has access to the patient's medical history in "real time," and is augmented with scientific information from the National Institute of Health (NIH), the Center for Disease Prevention and Control (CDC), The American Heart, Lung and Blood Institute (AHLBI), The Food and Drug Administration (FDA), The American Institute of Radiologists, The American Diabetes Association (ADA), the World Health Organization (WHO), and/or other guidelines. In an embodiment, the database can be used by the physician, care provider, or other user for informed, educated, and experienced judgment about a patient's treatment.

In an embodiment, diagnostic evaluation is specifically directed at quantifying the probability of cardiovascular, cerebrovascular accidents, and the progression of insidious diabetic sequelae within a 10 year time frame, based on the NIH's Farmington Heart Study point evaluation methodology. In other embodiments, other timeframes or evaluative methodologies are used. In an embodiment, the quantification of diabetic sequelae contributing risk factors as a "Target Organ Disease" is incorporated within the module. In an embodiment, a patient's risk for cardiovascular and cerebrovascular accidents is accessed within the following categories: BELOW AVERAGE RISK, AVERAGE RISK, MODERATELY ABOVE AVERAGE RISK, HIGH RISK. The risk assessment methodologies and guidelines described herein are merely illustrative examples. Other systems for measurement for patient risk assessment and guidelines are known in the art and can be used with the subject invention.

### Example Scenario

The following is an example of how an embodiment of a Disease Management System can function. Other embodiments, may utilize more, fewer, or different components or functions. Also, the functions performed in this scenario are merely examples. Other components or functions are described below and can be used in a different order. For this scenario, assume the patient is a male, non-smoker who is 50 years of age with high overall cholesterol, moderate good cholesterol, and moderate to high blood pressure. In an embodiment, his coronary heart risk profile evaluation would score as follows:

**TABLE 16.**

| Question or Condition | Answer | Score |
|---|---|---|
| Age | 50 | 3 |
| Total Cholesterol (240-279) | 255 | 2 |
| HDL Cholesterol (50-59) | 52 | 0 |
| Systolic Blood Pressure (140-159) | 145 | 2 |
| Diabetes | No | 0 |
| Smoker | No | 0 |
| Total Score | | 7 |

| | | |
|---|---|---|
| A total score of "7" on the coronary disease risk profile from table 16 can then be plotted on the risk assessment table (Table 7 above) along the row indicated by the number 7, and intersecting with the column with an age heading of 50-54. Therefore, in this embodiment, this patient has a risk score of "2.6," which indicates he has an "average risk" of CHD. The patient, with a risk score of 2.6, would then be prescribed a treatment regimen in accordance with the above matrix. In this scenario, the patient's quantified state of health data is advanced and correlated to a Prescriptive Module. | | |

The Prescriptive Module, in this embodiment, includes guidelines from: the NIH, the CDC, the AHLBI, the FDA, the ADA, WHO, the Manufacturers Guidelines, and/or any other available guidelines. Using the Prescriptive Module, a customized prescription is manually and/or electronically computed for the patient from the patient information and guidelines, and/or further verified for potential adverse reactions against normal prescriptive guidelines, and/or electronically and/or manually transmitted to participants contributing to the patient's state of health (e.g., the patient's medical history file in the physician's database, the Pharmacy, the relevant Third-Party Payer (Insurance Company/Medicare/ Medicaid), the patient's employer, the Agency maintaining the follow-up modality, and/or any other party). In an embodiment, the resulting prescription is delivered to the patient's home and/or any other location as further described below.

In an embodiment, a Dispensing Module is initiated with the receipt of a prescription from the patient's Physician, care provider, a Prescriptive Module, or other source. As further described below, the Dispensing Module can facilitate delivery of medicaments or other treatment components (e.g., motivational literature, educational literature, exercise equipment, scheduling or motivational messages, diet information, social networking contacts) to the patient. In a further embodiment, the Dispensing Module also receives compliance information back from the patient. Various compliance information can be collected with and/or without the assistance of the patient. In a further embodiment, the Dispensing Module transmits some or all of the compliance information to one or more persons involved in the patient's care or treatment. In a further embodiment, the Dispensing Module also evaluates the compliance information and can transmit a summary, highlights, or analysis of the compliance information to one or more persons involved in the patient's care or treatment.

In an embodiment, medicaments or other treatment components are delivered to the patient along with a dispensing apparatus. In an embodiment, the dispensing apparatus includes a dispensing device with sensors capable of sensing when the treatment components are moved, used, or not used for a selected length of time. Thus, compliance information can be derived from the dispensing apparatus. In an embodiment, such information is stored on the dispensing device or apparatus and later retrieved. In another embodiment, the dispensing apparatus comprises a communications device capable of sending such information to a computer on a network for further processing and/or transmission. The communication device can be incorporated into the body of the dispensing device or apparatus or as a separate component operably connected via a cable or other known means. In an embodiment, short-range wireless communications technologies, such as Bluetooth, can be used to connect the communication device to the dispensing device. As discussed above, various communication technologies can be used to accomplish transmission of information.

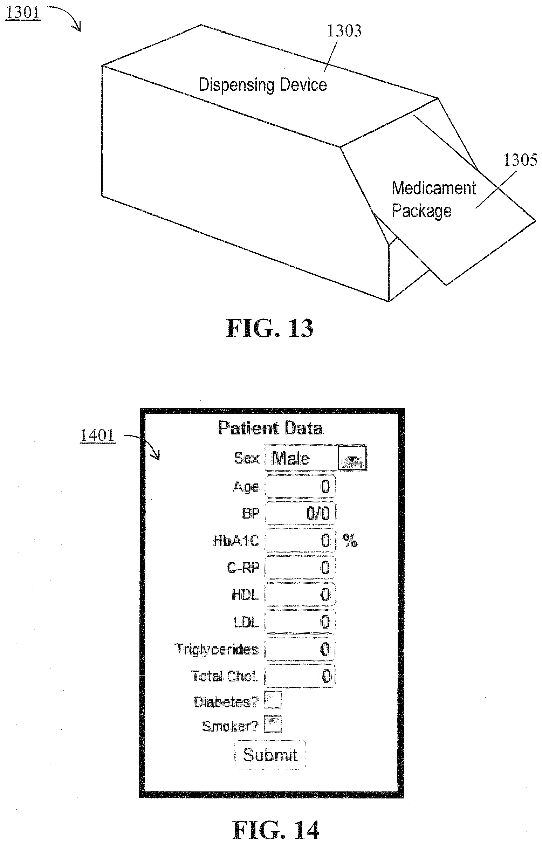

FIG. 13 illustrates an apparatus **1301** for dispensing medicaments in accordance with an embodiment of the subject invention. As shown, the apparatus includes a dispensing device **1303** and one or more packages **1305** for holding medicaments. In the embodiment shown, the dispensing device **1303** is shaped like a supply box. As discussed above, additional components can be incorporated into the body of such a supply box or connected to the supply box using various means known in the art. In an embodiment, the supply box has one or more ports or antennas on the back side of the device (not shown) for transmitting information. In an embodiment, the dispensing device **1303** includes one or more sensors capable of sensing movement or other information regarding the one or more packages **1305.** For example, the movement of one or more packages **1305** can be determined by a change in weight. In an embodiment, the packages **1305** themselves comprise the sensors. In a further embodiment, the dispensing device **1303** includes a communication device for communicating information.

In an embodiment, the use or non-use of the dispensing device **1303** or the movement or non-movement of the one or more packages **1305** triggers the storage and/or communication of such an event or non-event (known as "compliance information"). In an embodiment, the trigger occurs when such an event or non-event happens within a specified timeframe. In another embodiment, the trigger occurs regardless of timeframe. As discussed above, the dispensing device **1303** can communicate compliance information using various communications technologies, including but not limited to: Cellular Phone Technology (Short Message Service - "SMS," Global System for Mobile Communications - "GSM," and/or any other technology utilizing the cellular communication platform), land based telephone or other hardwire linked (either connected or separate) to the communication device, and/or satellite communication technology.

In an embodiment, the dispensing device includes one or more indicators for presenting information to a user. The indicator can alert the user of various issues or conditions. For example, the indicator may indicate that it is time to take a prescribed action including, not limited to, taking a dose of medicament, exercising, taking a test, attending an appointment, eating a regular meal, among other prescribed actions. The indicator may also indicate failure to take such actions on time. In an embodiment, the indicator alerts a user to problem with the dispensing device including, but not limited to, that the device is low on batteries, that the device is low on medicaments, that the device is disconnected from a communications device, among other problems. In an embodiment, the indicator is displayed visibly, such as via an LED, textual display, or graphical display. In an embodiment, the indicator is presented audibly, such as via a speaker. In an embodiment, the indicator is connected to a communication device and is enabled to communicate such information to a computer on a network. Such a computer can thereafter notify one or more persons or entities involved in the care and treatment of the relevant patient. In an embodiment, the indicator can be reset, disabled, or "snoozed" via an input.

In an embodiment, a Patient Management Module, such as FPMM **1211,** reviews data from the Patient Prescriptive Module, Dispensing Module, and/or any other source. In an embodiment, the Patient Management Module integrates electronic surveillance of the patient's prescription administration compliance with the use of the Internet, GSM technology, and human interaction with the patient. "Real Time" patient compliance/non-compliance data can be communicated to the physician, third party payers, the patient employer (if necessary), as well as the patient, and/or any other party. In an embodiment, the patient is reminded of missed doses. Health Club attendance, food purchases and consumption, and/or any other organization and/or activity that improves the patient's health (including but not limited to chronic disease[s]) and/or lowers the patient's health care costs can be electronically or by other means communicated to the Patient Management Module. In an embodiment, such information is further transmitted to relevant participants. In a further embodiment, the Patient Management Module evaluates and the degree of efficacy of the treatment regimen based on such information. In an embodiment, such measures and/or evaluations are communicated to relevant participants in real-time and/or any other time frame. In an embodiment, the progress and reduction of risk category is reinforced to the patient, third-party payers, employers, or any other relevant party that will advance the appropriate financial and/or other health awards and/or other motivation tools for the patient.

In an embodiment, a communication device known as a Patient Module Communication Device transmits the above described information to a recipient, a computer, a computer network or server, and/or non-networked servers, which may or may not utilize one or more intermediate communications and/or relaying technologies. Other communications means known in the art may be used with the subject invention.

### Patient Prescriptive and Monitoring Methodology

In an embodiment of the subject invention, persons involved in the patient's care or treatment have the option of using a manual and/or electronic data management system on a computer and/or non-computerized device that can be portable and/or non-portable called the Clinical Protocol Management Tool. This Clinical Protocol Management Tool utilizes and/or integrates information/data received from the Patient Module Communication Device specified above. This data management system may or may not provide data/information using charts, pictures, imaging, calendars, and/or any other method to communicate information that is inclusive but not limited to: patient disease management information (inclusive of but not limited to compliance) that includes, but not limited to, physician or other care giver's prescribed therapy (pharmaceuticals, medical imaging, consumables, exercise or other activity, or any other recommendation[s]).

In an embodiment, the Clinical Protocol Management Tool utilizes a scoring system and/or other systems of measurement for the patient's health risk factors. In an embodiment, the Clinical Protocol Management Tool provides a user of the Tool with a recommended treatment regime for a patient. In an embodiment, the treatment regime is given a specific name and/or symbol that may or may not be generated from the clinical protocol matrices for that combination and/or individual prescribed pharmaceuticals. In an embodiment, the user can use the Tool to change and/or customize and/or titrate dosages or other treatment regime components as indicated.

### Interfaces to the Disease Management System and Components

Next numerous example interfaces are described with reference to FIGS. 14-23C. These interfaces are merely examples. Embodiments of the subject invention can embody different interfaces comprising fewer, different, or additional components. Also the components depicted can be differently arranged and/or configured.

FIG. 14 illustrates an interface to a Diagnostic Module **1401** in accordance with an embodiment of the subject invention. In an embodiment of the subject invention, the interface **1401** can be used to input information about a patient.

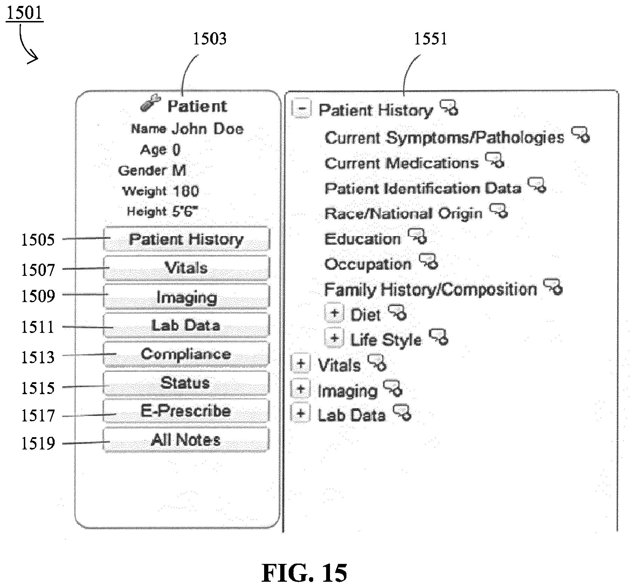

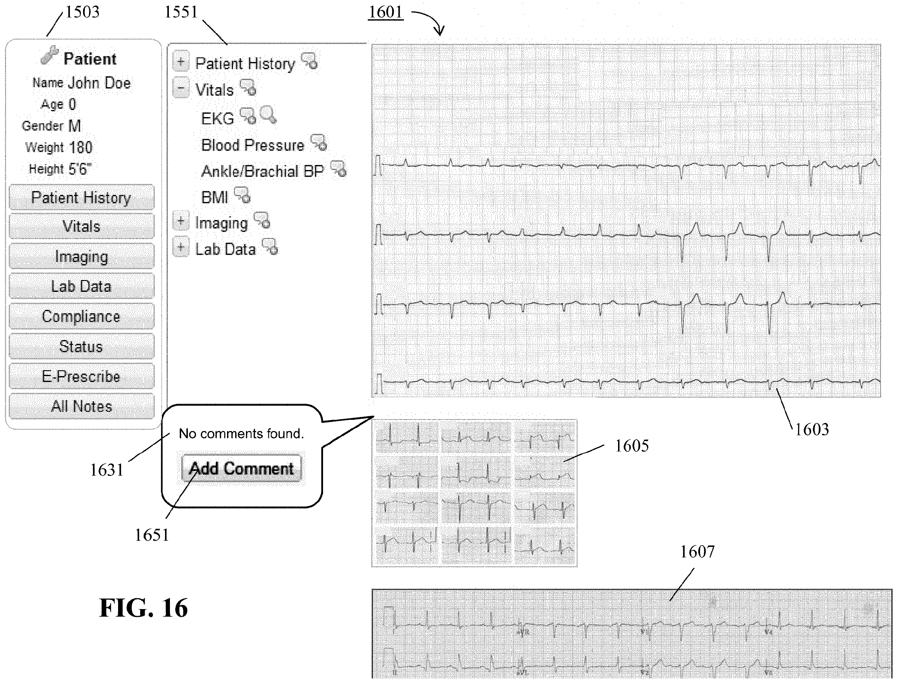

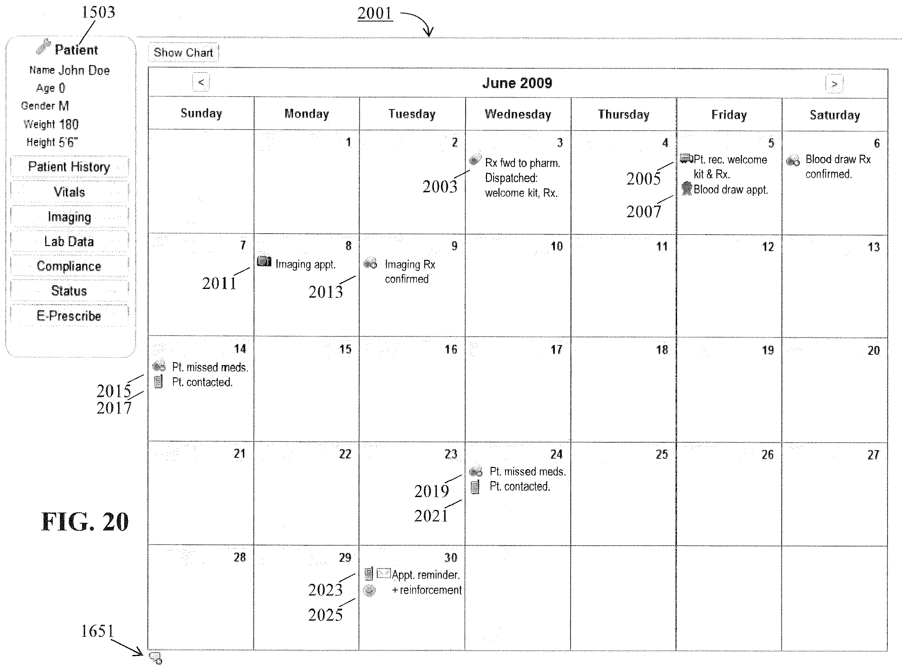

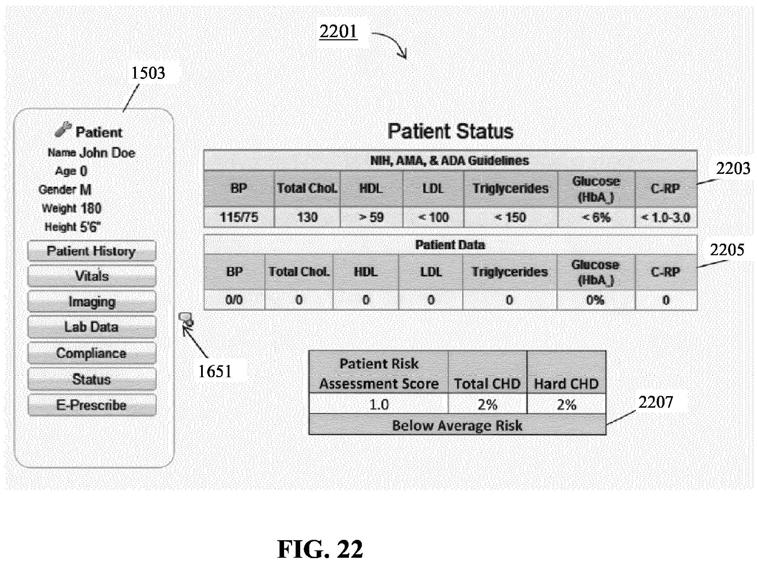

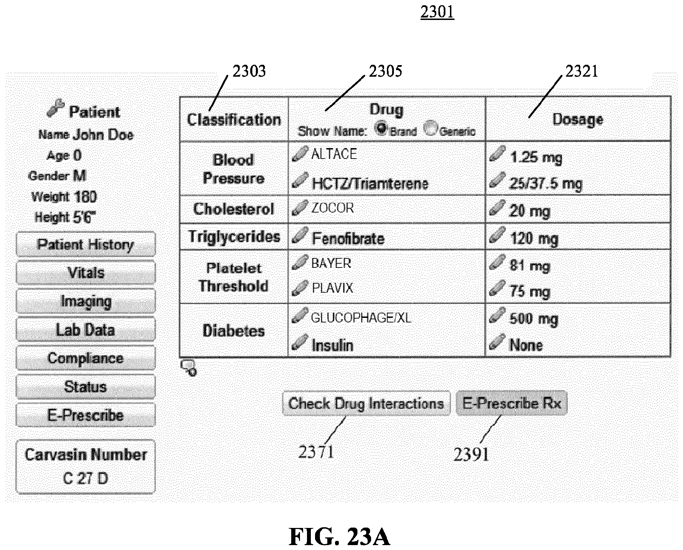

FIG. 15 illustrates an interface to a Disease Management System **1501** in accordance with an embodiment of the subject invention. In an embodiment of the subject invention, the interface **1501** can be used to access different components of a Disease Management System, such as the Disease Management System **1201.** In the embodiment shown, a Disease navigation pane **1503** is provided. A user of the Disease Management System can use the navigation pane **1503** to access different components of the Disease Management System. For example, buttons **1505-1511** can provide access to different interfaces to a Diagnostic Module, such as the Diagnostic Module **1203.** The embodiment shown also includes a patient chart interface **1551.** As further illustrated in FIGS. 16-19, the patient chart interface **1551** can be used to access different interfaces to patient information within the Diagnostic Module. In FIG. 15, the "Patient History" portion of the patient chart interface **1551** is expanded to allow access to various patient history information. In an embodiment, button **1513** provides access to an interface to a Feedback and Patient Management Module, such as FPMM **1211.** Example embodiments of such an interface are described below with reference to FIGS. 20-21. In an embodiment, button **1515** provides access to an interface to a Diagnostic Interpretive Module, such as Diagnostic Interpretive Module **1205.** An example embodiment of such an interface is described below with reference to FIG. 22. In an embodiment, button **1517** provides access to an interface to a Prescriptive Module, such as Prescriptive Module **1207.** An example embodiment of such an interface is described below with reference to FIGS. 23A-23C.

In an embodiment of the subject invention, notes or comments can be added or viewed by various users of a Disease Management System. These notes are appropriate for commenting on various data or results presented by such a system. In an embodiment, notes can be attached to the different components of the Disease Management System. In an embodiment, "global" notes can also be associated with the system as a whole. In the embodiment shown in FIG. 15, button **1519** provides access to all such notes regardless of the portion of the system they are associated with.

FIG. 16 illustrates an interface to a Diagnostic Module **1601** in accordance with an embodiment of the subject invention. In the embodiment shown, interface **1601** presents various patient information related to a patient's clinical status (e.g., **1603, 1605, 1607**). In the embodiment shown, the patient chart interface **1551** can be used to gain access to EKG, Blood Pressure, Ankle/Brachial BP, and BMI. Window **1631** illustrates an example of a note field that can be used to comment on the information presented. In this example, no notes are available. In an embodiment, button **1651** can be used to add a note.

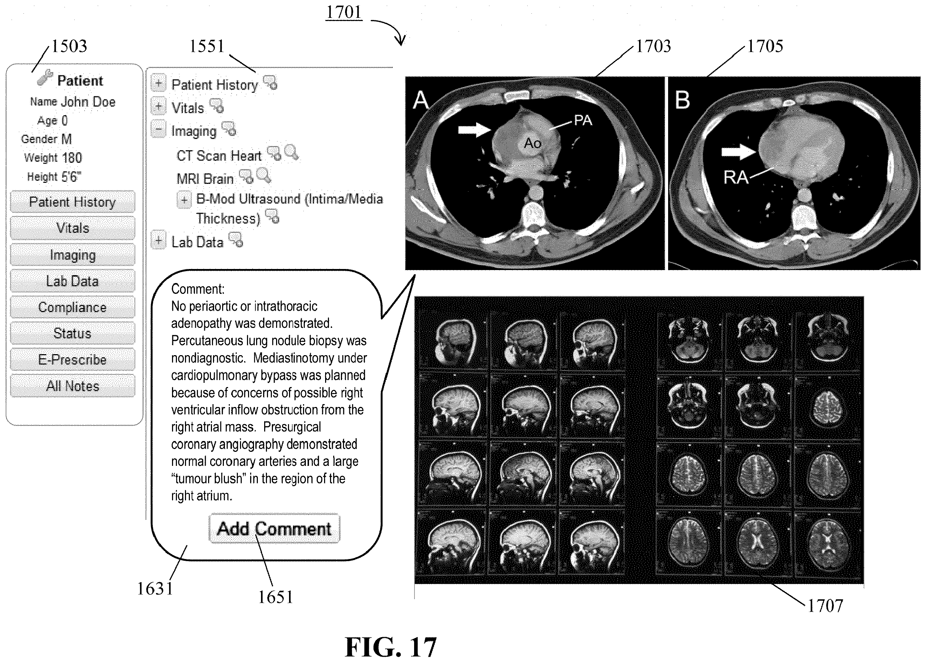

FIG. 17 illustrates an interface to a Diagnostic Module **1701** in accordance with an embodiment of the subject invention. In the embodiment shown, interface **1701** displays various imaging of a patient (e.g., **1703-1707**). In the embodiment shown, the patient chart interface **1551** can be used to gain access to additional imaging. Window **1631** illustrates an example of a note field with a comment related to image **1703.** In this example, button **1651** can be used to add additional notes.

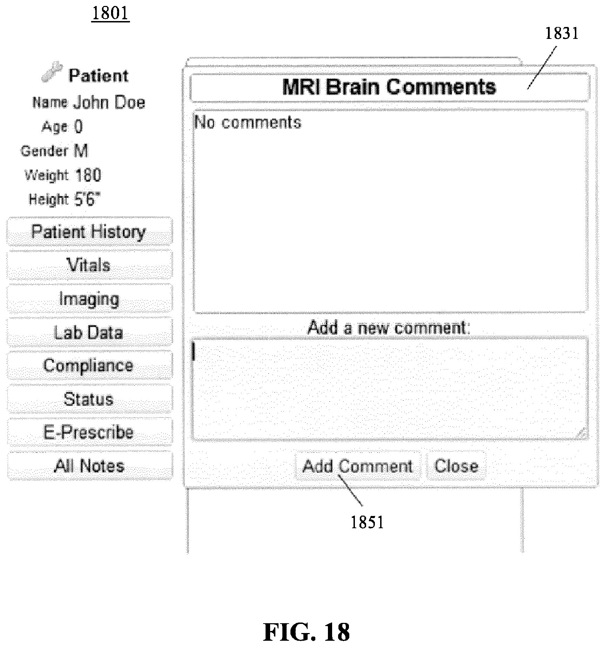

FIG. 18 illustrates an interface to a Diagnostic Module **1801** in accordance with an embodiment of the subject invention. In the embodiment shown, interface **1801** presents various information related to a Brain MRI of a patient. Window **1831** can be used to comment on the Brain MRI. In an embodiment, button **1851** can be used to add such a comment.

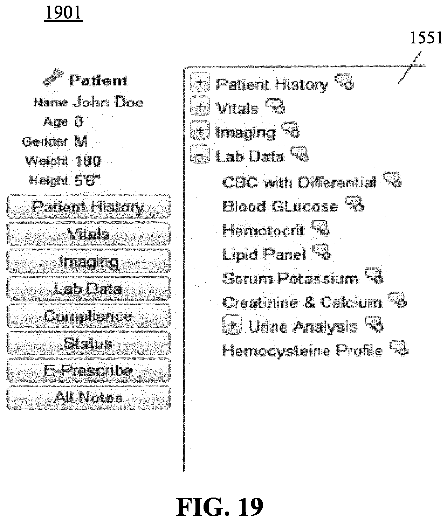

FIG. 19 illustrates an interface to a Diagnostic Module **1901** in accordance with an embodiment of the subject invention. In the embodiment shown, interface **1901** allows access to various patient information related to a patient's lab data. In the embodiment shown, the patient chart interface **1551** can be used to gain access to various lab data.

FIG. 20 illustrates an interface to a Feedback and Patient Management Module **2001** in accordance with an embodiment of the subject invention. In the embodiment shown, interface **2001** displays information regarding a treatment regimen prescribed for a patient. In the embodiment shown, this information is displayed on a calendar interface; however, various other interfaces can be used to display such information including a task or other list, a timeline, among other known interfaces. In the embodiment shown, interface **2001** displays both prescriptive and descriptive information about the treatment regimen. For example, interface **2001** includes prescriptive information about tests or other events to be performed by the patient or others as part of the treatment regimen. In example displayed, the patient is to have a blood draw appointment on Saturday, June 6, 2009 **(2007)** and an imaging appointment on Monday, June 8, 2009 **(2011).** These are merely examples other types of tests or other events can be prescribed as part of a treatment regime and displayed on the interface **2001.** In an embodiment, the interface **2001** can also show actions to be taken by other persons or entities involved in the patient's care. For example, on Tuesday, June 30, 2009, the patient is to be reminded of an appointment **(2023).** This reminder can be made by a healthcare provider or other person. In another embodiment, the reminder is electronically performed via a FPMM, such as FPMM **1211,** without human involvement. Interface **2001** also presents descriptive information, which describes events related to the patient's treatment that occurred or failed to occur as prescribed. As shown, some of the events can describe actions or omissions of the patient; others can describe actions or omissions of others. In the example displayed, a prescription was sent to a pharmacy and welcome kit was dispatched on June 3^{rd} **(2003).** As further discussed above, the treatment regimen can include various motivational, educational, or other literature provided to the patient. A welcome kit is an example of such literature. In an embodiment, the literature is customized for the patient by a FPMM, such as FPMM **1211.** In a further embodiment, the welcome kit introduces the patient to a social networking component of the treatment regimen as further described above. Also in the displayed example, a note **2005** indicates that the patient received the prescribed medicaments and the welcome kit on June 5^{th} and a note **2013** indicates that the imaging appointment on June 8^{th} was confirmed. As shown, the interface **2001** can also indicate omitted actions. For example, the interface **2001** indicates the patient missed taking one or more medicaments on June 14^{th} and June 24^{th} **(2015, 2019).** As discussed above, various alert messages can be sent to the patient to remind the patient to perform various actions prescribed by the treatment regimen. The interface **2001** shows that the patient was alerted to the missed doses on the same day **(2017, 2021).** In the displayed example, note **2025** indicates that positive reinforcement information was sent to the patient on June 30^{th}. Positive reinforcement information is an example of customized literature provided to the patient by a FPMM or other entity. In the embodiment shown, button **1651,** described above, has a different appearance but still performs the same functions.

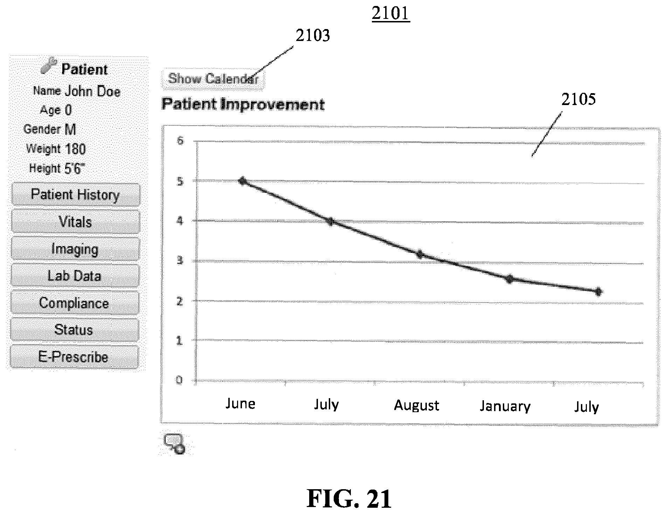

FIG. 21 illustrates an interface to a Feedback and Patient Management Module **2101** in accordance with an embodiment of the subject invention. In the embodiment shown, interface **2101** displays information regarding a patient's results related to a treatment regimen. Results can be displayed since initiation of the treatment regimen, since before initiation, for a subset date range, or other time period. In the embodiment shown, button **2103** provides access to a calendar interface, such as calendar interface **2001.** Also in the embodiment shown, an improvement chart **2105** is displayed. Various charts can be used presenting various results data for the patient. In the example shown, the patient's overall risk score is plotted over time. This type of information can motivate the patient to continue to improve. In an embodiment, this type of information is included in motivational literature as described above.

FIG. 22 illustrates an interface to a Diagnostic Interpretive Module **2201** in accordance with an embodiment of the subject invention. In the embodiment shown, interface **2201** displays guidelines for various risk factors **(2203)** and patient information corresponding to the risk factors displayed **(2205).** In the embodiment shown, interface **2201** also displays a risk score **(2207)** calculated from the risk factors. As discussed above, various guidelines, risk factors, and risk assessment methodologies can be used with the subject invention. In the embodiment shown, the risk score is further categorized into a risk category **(2207).** Estimates of absolute risk of CHD are also displayed **(2207).** Absolute risk is expressed as a percentage likelihood of developing CHD per decade. Total CHD risk equates to all forms of clinical CHD, whereas hard CHD includes clinical evidence of myocardial infarction and coronary death.

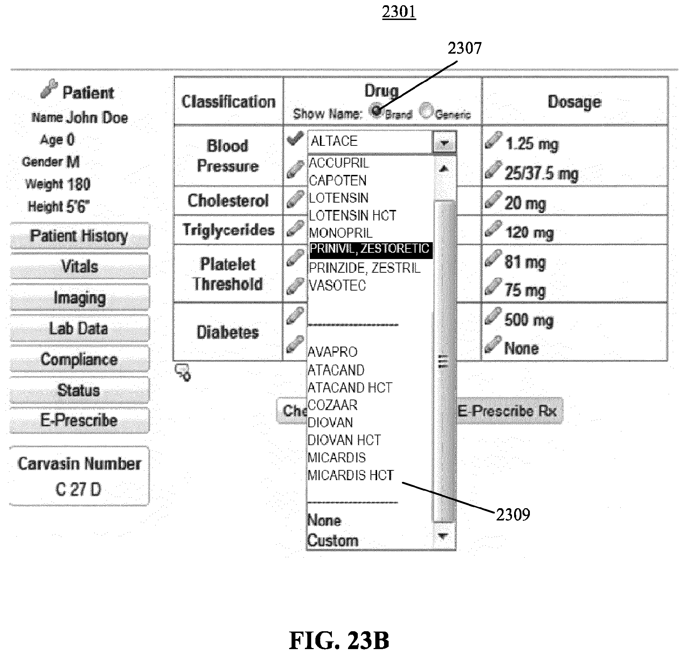

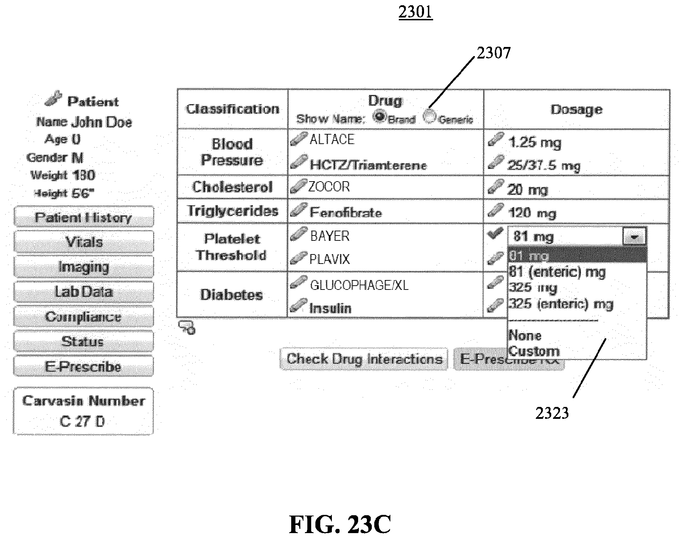

FIGS. 23A-23C illustrate an interface to a Prescriptive Module **2301** in accordance with an embodiment of the subject invention. In the embodiment shown, interface **2301** displays various drug classifications **(2303),** drugs within those classifications **(2305),** and available dosages of the drugs **(2321).** As shown in FIGS. 23B and 23C respectively, drop down menus are provided to allow a user to select a particular drug **(2309)** and a corresponding dosage **(2323).** Also in the embodiment shown, a radio-button **2307** can be used to change the interface to display generic names for the various drugs. Once a drug and dosage has been chosen, an indicia can be displayed corresponding to the selected treatment. As described above, various indicia can be used with the subject invention to indicate various medicaments and/or treatment regimens. In a further embodiment, other treatment components (e.g., exercise, diets, testing) can be selected via an expanded interface (not shown). In the embodiment shown, a check drug interactions button **2371** is provided. In an embodiment, button **2371** access a function which checks for counter-indications of the selected treatment regimen as described above. If counter-indications exist, they can be presented via the interface **2301** (not shown). In the embodiment shown, an E-Prescribe Drug button **2391** is also provided. In an embodiment, button **2391** initiates delivery of components of the selected treatment regimen to the patient. In a further embodiment, such delivery is initiated electronically without additional human intervention. In an embodiment, such delivery is accomplished via the method **1001.**

In an embodiment of the subject invention, a compliance monitoring device is provided that facilitates tracking of one or more patients' compliance with one or more treatment regimens. In an embodiment, the compliance monitoring device monitors the compliance of a single patient. In an embodiment, the compliance monitoring device monitors the compliance of a plurality of patients. In an embodiment, the compliance monitoring device monitors compliance with a single aspect of a treatment regimen(s), such as medication, diet, exercise, tobacco use, alcohol consumption, among other aspects of a treatment regimen. The dispensing device **1300** described above is one example of such a compliance monitoring device. The dispensing device **1300** is capable of monitoring a patient's compliance in taking one or more medicaments. Other compliance monitoring devices can be used. For example, in an embodiment, a key fob can be scanned when a patient purchases groceries and thereby make available information regarding the patient's food purchases. In another embodiment, a heart monitor provides information regarding the patient's level of physical activity.

In an embodiment, the monitoring device comprises at least one sensor capable of sensing information relevant to patient compliance. For example, in an embodiment, the monitoring device includes a barcode scanner and/or radio frequency identification (RFID) reader for scanning identification tags. In a particular embodiment, the at least one sensor senses movement of a MUDP or other medicament packaging as described above. In a particular embodiment, the at least one sensor senses the movement of liquid filled dose packaging, such as for a glaucoma medication. In an embodiment, a sensor can be used to monitor the clinical status of the patient, such as the patient's weight, heart rate, blood pressure, etc. In an embodiment, a sensor obtains the patient's EKG signal. In an embodiment, a sensor can be used to test one or more bodily fluids of the patient, such as blood, urine, sweat, semen, and/or other bodily fluids. For example, the sensor can be used to test one or more aspects of blood chemistry as known in the art. In an embodiment, the sensor provides a complete blood chemistry analysis or profile of the patient. In an embodiment, the sensor measures one or more blood serum levels such as glucose, cholesterol, or other blood serum levels. In an embodiment, a sensor can be used to test a tissue sample from the patient.

In an embodiment, the monitoring device stores and/or transmits information to one or more compliance manager modules as described below. In another embodiment, information can be transmitted directly to a Patient Module Communication Device, client device, and/or other user device. In an embodiment, information is transmitted to other program modules or devices. Such information can be transmitted over a backplane or via one or more wired or wireline communication technologies known in the art. In an embodiment, the monitoring device stores information for later retrieval. In another embodiment, the monitoring device transmits information on a real time and/or periodic basis, such as once an hour, once a day, once a week, etc.

In an embodiment, the monitoring device analyzes or processes information before storage or transmission. In an embodiment, signals from the at least one sensor are decoded or otherwise interpreted to inform compliance information. In an embodiment, information received from the at least one sensor over time is combined to inform compliance information. In an embodiment, information from a plurality of sensors is combined to inform compliance information. In an embodiment, such information is combined with other information received from data storage, an input interface, and/or a communication interface to inform compliance information.

In a further embodiment, the compliance information is further processed before transmission. For example, the compliance information can be rolled-up or otherwise summarized, calculations can be performed with the compliance information to yield useful results, or the content of the information can be sorted, filtered, or otherwise formatted. In an embodiment, a compliance report is generated as further discussed below. In an embodiment, the content, format, timing, and/or delivery mode of the compliance report is customized for the recipient of the report. In an embodiment, the report is customized based on the type of recipient receiving the report. For example, a patient can receive one report, while the patient's physician receives another. A third-party payor can receive still another report. A government agency, NGO, advertiser, and/or other user, for example, can receive a report stripped of all identifiable patient information. In an embodiment, the report is customized based on the preferences or characteristics of different recipient populations or particular recipients. For example, homeless patients can receive reports via a surrogate, such as a social worker or other agent, younger patients can receive reports via text message, while blind patients can receive reports via an automated voice message system. These examples are illustrative, other customizations can be used.

In an embodiment, the compliance report includes a compliance score which represents a patient's or population's degree of compliance with one or more treatment regimens. In an embodiment, the compliance score represents the degree of compliance of a particular patient. In an embodiment, the compliance score represents the degree of compliance with a particular treatment regimen. In an embodiment, the compliance score represents the degree of compliance with a particular aspect of one or more treatment regimens. In an embodiment, a compliance score is used to evaluate the efficacy or likely efficacy of a treatment regimen. In an embodiment, a treatment regimen is selected for a patient based in part on the patient's compliance score. For example, a low compliance score may indicate a preference for drug therapy or surgery over lifestyle changes in a patient, while a high score may indicate the opposite. In an embodiment, the compliance score is used to predict the reliability of an adverse incident report and/or study result. In an embodiment, a patient is rewarded or penalized based on the compliance score, as further described below.

The compliance score can have various forms and incorporate various data. For example, in an embodiment, the compliance score is a scalar quantity. The scalar quantity can be within a range of possible values. In an embodiment, the compliance score is a percentage. The compliance score can include a ratio of prescribed tasks complied with over prescribed tasks considered. Components of the compliance score can be weighted, with different types of tasks or different time periods having a greater or lesser effect on the score. For example, recent tasks can be weighted more highly. In an embodiment, the compliance score for a particular patient is normalized over a population of patients. In an embodiment, the compliance score can be generated based on analysis of prescribed tasks for a particular time period, e.g., a month, 90 days, a year. In another embodiment, the compliance score is cumulative and adjusts over time. In an embodiment, the compliance score, like a credit score, represents a patient's compliance over the last seven years. In other embodiments, shorter or longer time periods are used. In an embodiment, the compliance score represents a patient's compliance for all known prescribed tasks.

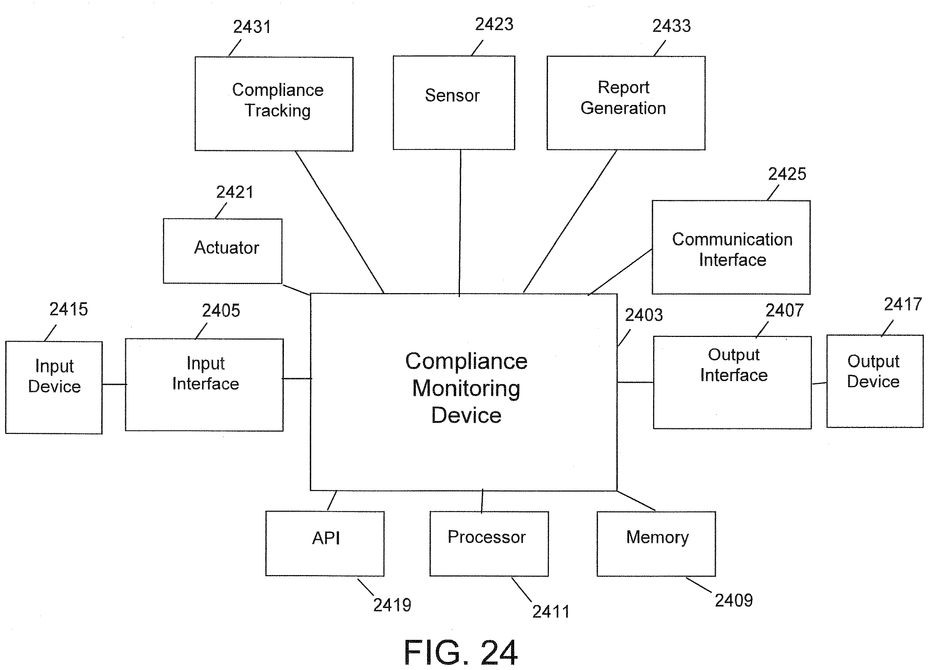

FIG. 24 is a functional block diagram of a compliance monitoring device **2403** in accordance with an embodiment of the subject invention. The device **2403** is only an illustrative embodiment of the invention. Other embodiments of such a device may include more, fewer, or different components. Or the components shown may be differently arranged.

In an embodiment, all or part of the compliance monitoring device **2403** is incorporated into another available device. For example, the compliance monitoring device **2403** can be incorporated into: a dispensing device, such as the dispensing device **1300;** a user device, such as the user device **2523A;** a Disease Management System, such as the Disease Management System **1201;** a Diagnostic Module, such as the Diagnostic Module **1203;** a Diagnostic Interpretive Module, such as the Diagnostic Interpretive Module **1205;** a Prescriptive Module, such as the Prescriptive Module **1207;** a Dispensing Module, such as the Dispensing Module **1209;** a Feedback and Patient Management Module, such as the Feedback and Patient Management Module **1211;** among other devices.

In the embodiment shown, the compliance monitoring device **2403** includes an input interface **2405,** an output interface **2407,** memory **2409** for program storage and/or data storage, and a processor **2411** for processing information. In an embodiment, the input interface **2405** includes an input device **2415** such as a mouse or other pointing device, a keyboard, a touch screen, or other input device known in the art. Other input devices for receiving information are known in the art and can be used with the subject invention. In an embodiment, the input interface **2405** serves to translate data received from the input device **2415** into a format usable by the compliance monitoring device **2403.** Thus, the input device **2415** and the input interface **2405** can be replaced without modifying the compliance monitoring device **2403** as known in the art. In an embodiment, the input device **2415** and/or the input interface **2405** are used to receive input from a user of the compliance monitoring device **2403.** In an embodiment, the user can set one or more parameters dictating operation of the compliance monitoring device **2403** via the input device **2415** and/or the input interface **2405.** In an embodiment, the manner in which compliance information is developed or presented can be set or modified via the input device **2415** and/or the input interface **2405.** In an embodiment, information input received via input device **2415** and/or the input interface **2405** can be incorporated into a compliance report or otherwise transmitted by the compliance monitoring device **2405.**

In an embodiment, the output interface **2407** includes an output device **2417** such as a monitor, printer, projector, or other display device, or a speaker or other audio device. Other output devices for presenting information are known in the art and can be used with the subject invention. In an embodiment, the output interface **2407** serves to translate data received from the compliance monitoring device **2403** into a format usable by the output device **2417.** Thus, the output device **2417** and the output interface **2407** can be replaced without modifying the compliance monitoring device **2403** as known in the art. In an embodiment, the output device **2417** and/or the output interface **2407** can be used to present information to a user of the compliance monitoring device **2403.** In an embodiment, the output device **2417** and/or the output interface **2407** can be used to present compliance information, a compliance report, a compliance score, and/or other information. In an embodiment, information received via a communication interface **2425** can be presented via the output device **2417** and/or the output interface **2407.** For example, an alert message can be presented as discussed above. In an embodiment, such an alert message can be generated by the compliance monitoring device **2403** itself.

In an embodiment, the compliance monitoring device **2403** includes a communication interface **2425** for transmitting information as discussed above. In an embodiment, the communication interface **2425** is a network or other communication interface, such as a modem, Ethernet controller, or other communication interface capable of supporting wireless or wireline communication. In an embodiment, the communication interface **2425** is an internal interface, such as a bus.

In an embodiment, the compliance monitoring device **2403** includes an application interface **2419** for sharing information with other applications. For example, the compliance monitoring device **2403** can include an interface for communicating with an electronic medical records (EMR) system. In an embodiment, the memory **2409** includes computer-readable media embodying a computer-program product as described above. In an embodiment, the memory **2409** includes a database or other device for data storage. Other memory devices are known in the art and can be used with the subject invention. In an embodiment, the compliance monitoring device **2403** includes multiple input interfaces **2405,** output interfaces **2407,** memories **2409,** processors **2411,** input devices **2415,** output devices **2417,** communication interfaces **2425,** or APIs **2419.**

In an embodiment, the compliance monitoring device **2403** includes a sensor **2423** for sensing information relevant to patient compliance. In an embodiment, two or more sensors can be included. As described above, such sensors can sense various information regarding the physical environment surrounding the compliance monitoring device **2403.** For example, in an embodiment, such sensors can sense the movement (or lack of movement) of a medicament or medicament packaging. As known in the art, various sensors can be used to directly or indirectly sense movement, such as a pressure sensor, light sensor, a strain gauge, a proximity sensor, among other sensors. In a particular embodiment, the packaging can include an RFID tag and an RFID system can be used. In another embodiment, such sensors can include testing equipment for measuring patient vitals, such as blood pressure, heart rate, weight. In a further embodiment, such sensors can analyze bodily fluid and/or tissue of a patient. For example, glucose meters can measure the amount of glucose in the patient's blood. Other testing equipment can be used to measure other bodily fluid and/or tissue of the patient. The examples provided here are illustrative. Other sensors are known in the art and can be used with the subject invention.

In an embodiment, the compliance monitoring device **2403** includes an actuator **2421** for affecting the physical environment surrounding the compliance monitoring device **2403.** In an embodiment, two or more actuators can be included. In an embodiment, such actuators are used to dispense medicaments. Various dispensing techniques can be used with the subject invention. A few illustrative examples are described above. In embodiments, such actuators work in concert with one or more sensors to collection information regarding the physical environment surrounding the compliance monitoring device **2403.** The examples provided here are illustrative. Other actuators are known in the art and can be used with the subject invention.

In an embodiment, the compliance monitoring device **2403** includes one or more program modules, such as a compliance tracking module **2431,** and/or a report generation module **2433.** Various functions of the compliance monitoring device **2403** can be performed, controlled, or facilitated by these or other program modules. As discussed above, the functions can be distributed in various manners. In an embodiment, the compliance tracking module **2431** functions to develop compliance information from one or more signals received from one or more sensors **2423,** as described above. In an embodiment, the report generation module **2433** functions to organize such compliance information into a report for distribution and/or presentation to one or more recipients, as described above.

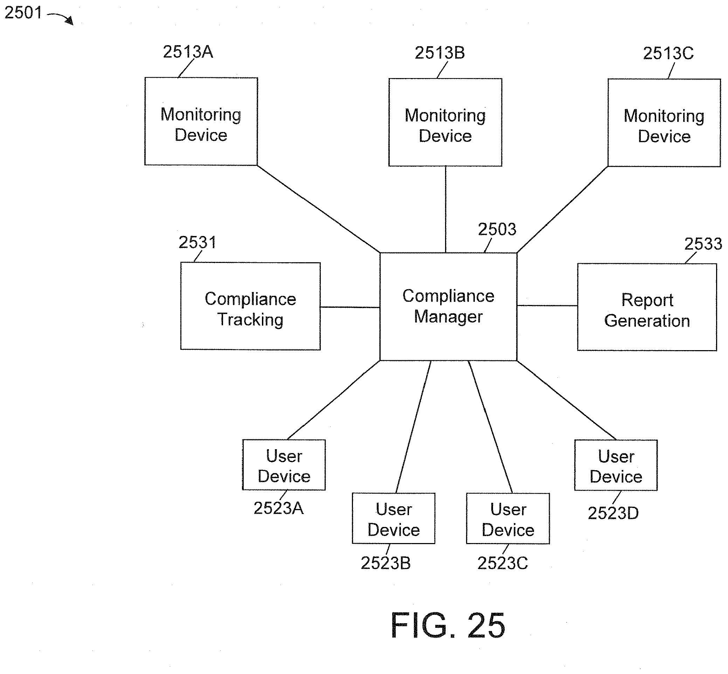

FIG. 25 is a functional block diagram of a compliance management system **2501** in accordance with an embodiment of the subject invention. The system **2501** is only an illustrative embodiment of the invention. Other embodiments of such a system may include more, fewer, or different components. Or the components shown may be differently arranged.

In the embodiment shown, the compliance management system **2501** includes a compliance manager module **2503,** one of more compliance monitoring devices **2513A-C,** and one or more user devices **2523A-C.** In a further embodiment, the compliance management system includes a plurality of compliance monitoring devices **2513A-C.** In a further embodiment, the compliance management system includes at least three compliance monitoring devices **2513A-C.** In a further embodiment, the compliance management system includes a plurality of user devices **2523A-C.** In a further embodiment, the compliance management system includes at least three user devices **2523A-C.**

The compliance monitoring devices **2513A-C** gather and/or generate compliance information, compliance reports, or other information and transmit such information to the compliance manger module **2503.** The compliance manager module **2503** then transmits compliance information, compliance reports, or other information to one or more user devices **2523A-C.** In an embodiment, such information is transmitted over a backplane. In another embodiment, such information is transmitted via one or more wired or wireline communication technologies known in the art. In a further embodiment, the communication is two-way. For example, the compliance manager **2503** can transmit requests for compliance information, compliance information, alerts, or other transmissions to the compliance monitoring devices **2513A-C.** The user devices **2523A-C** can also transmit requests for compliance information, compliance information, alerts, or other transmissions to the compliance manager module **2503.** In an embodiment, a single device performs the functions of both a monitoring device and a user device.

The compliance monitoring devices **2513A-C** can include such devices as the compliance monitoring device **2403,** the dispensing device **1300,** and other devices capable of gathering and/or generating compliance information, and transmitting such information. In a particular embodiment, one of the compliance monitoring devices **2513A-C** gathers and/or generates, and transmits the type of compliance information shown on the interface **2001.**

The user devices **2523A-C** can include various client devices or other computers, wherein each such computer is accessible to a user of the compliance monitoring system **2501** and capable of receiving some or all of the transmitted compliance information, compliance reports, or other information. In an embodiment, some or all of the user devices **2523A-C** are also capable of processing, storing, and/or presenting such information. In an embodiment, the user is a human being such as a patient or physician. In an embodiment, the user is an organization such as a company, government agency, or NGO. In an embodiment, such information is presented to a user via a web portal.

Various users may be interested in such compliance information for various purposes. For example, in an embodiment, one or more of the user devices **2523A-C** are accessible to a patient that is a subject of the compliance information or reports. The patient can receive alerts or other time-sensitive information via a technology meant to interrupt the activities of the patient. For example, the patient can receive an alert regarding a missed dose or exercise session via a mobile phone, PDA, pager, or similar device. The patient can also receive an alert message when it is time to order more of a prescribed medicament. In addition, the patient can receive progress reports via periodic emails (e.g., weekly, monthly) or other messages. In an embodiment, each patient can customize the content, format, timing, and/or delivery mode of the alerts and/or reports. In an embodiment, the patient can pull such information from a web portal as desired.

In a further embodiment, such compliance information or reports are also accessible to a physician via one or more user devices. In an embodiment, the physician can monitor compliance of some or all of the physician's patients via the compliance management system **2501.** In an embodiment, the physician receives alerts of emergent information via mobile phone, PDA, pager, or similar device. In an embodiment, the physician receives summary reports via periodic emails or other messages. Important information can be highlighted in these reports to draw the physician's attention. For example, particularly poor compliance or abnormal blood chemistry results can be presented with colored or flashing text. Other methods of highlighting information are known in the art and can be used with the subject invention. As with a patient, the physician can customize the content, format, timing, and/or delivery mode or the alerts and/or reports. In an embodiment, the physician can also access the compliance monitoring system when needed to obtain information about a particular patient, such as in advance of or during an office visit with the patient. In an embodiment, the compliance management system **2501** provides an alternative to a complete EMR system.

In a particular embodiment, one or more of the user devices **2523A-C** are accessible to a third-party payor, such as a health insurance company or health maintenance organization (HMO) that uses the compliance management system **2501** to obtain compliance information or reports regarding its program participants. In a further embodiment, the third-party payor varies the terms of the participant's contract based on the compliance information, reports, or score. For example, a program participant can be rewarded for high compliance with reduced premiums, co-pays, or deductibles. Conversely, the program participant can be punished for poor compliance with higher premiums, co-pays, or deductibles. In an embodiment, a monetary reward or penalty is applied. In an embodiment, non-monetary contract terms are varied. In an embodiment, poor compliance is a reason for nonrenewal.

In another embodiment, one or more of the user devices **2523A-C** are accessible to a pharmacy or other advertiser. In an embodiment, the advertiser uses information from the compliance management system **2501** to send targeted advertisements to patients, customers, or prospective customers. For example, the advertiser can send a message to a patient when it is time to order more of a particular medicament sold by the advertiser. In an embodiment, the advertiser sends the message as an alert via the compliance management system **2501.** In another embodiment, the advertiser sends the message via a delivery mode outside the compliance management system **2501.**

In another embodiment, one or more of the user devices **2523A-C** are accessible to a pharmaceutical company or other researchers performing drug trials or other studies. Such researchers can use compliance information from the compliance management system **2501** to determine whether study participants have taken the drug as prescribed in the study. Unexpected and/or negative results may be explained, if one or more of the study participants did not take the drug as prescribed in the study. In a particular embodiment, one or more of the user devices **2523A-C** are accessible to the Food and Drug Administration (FDA). As part of its Sentinel Initiative, the FDA has taken an active role in monitoring drug safety. The FDA can access compliance information via the compliance management system **2501** in order to verify that patient reporting an adverse drug event took the drug as prescribed. The Department of Health and Human Services (HHS), and other interested state, federal, and nongovernment agencies can also access compliance information for various purposes. For example, an interested agency can access compliance information via the compliance management system **2501** in order to monitor completion of prescribed courses of antibiotics.

In an embodiment, the compliance manager module **2503** adjudicates the compliance information transmitted to each of the user devices **2523A-C.** In an embodiment, such adjudication includes the content, format, timing, and/or delivery mode of a compliance report transmitted to the user device. Such content, format, timing, and/or delivery mode can be customized as described above. In a further embodiment, the content, format, timing, and/or delivery mode can vary depending on the type of user device receiving the transmission. In an embodiment, the content, format, timing, and/or delivery mode is customized based on user preferences.

In an embodiment, the compliance manager module **2503** includes one or more program modules, such as a compliance tracking module **2531,** and/or a report generation module **2533.** Various functions of the compliance monitoring device **2503** can be performed, controlled, or facilitated by these or other program modules. As discussed above, the functions can be distributed in various manners. In an embodiment, the compliance tracking module **2531** and/or the report generation module **2533** perform the respective functions of the compliance tracking module **2431** and/or the report generation module **2433** described above. In an embodiment, these functions are shared across the respective modules included in the monitoring devices **2513A-C** and the compliance manager module **2503.**

In an embodiment, the compliance manager module **2503** includes an authentication, authorization, and accounting (AAA) module (not shown). As known in the art, such an AAA module can be used to limit access to the compliance management system **2501** and/or facilitate billing for use of the system. Various billing schemes can be used. For example, a user can pay for a site license, access over a month or other period of time, and/or for individual transactions or reports. In an embodiment, advertisers access the compliance information one the compliance management system **2501,** but pay for advertisements delivered via the system.

In an embodiment, the compliance manager module **2503** is resident on a central server. In an embodiment, multiple servers can be used (not shown). In an embodiment, the servers function redundantly. In an embodiment, the servers each serve subsets of the monitoring devices **2513A-C** and/or the user devices **2523A-C.** In an embodiment, the servers each process compliance information related to different aspects of a treatment regimen. For example, one server can be tasked with processing compliance information related to medication, while another can be tasked with processing compliance information related to diet. In an embodiment, a peer-to-peer architecture is used, in which monitoring devices **2513A-C** communicate directly with user devices **2523A-C.** In an embodiment, the compliance manager module **2503** facilitates establishment of the peer-to-peer connections. In an embodiment, all or part of the compliance management system **2501** and/or the compliance manager module **2503** are incorporated into another available device. For example, they can be incorporated into: a compliance monitoring device, such as the compliance monitoring device **2403;** a dispensing device, such as the dispensing device **1300;** a user device, such as the user device **2523A;** a Disease Management System, such as the Disease Management System **1201;** a Diagnostic Module, such as the Diagnostic Module **1203;** a Diagnostic Interpretive Module, such as the Diagnostic Interpretive Module **1205;** a Prescriptive Module, such as the Prescriptive Module **1207;** a Dispensing Module, such as the Dispensing Module **1209;** a Feedback and Patient Management Module, such as the Feedback and Patient Management Module **1211;** among other devices. Various other manners of distributing the functions of the compliance management system **2501** and/or compliance manager module **2503** are possible and can be used with the subject invention.

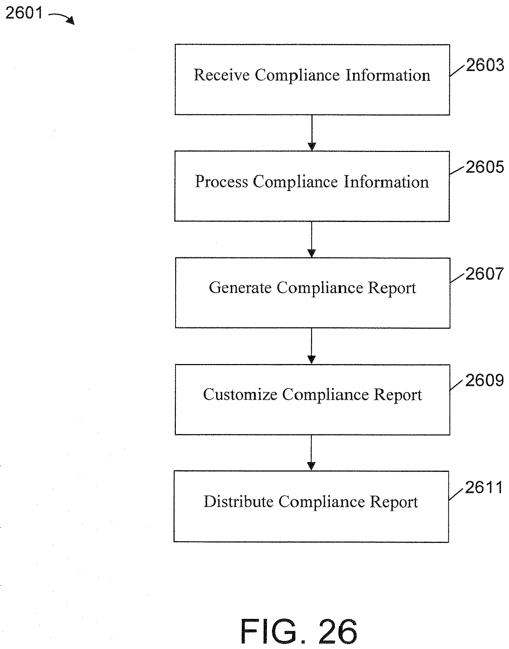

FIG. 26 illustrates a method **2601** for tracking and/or using patient compliance information in accordance with an embodiment of the subject invention. The method **2601** is only an illustrative embodiment of the invention. Other embodiments of such a method may include more, fewer, or different steps. Or the steps shown may be differently arranged.

In the embodiment shown, at a step **2603,** compliance information is received. In an embodiment, the compliance information is received from a compliance monitoring device such as the compliance monitoring device **2403.** In an embodiment, the compliance information is received from a user device such as the user devices **2523A-C.** In an embodiment, the compliance information is received from a sensor such as the sensor **2423.** In an embodiment, such information is received from multiple sources, including zero or more compliance monitoring devices, user devices, sensors, or other sources. In an embodiment, the step **2603** can be repeated and additional compliance information can be received over time. As discussed above, such compliance information can be transmitted using various communication technologies. In an embodiment, such information is transmitted over a backplane. In another embodiment, such compliance information is transmitted via one or more wired or wireline communication technologies known in the art. In an embodiment, various intermediate devices relay such compliance information from a source before it is received at the step **2603.**

In an embodiment, the compliance information includes relevant evidence to the issue of whether a patient has complied with an aspect of a prescribed treatment regimen. According to Federal Rules of Evidence, Rule 401: "Relevant evidence' means evidence having any tendency to make the existence of any fact that is of consequence ... more probable or less probable than it would be without the evidence. Both direct and circumstantial evidence can be considered. For example, in an embodiment, a statement by the patient, received via a user interface, indicating that the patient took a prescribed pill is received at the step **2603.** In another embodiment, a signal from a sensor indicating that a MUDP has moved is received at the step **2603.** In other embodiments, weight, heart rate, blood pressure or other measurements of the patient can be received as compliance information at the step **2603.** For example, if the patient's weight has increased over time, it is less likely that the patient is complying with diet and/or exercise aspects of a prescribed weight loss treatment regimen. In a further embodiment, test results of bodily fluid or tissue of the patient can be received as compliance information at the step **2603.** The examples presented herein are illustrative. Other compliance information can be received at the step **2603.**

At a step **2605,** the compliance information received at the step **2603** is analyzed or otherwise processed. Such processing can include decoding or otherwise interpreting the compliance information received. Such analysis can include combining various pieces of compliance information received. For example, compliance information received from one source can be compared or contrasted with information received from another source. In an embodiment, when multiple sources are in agreement a confidence rating associated with the compliance information is increased. Conversely, when sources disagree the confidence rating is decreased. In an embodiment, such information is combined with other information received from data storage, an input interface, and/or a communication interface to inform compliance information. For example, the patient's current weight can be compared to stored values for the patient's weight at different points in time. In an embodiment, the step **2605** can be repeated and additional compliance information received since the last iteration of the step can be included in the next iteration of the step. In a further embodiment, a confidence rating associated with compliance information is thereby updated in the next iteration of the step.

At a step **2607,** the processed compliance information from the step **2605** is used to generate a compliance report. As discussed above, the compliance information can be rolled-up or otherwise summarized, calculations can be performed with the compliance information to yield useful results, or the content of the information can be sorted, filtered, or otherwise formatted. In an embodiment, the compliance report includes a compliance score which represents the patient's degree of compliance with the aspect of the prescribed treatment regimen. In an embodiment, the compliance report includes a confidence rating for the compliance information and/or compliance score. As discussed above, in an embodiment, the compliance report and/or compliance score can combine information related to: multiple aspects of the prescribed treatment regiment; multiple treatment regimens; and/or multiple patients.

At a step **2609,** the compliance report from the step **2607** is customized according to various preferences. In an embodiment, as discussed above, the content, format, timing, and/or delivery mode of the compliance report is customized for the recipient of the report. In an embodiment, the report is customized based on the type of user receiving the report. In an embodiment, the report is customized based on the preferences or characteristics of different user populations or particular users.

At a step **2611,** the customized compliance report from the step **2609** is distributed to one or more users. In an embodiment, the compliance report is distributed to a plurality of users. In an embodiment, the compliance report is distributed to at least three users. In an embodiment, the compliance report is presented to at least one user via one or more user interfaces. In an embodiment, the compliance report is transmitted to one or more user devices such as the user devices **2523A-C.** In an embodiment, the compliance report is distributed to a plurality of user devices. In an embodiment, the compliance report is distributed to at least three user devices. In an embodiment, the compliance report is transmitted over a backplane. In another embodiment, the compliance report is transmitted via one or more wired or wireline communication technologies known in the art. In an embodiment, the compliance report is transmitted according to a preferred delivery mode as discussed above. In an embodiment, the steps **2607, 2609,** and/or **2611** are repeated to produce and/or distribute additional and/or updated reports.

In an embodiment, one or more of the steps of the method **2601** are preformed by one or more suitably programmed computers. In a particular embodiment, at least one of the steps **2605, 2607, 2609,** or **2611** are preformed by the one or more suitably programmed computers. In a particular embodiment, at least one of the steps **2605, 2607,** or **2609** are preformed by the one or more suitably programmed computers. In an embodiment, the one or more suitably programmed computers comprise a compliance management system **2501,** a compliance manager module **2503,** and/or a compliance monitoring device **2403.**

In an embodiment, information can be transmitted wirelessly via radio frequency (RF) transmission as known in the art. Various frequency bands, encoding methods, and protocols can be used. For example, VLF, LF, MF, HF, VHF, UHF, SHF, EHF, and/or other frequency bands can be used. AM, FM, and/or other known encoding methods can be used. Bluetooth, Wi-Fi (IEEE Standard 802.11), WiMax (Worldwide Interoperability for Microwave Access and IEEE Standard 802.16), Zigbee (IEEE Standard 802.15.4), and/or other known communication protocols can be used.

Embodiments of the subject invention can be used in treating various diseases and conditions including, but not limited to, glaucoma, leukemia, HIV, infectious diseases, kidney disease, obesity, cancer, diabetes, CHD, and CVD.

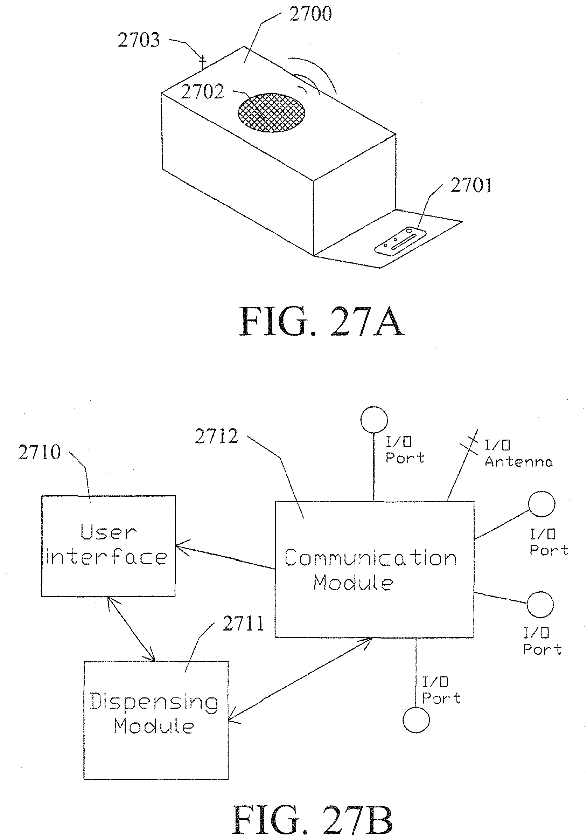

In accordance with certain embodiments of the subject invention, a compliance monitoring device, for example, the dispensing device such as described with respect to FIG. 13 is provided that includes capabilities beyond dispensing of medicaments and monitoring of user/patient compliance. FIG. 27A shows an example of such a dispensing device according to an embodiment of the invention. For example, referring to FIG. 27A, the dispensing device **2700** can be an enclosed container with a portion that opens for dispensing medicine **2701.** When a designated time for a patient to take the dispensed medicine occurs, the dispensing device **2700** can send a reminder signal to the patient. The reminder signal can be provided via a speaker **2702** or transmitted (via an antenna **2703** or connector) to another device over a wireless, wire, or hybrid (combination of wired and wireless) network. The reminder signal can be simple beeping or other alarm sound and/or a recorded message. The dispensing device **2700** can monitor the patient's compliance as well as non-compliance with a prescribed regimen. Information regarding the patient's compliance can be transmitted via the antenna **2703** or hard connection over wireless, wired, or hybrid networks to a health professional.

FIG. 27B shows a diagram of a system for a dispensing device according to an embodiment of the invention. As shown in Figure 27B, a user can interact with the dispensing device via a user interface (user interface module) **2710,** which communicates with the dispensing module **2711,** which controls delivery of the medicine contained in the dispensing device, and the communication module **2712,** which controls transmission and receipt of data out of and into communication ports (I/O ports and/or antennas) of the dispensing device. The communication module **2712** can be in communication with the user interface **2710** to provide data to the user from a device or component connected to the dispensing device via one of the communication ports. The communication module **2712** can also be in communication with the dispensing module **2711** in order to allow for remote control of the delivery of the medicine contained in the dispensing device.

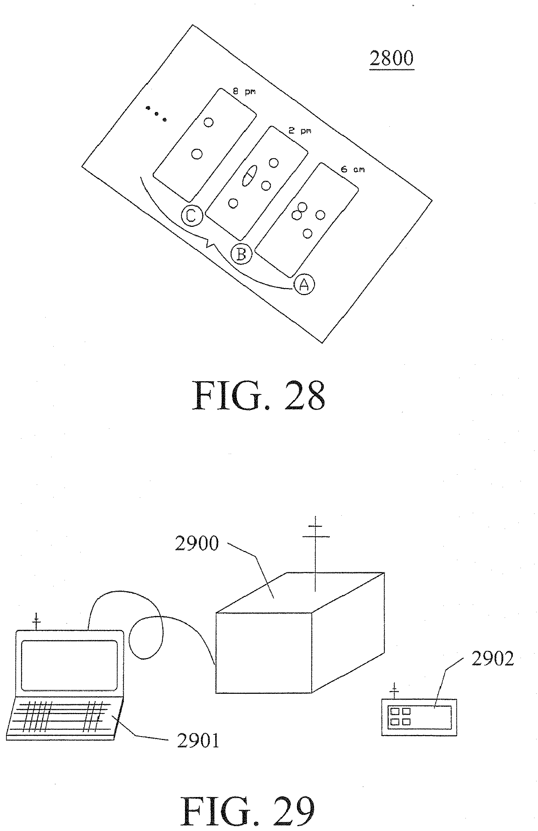

As previously described, the subject dispensing device is portable and can be provided to a patient with a prescribed number of packages or pouches of medication for use therein. The dispensing device can be provided to a patient in order to assess the degree of the patient's compliance with their therapeutic regimen while the patient is at home, at work, or at any place away from their health care professional's office. The portability enables a patient to carry and use the dispensing device anywhere. The medication may be pre-inserted by a pharmacy or other provider into the dispensing device, or refill packages may be inserted by the user/patient or caregiver. For example, referring to FIG. 28, the dispensing device **2800** can hold multiple medication packages including A, B, and C packages. Each medication package can include a combination of different pills. The combination in each package can be the same or different than that in other packages in the dispensing device **2800.** The dispensing device can dispense each medication package at a predetermined time. For example, package A can be scheduled for 6 am, package B can be scheduled for 2 pm and package C can be scheduled for 8 pm. This scheduled dispensing arrangement can be programmed to repeat each day until an ending date determined by a health professional according to a patient's health condition.

The dispensing device includes an enclosure that holds the medication packages. This enclosure can be locked to inhibit unauthorized access to the medication. In certain embodiments, the lock can be controlled automatically by programs in the dispensing device or by remote control by a third party. For example, a third party may lock the enclosure to prevent access to the medication.

Security of the medicine contained in the device and/or the data obtained by the compliance monitoring device can be important to maintain. For example, it can be important to ensure that the medication is being used by the appropriate patient in order to protect against unlawful distribution of controlled substances and to improve the likelihood that the patient is receiving the correct medication and not the medication of another patient. In addition, certain aspects of the compliance data may be required to be kept confidential in accordance with certain Patient Privacy Acts. Accordingly, in certain embodiments of the invention, the device includes security features such as biometric readers (or any security device that uses a biometric signature), personalized identification devices, and encryption capabilities. The biometric reader can determine via finger print analysis or other biometric signature of the patient or user in order to validate identity. The personalized identification device can be a dongle or thumb drive that can be connected via a USB or other communication port of the dispensing device to unlock and/or permit certain applications or access. The encryption can be used in the transmission of sensitive or private information over the network.

The dispensing device can be programmed to dispense medicine at a time to the patient according to a health professional's recommendation or prescribed regimen. When it is time for a patient to take a prescribed medicine, the dispensing device can provide a first signal (e.g., a sound) to remind the patient the time for taking medication. According to one embodiment, before dispensing of medicine to a patient, the dispensing device validates the patient's identity using a biometric signature of a patient such as fingerprint, retina, and/or voice. Once the patent is identified as the correct patient, the dispensing device can dispense the medicine out of a slot or aperture of the dispensing device or allow the patient to open the dispensing device and take a dispensed medicine (or package or pouch of medicines). The dispensing device may be opened by the patient's touch or voice command. For a touch-activated dispensing device, the dispensing device has a touch-sensitive region or mechanically operated fixture that allows the patient (or user) to open the dispensing device. While the dispensing device is touched, the patient's physical information can be obtained in real time. For example, information such as blood pressure, pulse, sugar level, and body temperature can be obtained. For a voice-activated dispensing device, the dispensing device opens upon a predetermined word. The dispensing device can include sensors for determining pitch, duration, and intensity in order to match to the voice-activation word.

Data can be transmitted to and from the dispensing device via wired or wireless networks (or a combination of wired and wireless networks). Peripheral devices can be used with the dispensing device to provide additional information regarding compliance and/or health of the patient. The dispensing device can communicate with the peripheral devices by direct connection between the dispensing device and the peripheral devices via a wired connection, including, but not limited to, Universal Serial Bus (USB), High Speed Serial Bus (e.g., FIREWIRE), and other electrical connectors (e.g., XLR, Video, plug/socket), or by remote communication via a wireless spectrum, including but not limited to, cellular, GSM, and Bluetooth. For example, as shown in FIG. 29, the dispensing device **2900** can have a direct connection to a first peripheral device such as computer **2901** via a wired network and/or a remote communication via a wireless network. Similarly, the dispensing device can remotely communicate with a second peripheral device such as smart phone **2902** by a wireless network.

The peripheral devices can provide medical and/or non-medical information to the dispensing device for internal processing by the dispensing device, transmission of the information to another device via the dispensing device, or for display to the user/patient of the dispensing device. The display can be visual and/or audio-based.

The dispensing device can include an embedded or remote (separate) camera (the camera may include a combined video camera and microphone for picking up audio as well as visual data). The camera can be used for monitoring the patient for direct observational therapy or for patient identity verification. In certain embodiments, the camera can be activated remotely or non-remotely. In one embodiment, the camera can be used to view the patient and the patient's pathological state.

The dispensing device can include a full color LCD display, LED display, or other screen or display that can communicate information to the patient. The display may be detachable. The display can include user input elements, such as a touch screen or buttons that enable interaction with the dispensing device and communication systems of the dispensing device.

The inclusion of an embedded camera and display screen can enable video conferencing via cellular connection video camera and sound technology to interface with healthcare providers and family members. Therefore, a third party viewing the patient (such as a healthcare provider, caregiver, or family member) can interact with the patient over a video chat or teleconference. The communication can be via cellular, WiFi, or any other method for transmission of data to which the dispensing device is configured to use.

In addition, emergency services can be provided, where patients may alert their caregivers, family members or emergency services (EMS) that they are in distress. For example, patients may use the inputs of the dispensing device related to the video or phone conferencing capabilities to alert their caregivers or emergency services that they are in distress. An optional GPS location service can be included to enable the patient's HIPPA approved healthcare ecosystem members and/or emergency services to be time sensitively dispatched to the patient's location.

In one embodiment, the dispensing device can include or can interact with a peripheral device having a microphone and/or speaker for use in communicating over a network such as a cellular or telephony network. Such embodiments can provide functionality similar to an emergency phone. The dispensing device can include a telephone that uses VOIP or other communication spectrum. The telephone can be integrated with a display screen of the dispensing device. In certain embodiments, the telephone is removable. The removability of the telephone can be full or partial. That is, the telephone may be fully removable and used separately from the dispensing device as a phone, smart phone, or other communication device, or the telephone may be partially removable such that certain functionality and communication remains with the dispensing device (for example, similarly to a portable phone).

The telephone can be a functional 3G (or 4G or cable or other network) enabled VOIP telephone. Patients can have the ability to communicate globally at low cost with enhanced clarity when using the telephone.

In addition to applications related to healthcare or communication, software applications for maps, games, can be incorporated into or used with the dispensing device.

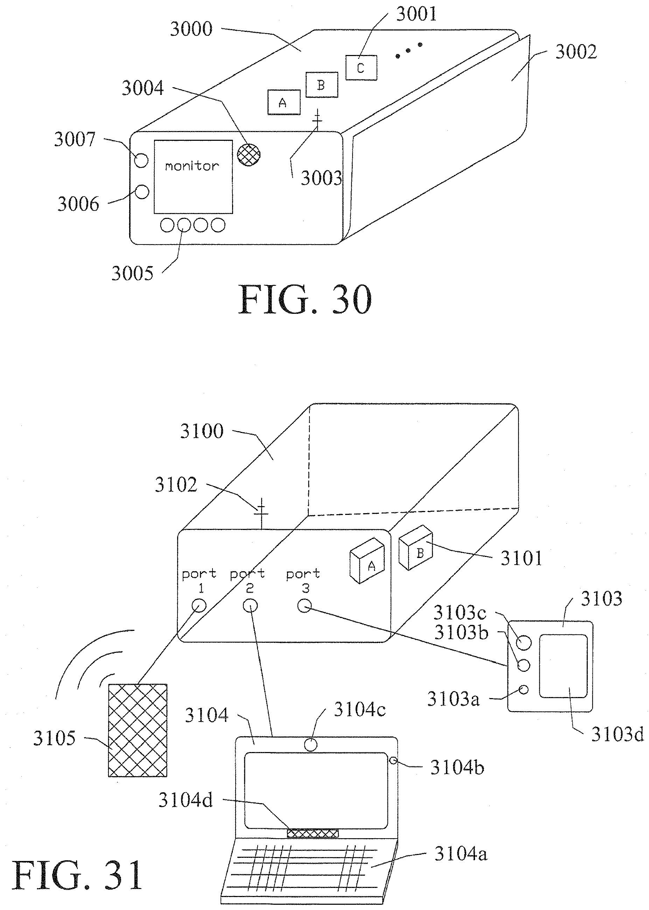

In one embodiment, the dispensing device can be a stand-alone device including a speaker, a microphone, a display screen, a keypad or representation provided on the display screen when the display screen is a touch screen, at least one sensor, a camera, and/or an antenna for bidirectional communication between a patient and a health professional or a non-medical personnel. The dispensing device can contain a range of processing power and components depending on the particular implementation. The dispensing device can be a stand-alone device **3000** as shown in FIG. 30, which contains all the necessary built-in hardware and software to perform a number of tasks. For example, the stand-alone device **3000** can contain medicine packages **3001** that can be dispensed out of the dispensing region **3002.** One or more antennas **3003** can be provided to communicate across a wireless spectrum. The device **3000** can further include at least one of a speaker **3004,** key pad **3005,** microphone **3006,** camera **3007,** and monitor/display **3008.** In one embodiment, the antenna, keypad (either part of a touch screen of the monitor **3008** or a separate keypad), microphone, camera, speaker, and monitor portion can be detachable. Of course, in certain embodiments, peripheral devices could be connected to the stand-alone device **3000** to provide additional features and/or provide measurements or data to the stand-alone device **3000.**

The dispensing device may be a low-processing (or dummy) type device. The low-processing dispensing device can allow for connection to a smart device (or combination of devices) providing a speaker, microphone, touch screen, sensor, and/or an antenna to make available functions of the peripheral device to the low-processing dispensing device. One such smart device is a smart phone.

For a low-processing (or dummy) type dispensing device such as shown in FIG. 31, one or more peripheral devices can be connected to the dispensing device in order to provide processing capabilities (e.g., advanced programs/software). For example, a low processing type dispensing device **3100** can contain medicine packages **3101** that can be dispensed from the dispensing device **3100** according to instructions programmed on the dispensing device **3100** or by a peripheral device, such as a smart phone **3103** or computer **3104,** providing the controls for the dispensing device **3100.** The dispensing device **3100** can include one or more communication ports (such as port 1, port 2, and port 3) for a wired connection and/or one or more antennas **3102** to communicate across a wireless spectrum. For the low processing type dispensing device **3100** one or more of a speaker, keypad (either part of a touch screen or a separate keypad), microphone, camera, speaker, and monitor/display can be provided via a peripheral device in communication with the dispensing device **3100.** Referring to FIG. 31, a smart phone **3103** can be connected either by a cable or a wireless spectrum such as Bluetooth to the dispensing device **3100** in order to provide components and functionality for the dispensing device **3100.** Such components and functionality can include, but are not limited to a speaker **3103a,** microphone **3103b,** camera **3103c,** display **3103d,** and keypad/touch screen (can be associated with display **3103d).** Similarly, a computer **3104** can be connected to the dispensing device wired or wirelessly to provide processing power and additional features available with the computer (such as a keypad **3104a,** a microphone **3104b,** camera **3104c,** or speaker **3104d**). In certain embodiments, peripheral devices, such as a speaker **3105** can be provided to extend the distance from which a sound transmitted from a connected phone **3103,** computer **3104,** or the dispensing device **3100** can be heard.

The dispensing device can include software or operating systems directed to specific program applications that are customizable to the patient or specific task. As one example, the software can include applications for interacting with the peripheral devices, for alerting emergency medical services of need for help, or to send out the location of the dispensing device. The dispensing device can include internal hardware or software providing for GPS or cellular triangulation methods, and can include gyros or accelerometers for determining orientation or movement of the dispensing device.

The dispensing device can track the movement (or non-movement) of the packages or pouches contained in the dispensing device. The tracking can be accomplished via barcode or RFID scanning within the dispensing device, weight sensing, and/or movement sensing of the packages or pouches or the mechanism that causes or allows the packages or pouches to move within the dispensing device.

As described above, the dispensing device can be in the form of a portable unit that contains pouches of packages that are customized to the patient. The packages, either in series or not in series, can be loaded into the dispensing device. When the appropriate time for dispensing occurs, the dispensing device can alert the patient visually and/or with sound that the next dose is ready to be taken by the patient. The dispensing time can be programmed in the dispensing device or remotely controlled. If the patient fails to comply, the device can alert the patient by sending out a signal of non-compliance.

The dispensing device can automatically advance the roll or series of pouches of medication. In addition, the dispensing device can control and keep track of the medications being dispensed. According to certain embodiments, if the patient fails to take the medication within a specific time period, then the dispensing device can store the medication in a separate or attached compartment. The removal and/or storage of pouches not taken by the patient can be used to track compliance.

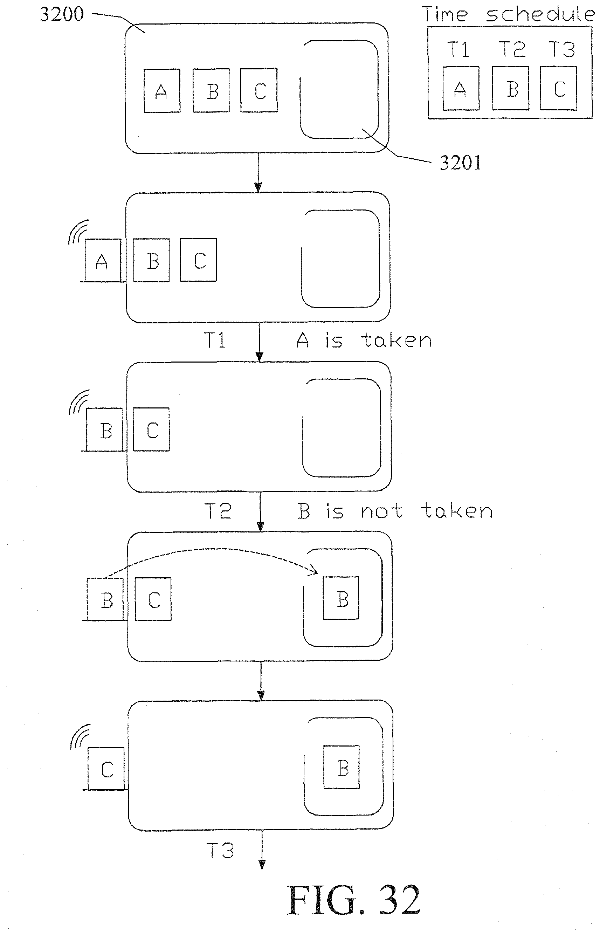

For example, referring to FIG. 32, the dispensing device **3200** can be configured to dispense a series of pouches A, B, and C according to a set time schedule, where each pouch can be of multiple or singular medicine. If the patient takes pouch A during the appropriate time period **(T1),** but misses the time period **(T2)** for taking pouch B, the dispensing device can remove B from the line of pouches and store B in the device (storage region **3201**) for later disposal or use. Then, pouch C will be next in line to be dispensed during the time **(T3)** for taking pouch C.

A caregiver/administrator may also remotely or non-remotely choose to remove certain medications from the dispensing device, and the dispensing device can then store these selected medications when signaled remotely (wireless) or non-remotely. Therefore, a third party may, over a network or other communication platform, change the order or remove certain individual pouches from the dispensing device or change the order to be dispensed to the patient.

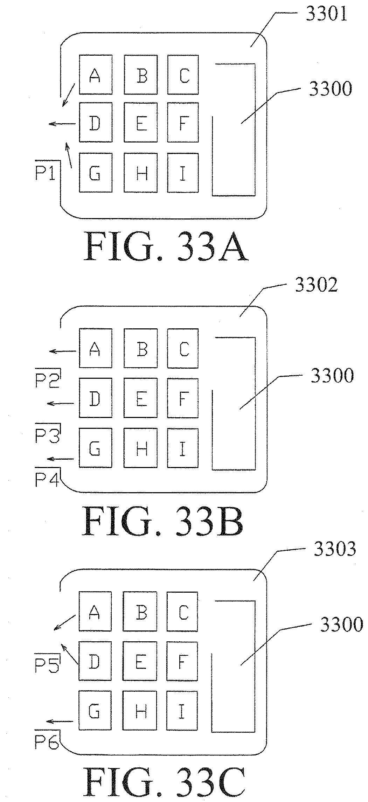

In certain embodiments, the dispensing device can contain multiple lines of pouches. For example, referring to FIGS. 33A-33C, the dispensing device can be configured to dispense a first line with a series of pouches A, B, and C, a second line with a series of pouches D, E, and F, and a third line with a series of pouches G, H, and I. The dispensing device can include one or more ports for dispensing the three different lines of medicine. One or more of the three lines can be dispensed via the same port. FIG. 33A shows using a same port **P1** to dispense the three lines, FIG. 33B shows using different ports **P2, P3,** and **P4** for each of the three lines, and FIG. 33C shows dispensing two of the three lines out of the same port **P5.** Each pouch of medication can be dispensed according to a particular time. If the patient fails to take a pouch during the appropriate time, the dispensing device can remove the pouch from a particular line for storage. It should be understood that although three lines are described in this example, the number of lines of packages are not limited to three. For example, the dispensing device can include one line of pouches through 100 lines of pouches, or more. Each line of pouches may contain, but are not limited to 1-5000 individual pouches of medication. The lines of pouches may be advanced forward or backward using different technologies, including but not limited to, separate or singular drives wheels or any other method to advance, cut, move, or store the medication pouches. These pouches may be attached to each other or may be separate from each other.

In a further embodiment, in addition to alerts regarding a missed dose or reminder to take a prescribed medicament, advertisements and/or coupons can be transmitted to the dispensing device and received by the patient/user of the dispensing device via a visual and/or audio output of the dispensing device or peripheral device in communication with the dispensing device. In one implementation, the dispensing device receives, via a transmission signal, advertising and coupons with a barcode (2D or otherwise) or other code system available for scan and use by the consumer. The coupon can be printed via a peripheral device in communication with the dispensing device. The coupon may also be visualized on a display of the dispensing device.

Embodiments of the subject dispensing device are configured for interaction with a variety of peripheral or satellite devices. Once the dispensing device is connected with peripheral devices, the user interfaces (Uls) of the peripheral devices can be merged to the dispensing device. In addition to the Uls of the peripheral devices, various applications of the peripheral devices can be accessed and used as long as the dispensing device is connected with the peripheral devices. The dispensing device can be attached and detached with the peripheral devices directly through one or more connection ports built in the dispensing device or via a wireless spectrum, including, but not limited to, cellular, GSM, and Bluetooth.

The dispensing device can interact wirelessly and seamlessly with multiple consumer health care devices in order to monitor, in real time, a patient's vital healthcare data. The dispensing device can transmit the information from the consumer health care devices to a database such as a cloud-based database. The information can be transmitted utilizing enhanced encryption technology and may be securely stored in the database. Examples of peripheral devices that the subject dispensing device can be configured to interact with include, but are not limited to, Blood Pressure (BP) monitoring devices, weight monitoring scale, breath or condensate analyzers, internal or external probe or sensor devices, glucometer monitoring the patient's Glucose levels in real time, Electrocardiograms (EKG) for cardiac patients, intraocular pressure (IOP) measurement devices for diagnosing glaucoma, other ophthalmologic pathology screening, diagnostic or treatment device, and "home-based" one drop blood or urine analysis devices. The information obtained from the peripheral devices can be accessible to the patient's healthcare ecosystem members or any other group or individual with access to the cloud database.

In certain embodiments, the dispensing device functions as an access point for other devices to access the database or other wired or wirelessly connected devices. For example, a peripheral device can communicate with the dispensing device to request access to a database containing information such as prior health care data or other related or relevant data. The peripheral device may be used to take a measurement and receive additional data via the dispensing device in order to perform a calculation or provide a comparison with measurements taken over time, measurements taken by another device, or measurements taken by another person. The final determination of the calculation, the comparison, and/or the measurement can be provided to the dispensing device for use in controlling the dispensing of medication, for storage in a database, or transmission to another party. In addition, the dispensing device can facilitate communication between two devices. For example, one peripheral device can provide a signal regarding a measurement or other data to the dispensing device, and the dispensing device can transmit the data to a second peripheral device for analysis or other purpose. In one embodiment, a communication module (such as communication module **2712** shown in FIG. 27B) controls access and routing of requests by peripheral devices.

As previously described, the dispensing device can include sensors that are internal, external, or removable from the dispensing device. These sensors can be provided for analyzing user-specific samples, such as, blood, urine, saliva, serous fluid, mucosal fluid, or another sample the patient may insert or place on the sensor(s). This patient data may be analyzed by the dispensing device or transmitted by the sensing device to another device for analysis. The data obtained from the sensor(s) can be transmitted to a third party for analyzing and/or recording on a third party server.

The dispensing device can be configured to communicate with peripheral (or satellite) devices via wired or wireless connections. The peripheral devices that the dispensing device can communicate with for retrieving data of a patient include, but are not limited to a blood pressure cuff (sphygmometer), scale for determining the weight or BMI of a patient, breath or condensate analyzers, internal or external probe or sensor devices, IOP measurement device, other ophthalmologic pathology screening, diagnostic or treatment device, glucometer, HBA1C monitor, heart or cardiovascular monitors (eg., EKG, ECG, etc.), blood oxygen monitors or sensors, respiratory monitors, or any other sensor that detects body chemistry, electrical rhythm, or any other biochemical and/or biomedical signature of the patient.

For example, if a blood sample or weight reading is desired, the dispensing device can provide a signal by generating an audio or visual signal either from the dispensing device or from the peripheral device that the peripheral device is ready to be used or that it is time for the patient to perform an action such as depositing their blood sample or stepping on a scale to detect the weight of the patient. In certain implementations, a third party can communicate with the peripheral devices via a connection to the dispensing device or a distinct connection to the peripheral device.

In another example, when medicaments dispensed by the dispensing device include coatings or other additive chemicals or structures that when ingested by a patient provide an indication of being in the body of the patient, the dispensing device can receive the compliance data indicating that the medicament was taken by the patient. For example, the dispensing device can communicate to the patient that a breath or blood sample is needed to be taken and when the patient provides the breath or blood sample, the dispensing device can receive the data from the peripheral or internal sensor or device that determines whether an indication of the coating or other chemical or body reaction to the coating or other chemical has occurred. The dispensing device can then communicate such data as needed for determining or reporting compliance.

In a further embodiment, a separate (or second) dispensing device or medication container that stores liquids, gases, individuals pills, individuals medications, or any other item can be considered a peripheral storage device to the subject dispensing device. The subject dispensing device can provide an alert with audio and/or visual signals to indicate that the patient should access the peripheral storage device. The peripheral storage device may also contribute to the patient's overall compliance calculations. For example, the peripheral storage devices may also provide auditory and/or visual signals to alert the patient when they are ready for use and/or should be used to maintain compliance with a third party or caregivers instructions. The instructions for the device to function and alert patients may be programmed remotely or non-remotely.

For embodiments where a peripheral device is a separate or second dispensing device, the second dispensing device may, for example, only contain pouches of medication that can also be tracked for patient compliance. Multiple dispensing devices can be configured to communicate together or with one another.

The data generated and/or obtained by the peripheral devices and the data generated and/or obtained by the dispensing device can be used for the purposes of the patient management module **1211** (FPMM) as described with respect to FIG. 12.

Further, the compliance and other patient information that are collected from the dispensing device and its peripheral devices can be provided to a third party database that can be utilized for both prospective and retrospective research and other marketing purposes by a designated third party. The data collected to be used as described above, includes but is not limited to patient outcomes, patient compliance of medication, bioinformatic data, peripheral device data, and any other data collected on the patient from the patient or caregiver or other third party.

Furthermore, an enhanced drug utilization security can be provided by maintaining complete integrity of the prescriptive chain. By incorporating a biometric reader or other security in the subject dispensing devices, caregivers can be notified when narcotics or other medications are being removed or refilled or "by-pass-stored" by physician, payer or law enforcement directives.

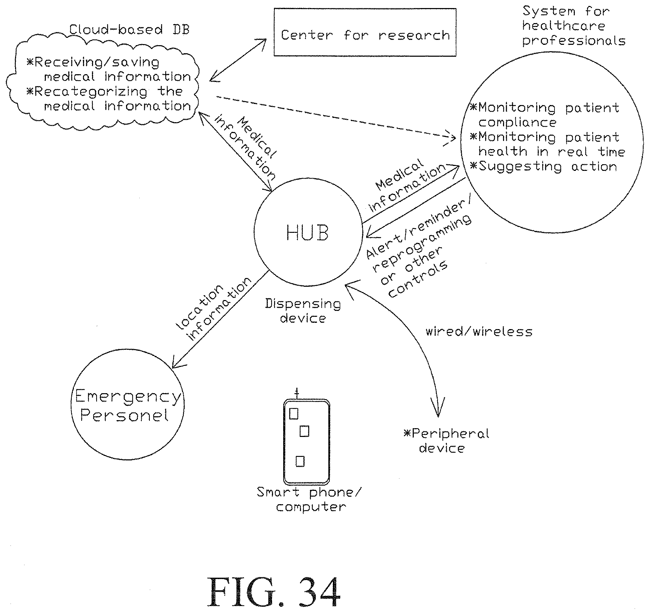

Advantageously, the dispensing device can be considered a therapeutic hub, in which the dispensing device acts to provide a central unit for medication compliance and other healthcare related activities. FIG. 34 shows a diagram of a therapeutic hub in accordance with an embodiment of the invention. As shown in FIG. 34, a dispensing device (hub) can interact with third parties and peripheral devices. For example, the third parties can include but are not limited to health care systems run by health professionals, cloud-based database (DB), and Emergency services. The cloud-based DB can receive, save, and categorize medical information for access by the health care systems run by the health professionals or a center for research having particular access to the cloud-based DB. The ability of the dispensing device to interact with peripheral devices, provide a means for communication, analysis and manipulation of medical data enable an ever expanding role for the dispensing device in a patient's life.

The dispensing device can collect, process and deposit medical data into a "cloud" for use in determining and aiding medical compliance for the patient, and to provide data that can accessible to researchers or other specified users in critical defined tier formats in accordance with HIPPA regulations. The dispensing device can also act as an access point for other devices to access data in the cloud. For example, a medical provider's computer or smart phone can interact with the dispensing device and request the dispensing device to access the cloud database and provide certain data. This may be useful where a particular dispensing device has special permissions or security features that enable access to a particular piece of data in the cloud database.

The above-described methods, systems, interfaces, and data structures can be implemented as computer-readable code in one or more computer-readable media. As is known in the art, data and instructions can be stored in a single computer-readable medium or distributed amongst multiple computer-readable media.

The claims appended hereto are meant to cover modifications and changes within the scope and spirit of the present invention.

All patents, patent applications, provisional applications, and publications referred to or cited herein are incorporated by reference in their entirety, including all figures and tables, to the extent they are not inconsistent with the explicit teachings of this specification.

It should be understood that the examples and embodiments described herein are for illustrative purposes only and that various modifications or changes in light thereof will be suggested to persons skilled in the art and are to be included within the spirit and purview of this application.

It should be understood that any reference in this specification to "one embodiment," "an embodiment," "example embodiment," "further embodiment," "alternative embodiment," etc., is for literary convenience. The implication is that any particular feature, structure, or characteristic described in connection with such an embodiment is included in at least one embodiment of the invention. The appearance of such phrases in various places in the specification does not necessarily refer to the same embodiment. Further, when a particular feature, structure, or characteristic is described in connection with any embodiment, it is submitted that it is within the purview of one skilled in the art to affect such feature, structure, or characteristic in connection with other ones of the embodiments.

It is to be understood that embodiments of the invention can be carried out by specifically different equipment and devices as that explicitly described, and that various modifications, both as to equipment details and operating procedures can be effected without departing from the scope of the invention itself. All or some of the embodiments can be selectively combined to yield variants. Many different arrangements of the various components depicted, as well as components not shown, are possible without departing from the spirit and scope of the present invention. A skilled artisan can develop alternative means of implementing the aforementioned improvements without departing from the scope of the present invention. It will be understood that certain features and subcombinations are of utility and can be employed without reference to other features and subcombinations and are contemplated within the scope of the claims. Not all steps listed in the various figures need be carried out in the specific order described. Further, it should be understood that, although the present invention has been described with reference to specific details of certain embodiments thereof, it is not intended that such details should be regarded as limitations upon the scope of the invention except as and to the extent that they are included in the accompanying claims.

### Items according to the disclosure

Item 1 A therapeutic hub, comprising:
   a dispensing device comprising a dispensing module for control of delivery of at least one medicament to be taken by a patient, a communication module for control of transmitting and
   receiving data via at least one communication port, the data including compliance information of the patient;
   a user interface for receiving user input to the dispensing device and providing video and audio output to the user.
Item 2 The therapeutic hub according to item 1, wherein the dispensing device comprises a first cavity in communication with a dispensing output for storing the at least one medicament before being dispensed to the patient via the dispensing output; and a second cavity in communication with the first cavity for storing medicament removed from the first cavity but not dispensed to the patient.
Item 3 The therapeutic hub according to item 1, wherein the dispensing module is configured to cause the medicament removed from the first cavity but not dispensed to the patient to be stored in the second cavity according to predetermined conditions.
Item 4 The therapeutic hub according to item 3, wherein the predetermined conditions are at least one of the conditions selected from the group consisting of when a patient fails to take the medicament at a predetermined time, when a signal is received to remove the medicament from being dispensed, and when a signal is received indicating a change in prescription.
Item 5 The therapeutic hub according to item 1, wherein the at least one communication port comprises an antenna.
Item 6 The therapeutic hub according to item 1, wherein the at least one communication port comprises a wired connection port.
Item 7 The therapeutic hub according to item 1, wherein the dispensing device further comprises a processing module for analyzing input provided by a peripheral device in communication with the dispensing device and outputting the analyzed input to the user interface or the communication module for transmission to a device in communication with the dispensing device.
Item 8 The therapeutic hub according to item 1, wherein the at least one communication port is configured to communicate with one or more peripheral devices selected from the group consisting of a blood pressure cuff, a weight or BMI scale, an intraocular pressure measurement device, an ophthalmologic pathology screening, diagnostic or treatment device, a breath analyzer, a glucometer, a HBA1 C monitor, a heart or cardiovascular monitor, a blood oxygen monitor or sensor, and a respiratory monitor.
Item 9 The therapeutic hub according to item 1, wherein the at least one communication port is configured to communicate with a smart phone or computer, wherein the smart phone or computer provides a processing control for features of the dispensing device.
Item 10 The therapeutic hub according to item 1, wherein the dispensing device is configured to provide a signal to the patient indicating that it is time for the patient to perform an action by generating an audio and/or visual signal from the user interface of the dispensing device.
Item 11 The therapeutic hub according to item 1, wherein the dispensing device is configured to provide a signal to the patient indicating that it is time for the patient to perform an action by generating an audio and/or visual signal from a peripheral device wired or wirelessly connected with the dispensing device via the communication module.
Item 12 The therapeutic hub according to item 1, wherein the dispensing device is configured to receive a control signal via the at least one communication port, wherein the control signal provides a remote control of one or more functions of the dispensing device.
Item 13 The therapeutic hub according to item 1, further comprising a storage device in communication with the dispensing device, the storage device having at least one cavity.
Item 14 The therapeutic hub according to item 13, wherein the dispensing module of the dispensing device controls delivery of at least one medicament to be taken by the patient that is stored in the storage device.
Item 15 The therapeutic hub according to item 1, wherein the communication module transmits data to a cloud-based database.
Item 16 The therapeutic hub according to item 15, wherein the communication module is further configured to control access of a peripheral device to the cloud-based database.
Item 17 The therapeutic hub according to item 1, wherein the dispensing device comprises a GPS unit, accelerometer, or gyroscope unit for providing positioning data, wherein the communication module is configured to transmit the positioning data to a third party.
Item 18 The therapeutic hub according to item 1, wherein the dispensing device further comprises a security module for the communication module, the security module providing at least one security feature from the group consisting of encryption of data transmitted from the communication module and verification of third party source permissions for control operations of the dispensing device received via the at least one communication port.
Item 19 The therapeutic hub according to item 1, wherein the dispensing device further comprises a biometric reader or personalized registration device and a security module for analyzing a signal received from the biometric reader or personalized registration device.

## Claims

1. A compliance monitoring system, comprising:
a scanner or camera that senses or visualizes a medication or a container;
a biometric reader;
a database that stores patient related data; and
a computer-executable instructions comprising stored instructions that when executed cause at least one processor to:
determine medication compliance by a patient by comparing detection of the biometric reader to the scanner that scans or captures via a camera or reader the medication or the container; and
store data collected from the reader, scanner, and from determination of medication compliance in the database.

2. The compliance monitoring system of claim 1, wherein the system further comprises a device that is a mobile device, a wearable electronic device, a computing device, a biometric data-point collection device, an automated personal assistant, or an artificial intelligence assistant for accessing the system while elevating compliance and patient health management system.

3. The compliance monitoring system of claim 1, wherein the system collects exercise data, gym data, efficacy of prescription data, food purchases, real-time patient biometric information, and/or past healthcare data from disparate health data repositories of the patient.

4. The compliance monitoring system of claim 1, wherein the database further stores information from a pharmacy and/or electronic medical record, and correlate disparate database(s) for management and elevation of patient health, and uses the database to provide a medication schedule to the patient.

5. The compliance monitoring system of claim 1, wherein the scanner or camera scans barcodes and/or RFID tag.

6. The compliance monitoring system of claim 1, wherein the biometric reader detects a face, fingerprint, blood pressure, pulse, blood sugar level, and/or body temperature.

7. The compliance monitoring system of claim 1, wherein the computer-executable instructions are further configured to notify the patient with specific information, refill reminders, pill recalls, and/or clinical information.

8. The compliance monitoring system of claim 1, wherein the computer-executable instructions are further configured to establish an electronic communication channel between the patient and a pharmacy, a logistics carrier, and/or a freight carrier.

9. The compliance monitoring system of claim 1, wherein the computer-executable instructions are further configured to permit a healthcare professional, health payers, and/or employers to access and interact with data collected from the reader and/or the scanner.

10. The compliance monitoring system of claim 1, wherein the computer-executable instructions are further configured to permit a social networking site to collect data.

11. The compliance monitoring system of claim 1, wherein the computer-executable instructions are further configured to generate a coupon or incentives on medication purchases.

12. The compliance monitoring system of claim 1, wherein the computer-executable instructions are further configured to use one or more connected evidence based medicine and/or therapeutic database(s), electronic medical record(s), specific patient health record(s), and/or physician decision making support system(s).

13. The compliance monitoring system of claim 1, wherein the computer-executable instructions are further configured to permit a healthcare professional to manage patient health / treatment in real-time.

14. The compliance monitoring system claim 1, further comprising a dispensing system having at least one or more substrate(s), wherein one or more substrate(s) represent a drug dose or a treatment regimen.

15. The compliance monitoring system of claim 14, wherein the drug dose or the treatment regimen is delivered over a daily, weekly, monthly, quarterly, or longer period(s) of time.

16. The compliance monitoring system of claim 14, wherein the dispensing system further comprises titrating the medication in real-time.

17. The compliance monitoring system of claim 14, wherein the dispensing system further comprises storing one or more unique medicament formulations of commonly prescribed drugs.

18. The compliance monitoring system of claim 14, wherein the dispensing system further comprises dispensing a compound single formula of multiple therapeutics and/or a multidose Unit Packaging modality.

19. The compliance monitoring system of claim 14, wherein the dispensing system further comprises providing an operator confirmation feedback before the medication is finalized for dispensing.

20. The compliance monitoring system of claim 1, further comprising a display and interactive input controls.

21. The compliance monitoring system of claim 1, wherein the computer-executable instructions are further configured to provide an compliance score communicated to the patient, a healthcare provider, a health payer, an employer and/or a patient's family.

22. The compliance monitoring system of claim 21, wherein the healthcare provider, a health payer, and/or employer uses the compliance score to provide incentives for patient compliance.

23. The compliance monitoring system of claim 21, wherein the compliance score is calculated using data from the biometric reader, vocation of the patient, education level of the patient, weight of the patient, patients number of medications, disease condition, or medical treatment, racial background, and/or location of the patient.

24. The compliance monitoring system according to claim 1, further comprising a GPS unit.

25. The compliance monitoring system according to claim 24, wherein the computer-executable instructions is configured to use the GPS unit to provide the GPS location of a medication compliance event.
